(19) Europäisches Patentamt
European Patent Office
Office européen des brevets

(11) Publication number: **0 049 071**
**B1**

(12) **EUROPEAN PATENT SPECIFICATION**

(45) Date of publication of patent specification: **19.12.84**

(21) Application number: **81304225.6**

(22) Date of filing: **15.09.81**

(51) Int. Cl.$^3$: **C 07 D 261/14,**
C 07 D 231/40,
C 07 D 233/88,
C 07 D 237/20,
C 07 D 249/14,
C 07 D 271/06,
C 07 D 271/10,
C 07 D 275/02,
C 07 D 285/08,
C 07 D 285/12, C 07 D 417/04

(54) **N-arylbenzamide derivatives.**

(30) Priority: **16.09.80 US 187675**

(43) Date of publication of application:
**07.04.82 Bulletin 82/14**

(45) Publication of the grant of the patent:
**19.12.84 Bulletin 84/51**

(84) Designated Contracting States:
**AT BE CH DE FR GB IT LI LU NL SE**

(56) References cited:
**DE-A-1 923 939**
**DE-A-2 436 179**
**GB-A-1 547 564**
**GB-A-1 548 397**
**GB-A-1 551 735**
**US-A-2 953 491**
**US-A-4 062 861**
**US-A-4 141 984**

**The file contains technical information submitted after the application was filed and not included in this specification**

(73) Proprietor: **ELI LILLY AND COMPANY**
**307, East McCarty Street**
**Indianapolis Indiana 46285 (US)**

(72) Inventor: **Burow, Kenneth Wayne, Jr.**
**5449 East 62nd Street**
**Indianapolis Indiana 46220 (US)**

(74) Representative: **Crowther, Terence Roger et al**
**Erl Wood Manor**
**Windlesham Surrey GU20 6PH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 049 071 B1**

Courier Press, Leamington Spa, England.

## Description

This invention concerns a class of novel N-arylbenzamide derivatives, which have been discovered to be effective herbicides.

The use of herbicides to control selectively the growth of unwanted vegetation in crops grown for human and domestic animal consumption is well established. However, a number of problems are associated with the continued use of chemical herbicides, including environmental pollution, crop tolerance, weed resistance, as well as economic considerations. Accordingly, the search continues for new herbicides which are economical and which are selectively toxic to unwanted vegetation.

A wide variety of benzamides are known in the art. Ward, for example, in U.S. Patent No. 4,141,984, discloses certain thiadiazolyl benzamides which are said to possess insecticidal efficacy. Kaplan *et al*, in U.S. Patent No. 4,189,495, disclose a series of 2-methoxybenzamides which allegedly possess pharmacological activity. US—A—2953491 discloses certain N-(4H-1,2,4-triazol-3-yl)benzamides having biocidal activity against radish seeds. There is no disclosure of substituted triazolyl benzamides. DE—A—2436179 discloses 3-benzoylamino-5-*t*-butylisoxazole, which is alleged to be useful as a herbicide.

It has now been discovered that N-arylbenzamide derivatives of formula (I)

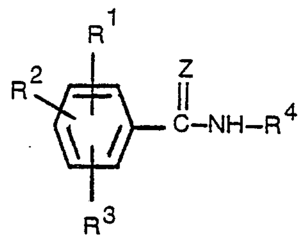
(I)

wherein:

Z is oxygen or sulfur;

$R^1$ is hydrogen, halogen, $C_1$—$C_4$ alkyl, or $C_1$—$C_4$ alkoxy;

$R^2$ is hydrogen, halogen, $C_1$—$C_4$ alkyl, $C_1$—$C_4$ alkoxy, $C_1$—$c_4$ alkylthio or trifluoromethyl;

$R^3$ is hydrogen, halogen, $C_1$—$C_4$ alkyl, $C_1$—$C_4$ alkoxy or $C_1$—$C_4$ alkylthio; provided that when one of $R^1$, $R^2$ or $R^3$ is alkyl, one or both of the other phenyl substituents is other than hydrogen; and when $R^2$ is trifluoromethyl, one or both of $R^1$ and $R^3$ is other than hydrogen;

$R^4$ is an aryl group selected from

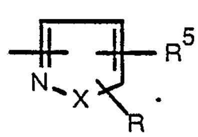 , 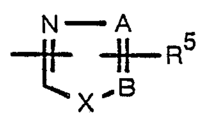 , 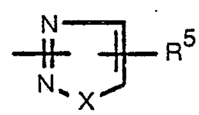 ,

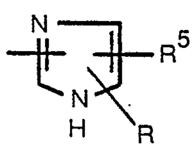 or 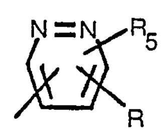

wherein:

A is CH or N and B is CH or N, provided that one of A and B is CH and the other is N;

X is NH, O or S;

R is hydrogen or $C_1$—$C_4$ alkyl;

$R^5$ is

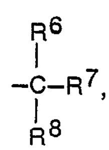 or 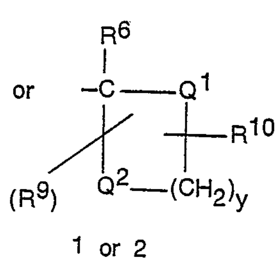

wherein:

y is an integer from 0 to 5;

$R^6$, $R^7$ and $R^8$ independently are hydrogen, $C_1$—$C_{13}$ alkyl, halo-$C_1$—$C_{13}$ alkyl, $C_2$—$C_{13}$ alkenyl, $C_2$—$C_{13}$ alkynyl, $C_1$—$C_4$ alkoxy-$C_1$—$C_6$ alkyl, $C_1$—$C_4$ alkylthio-$C_1$—$C_6$ alkyl, $C_1$—$C_6$ alkoxy, $C_2$—$C_4$ alkanoyloxy-$C_1$—$C_6$ alkyl, $C_1$—$C_6$ alkylthio, or

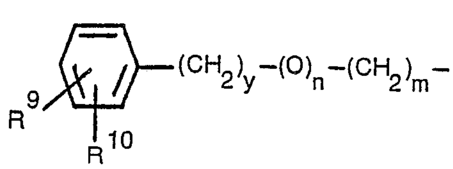 , or 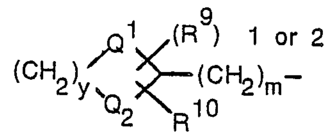

in which

m is an integer from 0 to 4;

n is zero or 1; and

$R^9$ and $R^{10}$ independently are hydrogen, halogen, $C_1$—$C_4$ alkyl or $C_2$—$C_4$ alkenyl;

$Q^1$ and $Q^2$ are independently $CH_2$, O, S, $CH_2O$ or $CH_2S$, provided that when $Q^1$ and $Q^2$ are both other than $CH_2$, y is not 0; and provided that $R^2$ and $R^3$ are other than hydrogen when $R^4$ is

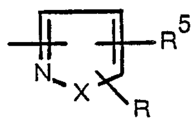 or 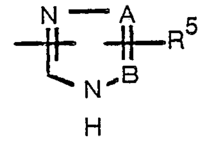

and their agronomically-acceptable salts, are effective herbicides.

One valuable class of benzamide derivatives of formula (I) which may be mentioned are those compounds wherein Z is oxygen, $R^4$ is

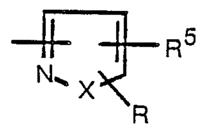 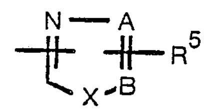

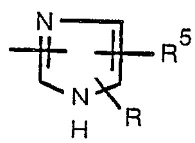 or 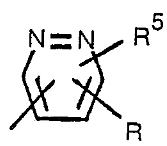

where R is hydrogen; $R^5$ is

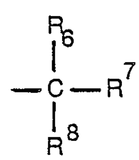 or 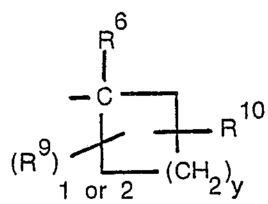

wherein:

$R^6$ and $R^7$ independently are hydrogen, $C_1$—$C_4$ alkyl, $C_2$—$C_4$ alkenyl or $C_2$—$C_4$ alkynyl;

y is an integer from 0 to 4;

$R^8$ is hydrogen, $C_1$—$C_{13}$ alkyl, halo-$C_1$—$C_{13}$ alkyl, $C_2$—$C_{13}$ alkenyl, $C_2$—$C_{13}$ alkynyl, $C_1$—$C_4$ alkoxy-$C_1$—$C_6$ alkyl, $C_1$—$C_4$ alkylthio-$C_1$—$C_6$ alkyl,

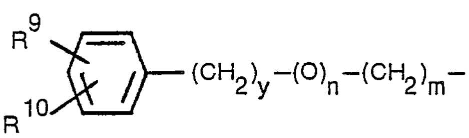 or 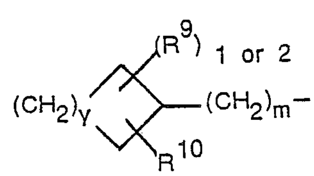

wherein:

m is an integer from 0 to 4;

n is zero or 1; and

$R^9$ and $R^{10}$ independently are hydrogen, halogen, $C_1$—$C_4$ alkyl or $C_2$—$C_4$ alkenyl.

Preferred compounds are those defined by the above formula wherein one or more of the following characteristics apply:

(a) Z is oxygen;

(b) R is hydrogen;

(c) $R^1$ is hydrogen and $R^2$ and $R^3$ independently are $C_1$—$C_4$ alkoxy, $C_1$—$C_4$ alkyl, or $C_1$—$C_4$ alkylthio, especially when attached at the 2 and 6-positions;

(d) $R^1$ is hydrogen and $R^2$ and $R^3$ independently are $C_1$—$C_4$ alkoxy, preferably attached at the 2 and 6-positions;

(e) $R^1$ is hydrogen, $R^2$ is 2-methoxy or ethoxy and $R^3$ is 6-methoxy or ethoxy;

(f) $R^4$ is 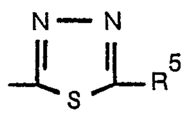 ;

(g) $R^4$ is 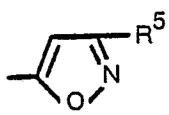 ;

(h) $R^4$ is 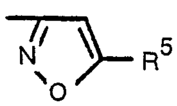 ;

(i) $R^5$ is 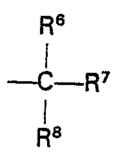

wherein $R^6$ is $C_1$—$C_4$ alkyl;

(j) $R^5$ is 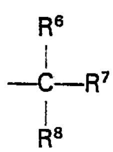

wherein one or both of $R^6$ and $R^7$ independently are $C_1$—$C_4$ alkyl;

(k) $R^5$ is 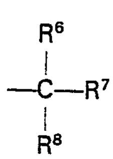

wherein one or both of $R^6$ and $R^7$ independently are $C_1$—$C_4$ alkyl and $R^8$ is $C_1$—$C_{13}$ alkyl or cycloalkyl of the formula

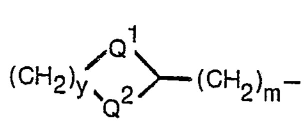

wherein $Q^1$ and $Q^2$ are both $CH_2$;

(l) $R^5$ is 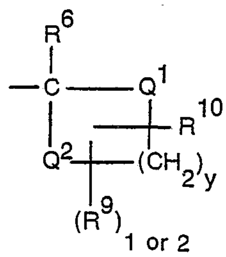

wherein $R^6$ is $C_1$—$C_4$ alkyl, $R^9$ and $R^{10}$ both are hydrogen and $Q^1$ and $Q^2$ are both $CH_2$;

(m) $R^5$ is 1-ethyl-1-methylpropyl;

(n) $R^4$ is 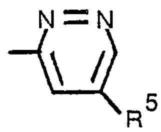 ;

(o) $R^4$ is 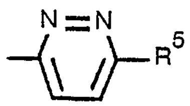 .

The N-arylbenzamide derivatives of formula (I) or their agronomically-acceptable salts will normally be sold as a herbicidal formulation together with one or more agronomically-acceptable carriers or diluents therefor. Formulations comprising one or more other compatible herbicides are also contemplated.

Additionally provided by the invention is a method for controlling the growth of undesired vegetation comprising applying to the locus where vegetative control is desired a herbicidally-effective amount of a benzamide defined by the above general formula. The herbicidal method is ideally carried out employing the preferred benzamides as delineated above. For example, a preferred method comprises applying to the locus where the growth of unwanted vegetation is to be eliminated or retarded a herbicidally effective amount of a benzamide of the formula

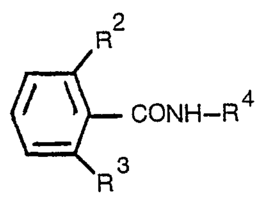

wherein $R^2$ and $R^3$ independently are $C_1$—$C_4$ alkoxy such as methoxy or ethoxy, and $R^4$ is selected from

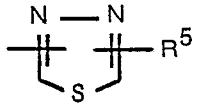 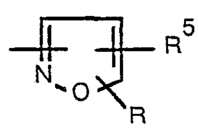 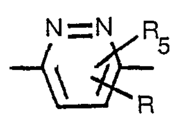

wherein R is hydrogen, wherein $R^5$ is

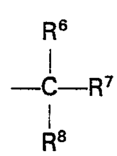

and $R^6$ and $R^7$ independently are $C_1$—$C_4$ alkyl and $R^8$ is $C_1$—$C_{13}$ alkyl or cycloalkyl of the formula

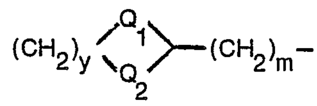

wherein $Q_1$ and $Q_2$ both represent methylene.

$R^1$, $R^2$ and $R^3$ are used in the above formula to define groups which include $C_1$—$C_4$ alkyl, $C_1$—$C_4$ alkoxy, $C_1$—$C_4$ alkylthio, and halogen. The term "$C_1$—$C_4$ alkyl" refers to both straight and branched chain alkyl groups such as methyl, ethyl, *iso*propyl and *tert.*-butyl.

The term "$C_1$—$C_4$ alkoxy" refers to the same $C_1$—$C_4$ alkyl groups which are linked through an oxygen atom. Such groups include methoxy, *n*-propoxy, *n*-butoxy and the like. Typical "$C_1$—$C_4$ alkylthio" groups include methylthio, ethylthio and *iso*propylthio. "Halogen" as used herein refers to fluoro, chloro, bromo and iodo. As noted above, preferred benzamides are those wherein $R^1$ is hydrogen and $R^2$ and $R^3$ are alkoxy, especially methoxy or ethoxy attached at the 2 and 6-positions of the benzamide phenyl ring.

$R^4$ in the above formula defines an aryl moiety such as isoxazolyl, thiadiazolyl, isothiazolyl, triazolyl, pyrazolyl, oxadiazolyl, and pyridazinyl.

Preferred benzamide derivatives of formula (I) are those defined by the above formula wherein $R^4$ is 3-isoxazolyl, 5-isoxazolyl or 1,3,4-thiadiazolyl.

The aryl moiety defined by $R^4$ is required to bear a substituent defined by $R^5$. It has been found that the most active, and accordingly the most preferred benzamide derivatives, are those wherein the aryl substituent defined by $R^5$ is a sterically hindered or bulky group. The concept of a sterically hindered or bulky group is known to those skilled in the art. Such terms include herein groups which are attached to the aryl ring through a secondary or tertiary carbon atom. Cyclic groups which are linked through a secondary or tertiary carbon atom also are bulky groups, as are cyclic groups attached directly to the aryl moiety.

A typical $R^5$ substituent which is preferred is a bulky alkyl group. For example, $R^5$ represents an alkyl substituent when it refers to a group of the formula

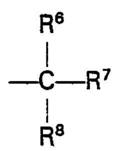

and $R^6$ and $R^7$ refer to hydrogen or $C_1$—$C_4$ alkyl and $R^8$ is $C_1$—$C_{13}$ alkyl. The most bulky alkyl groups clearly are those wherein one or both of $R^6$ and $R^7$ independently are $C_1$—$C_4$ alkyl, and benzamides bearing such substituents are preferred. Typically preferred bulky alkyl groups thus include *tert.*-butyl, 1,1-dimethylpropyl, 1,1-diethylpentyl, 1-ethyl-1-propylhexyl, 1-ethyl-1-methylpropyl, 1,1-diethyloctyl, 1,1-diethylpropyl, 1-methyl-1-*n*-propylheptyl, 1-ethyl-1-*iso*propyloctyl, and 1,1-di-*n*-propyldecyl.

Other bulky alkyl groups which can be utilized include 1,2-dimethylbutyl, 1,2-dimethylheptyl, 2,2-dimethylpentyl, 1-ethylheptyl, 2,2-diethylpropyl, 1,1-dimethyl-2,2-diethylbutyl, 1,1,2,2-tetramethylhexyl, and 1-methyl-1-*n*-propyl-2-ethyloctyl.

The aryl substituent defined by $R^5$ in the above formula can be any of several bulky substituents other than a straight or branched chain alkyl group, including cycloalkyl of the formula

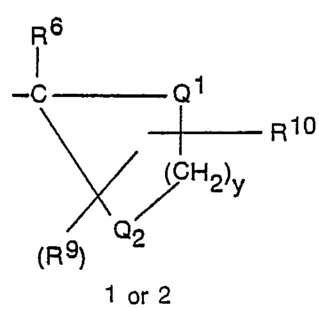

wherein $R^6$ is $C_1$—$C_4$ alkyl; $R^9$ and $R^{10}$ independently are hydrogen or $C_1$—$C_4$ alkyl; $Q^1$ and $Q^2$ are $CH_2$; and y is an integer from 0 to 4. Typical examples of such groups include 1-methylcyclobutyl, 1-ethylcyclopentyl, 1-ethylcyclohexyl and 1-methylcycloheptyl.

$R^5$ also refers to haloalkyl groups, alkenyl groups, and alkynyl groups, for instance when $R^5$ is —$CR^6R^7R^8$ wherein $R^8$ is halo-$C_1$—$C_{13}$ alkyl, $C_2$—$C_{13}$ alkenyl or $C_2$—$C_{13}$ alkynyl respectively. Examples

of such haloalkyl, alkenyl and alkynyl substituents as defined by $R^5$ include 1,1-dimethyl-3-chloropropyl, 1,2-diethyl-3,4-dibromohexyl, 1-ethyl-1-*n*-propyl-8-iodononyl, 1,1-di-*n*-propyl-4-fluorobutyl, 1-methyl-1-*iso*propyl-12-chlorododecyl, 1,1-dimethyl-3-butenyl, 2,3-diethyl-4-hexenyl, 1,1-diethyl-2,2-dimethyl-5-hexenyl, 1,1,2,2,3,3-hexamethyl-9-decenyl, 1,1-dimethyl-4-hexynyl, 1,2-diethyl-3-heptynyl, and 1-*iso*propyl-3-butynyl.

$R^5$ in the above formula additionally refers to $CR^6R^7R^8$ wherein $R^8$ is $C_1$—$C_4$ alkoxy-$C_1$—$C_6$ alkyl and $C_1$—$C_4$ alkylthio-$C_1$—$C_6$ alkyl. These terms refer to straight or branched carbon chains from one to six carbon atoms in length which bear a $C_1$—$C_4$ straight or branched carbon chain linked through oxygen or sulfur. Preferred alkoxyalkyl and alkylthioalkyl groups defined by $R^5$ are those which are sterically hindered or bulky, for example those which include a branched carbon chain attached directly to the aryl group (i.e. where one or both of $R^6$ and $R^7$ are alkyl). Such preferred groups include 1,1-dimethyl-2-methoxyethyl, 1-ethyl-3-methylthiopropyl, 1,1-diethyl-2-*iso*propoxy-ethyl, 1-*n*-propyl-3-*tert.*-butylthiopropyl and 2-ethyl-3-methyl-3-*n*-butoxypropyl.

$R^5$ also defines an alkyl group which bears a phenylalkyl or phenylalkoxy moiety, or a cycloalkyl moiety. For example, $R^5$ refers to the group —$CR^6R^7R^8$ in which $R^8$ includes phenylalkyl, phenylalkoxy, cycloalkyl and cycloalkylalkyl substituents defined by the formulas

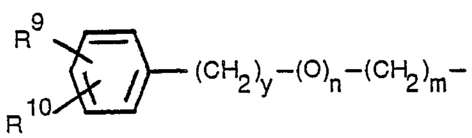

and

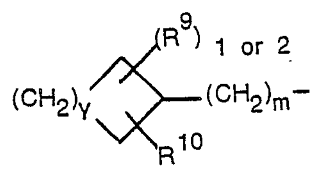

wherein y, m, n, $R^9$ and $R^{10}$ are defined above. Examples of benzamide aryl substituents thus defined by $R^5$ include 1,1-dimethyl-2-phenoxyethyl, 1,1-diethyl-3-benzyloxypropyl, 1,1-diethyl-2-phenylethyl, 1-*n*-propyl-3-(2-chlorophenyl)butyl, 1,1-dimethyl-2-cyclohexylethyl, 1-ethyl-2-methyl-2-(3-allylcyclohexyl)-ethyl, 1,1-diethyl-2-(3-phenylpropoxy)ethyl or 1-*iso*propyl-4-(2,2-dichlorocylopentyl)butyl.

The N-arylbenzamide derivatives of formula (I) can be prepared by processes which are presently known in the art or which are analogous to art-known synthetic methods. A preferred method for preparing the compounds is by acylation or an aryl amine of the formula $H_2NR^4$ with an appropriately substituted benzoic acid derivative according to the following sequence:

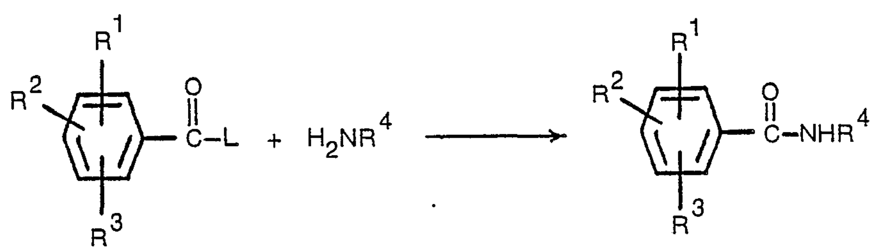

wherein $R^1$, $R^2$, $R^3$ and $R^4$ are as defined above, and L is a good leaving group, for example lower alkanoyloxy such as formyloxy or acetoxy; halide such as chloro, bromo or iodo, or an activated ester forming group such as pentachlorophenoxy. A preferred good leaving group is halogen such as chloro or bromo.

To form benzamide derivatives of formula (I) in which Z is sulfur, it is necessary to thiate the ketone group of the amide formed in the above acylation reaction. Suitable thiating agents are phosphorous pentasulfide or Lawesson's Reagent (*Tetrahedron Letters, 22,* 4061 (1980)). The thiation is preferably accomplished by refluxing the amide starting material with the thiating agent in an aprotic organic solvent such as dioxane or toluene. Typical reaction temperatures range from 50 to 150°C.

The acylation reaction can be carried out by commingling the benzoic acid derivative with about an equimolar quantity of the aryl amine in a mutual solvent such as tetrahydrofuran, diethyl ether, dichloromethane, dioxane, dimethylsulfoxide, dimethylformamide, benzene or toluene. If desired, a base can be utilized in the acylation reaction to act as an acid scavenger. Commonly used bases include sodium carbonate, sodium hydride, potassium carbonate, sodium hydroxide, pyridine, triethylamine and related bases. The acylation generally is substantially complete after about two to about ninety hours when carried out at a temperature of about 20 to about 200°C, preferably from about 30 to about 120°C. The product of the reaction, an N-aryl benzamide of the invention, can be isolated by simply removing the reaction solvent, for instance by evaporation under reduced pressure. The product can be further purified if needed by any of several routine methods, including crystallization from solvents such as ethanol, ethyl acetate, diethyl ether or toluene; chromatography over solid supports such as silica or alumina, and related purification techniques.

The preparation of the benzamide derivatives of formula (I) requires the use of starting materials which are either known or readily preparable by methods familiar to those in the art. Most of the substituted benzoic acids which are required are commercialy available. The reactive derivatives of such benzoic acids are prepared by routine procedures. For example, a preferred synthetic method according to this invention requires the use of substituted benzoic acid halides or mixed anhydrides. The acid halides are prepared by reaction of a benzoic acid with a halogenating agent such as oxalyl chloride, thionyl chloride, phosphorus trichloride or phosphorus tribromide.

The benzoic acid anhydrides which can be condensed with an amine to form a benzamide can be made by reacting a benzoic acid alkali metal salt, for example a sodium or potassium salt, with an acid halide such as benzoyl chloride or acetyl bromide.

Benzoic acid derivatives which are commonly employed in the synthesis of the benzamides of this invention include the following:

2,6-dimethoxybenzoyl chloride;
2,6-dimethoxybenzoyl formic anhydride;
2,6-diethoxybenzoyl bromide;
2,6-di-*n*-propoxybenzoyl iodide;
2,6-di-*iso*propoxybenzoyl bromide;
2-methoxy-6-*n*-propoxybenzoyl chloride;
2-methoxy-3-ethoxybenzoyl bromide;
3-ethoxy-5-*n*-propoxybenzoyl acetic anhydride;
2,4,6-triethylbenzoyl bromide;
3,5-di-n-butylbenzoyl chloride;
2-methoxy-6-chlorobenzoyl bromide;
2,6-di-*n*-propoxybenzoyl acetic anhydride;
2-methoxy-6-fluorobenzyl bromide;
2,6-dimethoxy-4-trifluoromethylbenzoyl chloride;
2,6-diethylbenzoyl iodide;
2,4,6-trimethoxybenzoyl chloride;
2-methoxy-6-ethylthiobenzoyl bromide and
2-methoxy-6-ethyl-4-trifluoromethyl benzoyl bromide.

The aryl amines of the formula $H_2NR^4$ which are required as starting materials are either readily available commercially or are preparable by art-recognized procedures. For example, 3-substituted-5-aminoisoxazoles can be prepared by reaction of a suitable carboxylic acid ester such as $R^5COOCH_3$ with acetonitrile and a strong base such as sodium hydride, followed by reaction with hydroxylamine according to the following scheme:

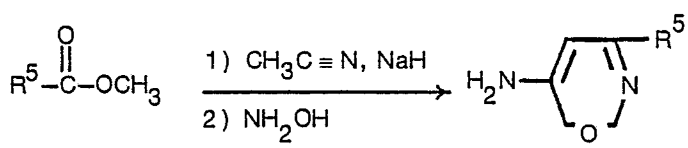


Similarly, 5-substituted-3-amino-isoxazoles can be prepared by iminoether formation with a suitably substituted $\beta$-ketonitrile followed by treatment with hydroxyimine to form an iminium salt, and finally reaction with a protonic acid according to the following general scheme:

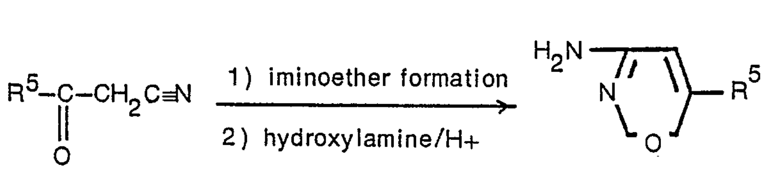


The thiadiazole amines which are required as starting materials can be prepared by reaction of a carboxylic acid halide with thiosemicarbazide as follows:

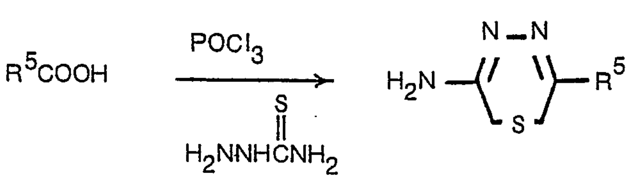


Similarly, pyrazole amines can be synthesized by reacting hydrazine with a suitably substituted $\beta$-keto nitrile according to the following scheme:

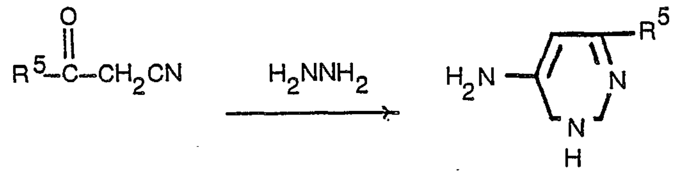


Hydrazine is also utilized in the synthesis of triazole derivatives according to the following path:

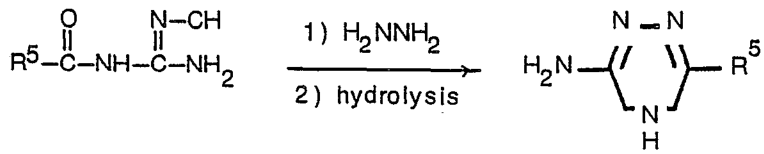


Imidazolyl amines can be prepared by reacting cyanamide with an aminomethyl ketone according to the following scheme:

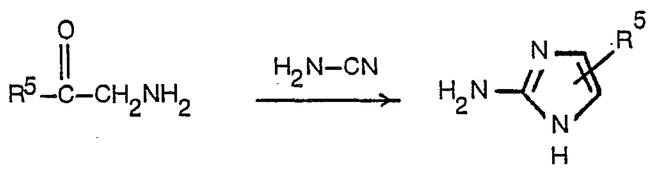


Aminomethyl ketones also are reacted with ammonia, hydrogen sulfide and hydrogen peroxide to give amino isothiazoles:

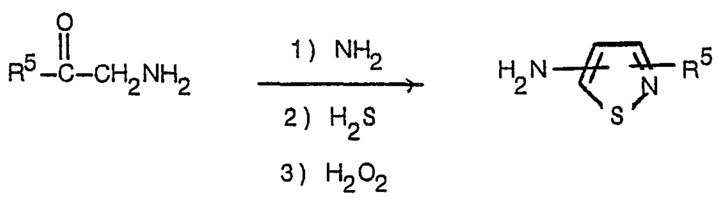


Amino oxadiazoles are readily prepared by reaction of an amide oxime with trichloroacetic anhydride followed by treatment with ammonia:

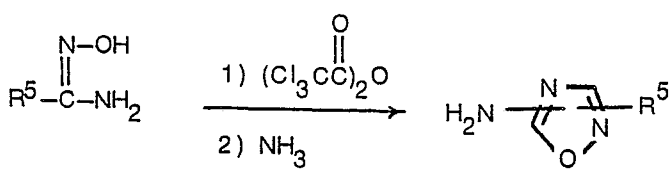


Amino pyridazines are prepared by reaction of ammonia with a suitably substituted halopyridazine according to the following scheme:

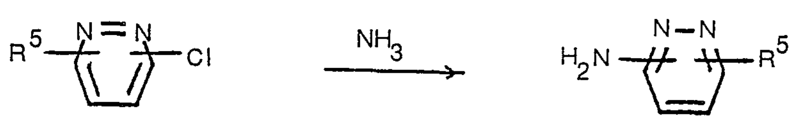


These and other synthetic paths leading to aryl amines of the formula $H_2NR^4$ are well known to those skilled in the art of organic chemistry.

As already pointed out, preferred aryl substituents defined in the above general formula by $R^4$ include 3-isoxazolyl, 5-isoxazolyl and 1,3,4-thiadiazol-2-yl. Typical examples of isoxazolyl amines and thiadiazolyl amines which are employed as starting materials to be acylated with a benzoic acid derivative include the following:

3-*tert.*-butyl-5-aminoisoxazole;

3-(1,1-dimethylpentyl)-5-aminoisoxazole;
3-(1,1-diethyl-4-ethoxybutyl)-5-aminoisoxazole;
3-(1-ethylcyclohexyl)-5-aminoisoxazole;
3-(1,2-diethyl-5-hexenyl)-5-aminoisoxazole;
3-(2,2-di-*n*-propyl-4-cycloheptylbutyl)-5-aminoisoxazole;
3-[1,1-dimethyl-3-(4-phenylbutoxy)propyl] -5-aminoisoxazole;
3-(1,1-diethyl-5-cyclohexylpentyl)-5-aminoisoxazole;
3-(1-*n*-butyl-2,3-dichlorocyclopropyl)-5-aminoisoxazole;
3-amino-5-(1,1-diethylheptyl)isoxazole;
3-amino-5-(1-*iso*propyl-3-cyclohexylpropyl)isoxazole;
3-amino-5-(1,1-dimethyl-6-heptenyl)isoxazole;
3-amino-5-[1,1-diethyl-3-(2,6-dibromophenyl)propyl]isoxazole;
3-amino-5-(1,1-di-*n*-propyl-6-iodohexyl)isoxazole;
3-amino-5-(1,2-dimethyl-3-ethylthiopropyl)isoxazole;
3-amino-5-(2,3-dimethyl-5-*iso*butoxypentyl)isoxazole;
3-amino-5-(1,4-dimethyl-2,2,3-triethyl-5-bromopentyl)isoxazole;
2-amino-5-*tert.*-butyl-1,3,4-thiadiazole;
2-amino-5-(1-ethyl-1-methylpropyl)-1,3,4-thiadiazole;
2-amino-5-(1,1-diethyl-5-ethylthiopentyl)-1,3,4-thiadiazole;
2-amino-5-[2,2-di-*n*-propyl-4-(2-methylphenoxy)butyl]-1,3,4-thiadiazole;
2-amino-5-(1-ethyl-3-*iso*propyl-4-chlorocyclopentyl)-1,3,4-thiadiazole;
3-amino-5-(2-*n*-propyl-3-methyl-5-*tert.*-butylthiopentyl)-1,3,4-thiadiazole;
2-amino-5-(1,1-dimethyl-3-iodopropyl)-1,3,4-thiadiazole;
2-amino-5-(1,1-di-*n*-propyldecyl)-1,3,4-thiadiazole;
2-amino-5-(1,1-diethyl-6-heptynyl-1,3,4-thiadiazole;

Additional aryl amines which can be employed in the synthesis of the benzamide derivatives of this invention include oxadiazoles, triazoles, isothiazoles, imidazoles, pyrazoles, and pyridazines. Illustrative of the such commonly used aryl amines are:

2-amino-5-(1-ethyl-1-methylpropyl)-1,3,4-oxadiazole;
2-amino-5-*tert.*-butyl -1,2,4-oxadiazole;
2-amino-5-(1,1-dimethyl-6-*iso*propoxyhexyl)-1,3,4-oxadiazole;
5-amino-3-(1-*n*-propylcycloheptyl)-1,3,4-oxadiazole;
3-amino-5-(1,1-dimethyl-3-ethyl-4-methylthiobutyl)-1,2,4-oxadiazole;
2-amino-5-[1,1-di-*n*-propyl-3-(4-bromophenyl)propyl-1,3,4-oxadiazole;
2-amino-1,3,4-triazole;
3-amino-5-*tert.*-butyl -1,2,4-triazole;
2-amino-5-(1,1-diethylhexyl)-1,3,4-triazole;
2-amino-5-(1-*n*-propyl-2,2-dibromocyclopropyl)-1,3,4-triazole;
3-amino-5-(1,1-diethyl-4-fluorobutyl)-1,2,4-triazole;
2-amino-5-(1,1-dimethyl-2,2-diethyl-3-*n*-propyl-4-pentenyl)-1,3,4-triazole;
3-amino-5-(1,1-diethylpropyl)-1,2,4-thiazole;
5-amino-3-[1-(2-butynyl)cyclohexyl]-1,2,4-oxadiazole;
5-amine-2-(1,1-dimethyl-3-chloropropyl)-1,3,4-oxadiazole;
3-amine-5-(1,1-diethylheptyl)-1,2,4-oxadiazole;
2-amino-5-(1-ethyl-1-methyl-6-*n*-butylthiohexyl)-1,3,4-triazole;
5-amino-3-(1,1-diethyl-2-methylhexyl)isothiazole;
5-amino-3-*tert.*-butylisothiazole;
5-amino-3-(1-*n*-propylcycloheptyl)isothiazole;
5-amino-3-[1,1-diethyl-4-(3-methylcyclopentyl)butyl]isothiazole;
3-amino-5-(1,1-diethylhexyl)isothiazole;
3-amino-5-[1-ethyl-2-methyl-4-(3-phenoxypropyl)octyl]isothiazole;
3-amino-5-(1,2,3-trimethyl-5-chloroheptyl)isothiazole;
3-amino-5-(1,1-diethyl-5-*n*-butylthiopentyl)isothiazole;
5-amino-3-(1,1-di-*n*-propylhexyl)pyrazole;
5-amino-3-*tert.*-butylpyrazole;
5-amino-3-(1-ethyl-1-methylpropyl)pyrazole;
5-amino-3-(1,1-diethyl-4-pentenyl)pyrazole;
5-amino-3-[1,1-dimethyl-4-(3-phenylpropoxy)butyl]pyrazole;
5-amino-3-(2,2-diethyldecyl)pyrazole;
3-amino-5-(1-ethyl-4,4-dimethyl-5-chloropentyl)pyrazole;
3-amino-5-(1-ethyl-1-methylpropyl)pyrazole;
3-amino-5-(1,1-dimethyl-6-*n*-butoxyphenyl)pyrazole;
2-amino-5-(1-ethyl-1-methylpropyl)-imidazole;
2-amino-5-[1-(2-propenyl)-1-(2-propynyl)-4-hexenyl]-imidazole;
2-amino-5-[1-ethyl-4-phenylbutyl)imidazole;

3-amino-5-(1,1-dibutylhexyl)pyrazole;
3-amino-5-(1,1-diethyl-4-cyclohexylbutyl)pyrazole;
3-amino-6-(1-ethyl-1-methylpropyl)pyridazine; and
3-amino-6-(1-ethylcyclohexyl)pyridazine.

A particularly preferred class of benzamide derivatives of formula (I) is represented by the formula:

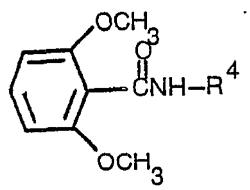

in which $R^4$ is

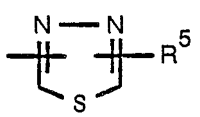 , 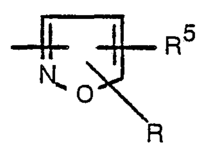 or 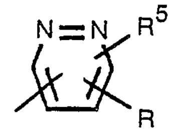

where R is hydrogen and wherein $R^5$ is

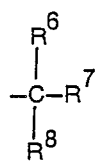 or 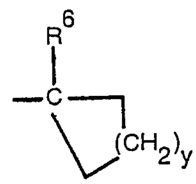

in which $R^6$ is $C_1$—$C_4$ alkyl, $R^7$ is hydrogen or $C_1$—$C_4$ alkyl, $R^8$ is $C_1$—$C_{13}$ alkyl and y is an integer from zero to four.

Among the most preferred compounds falling within this preferred class are:
N-[3-(1-ethyl-1-methylpropyl)-5-isoxazolyl]-2,6-dimethoxybenzamide;
N-[3-(*tert.*-butyl)-5-isoxazolyl]-2,6-dimethoxybenzamide;
N-[5-(1-ethyl-1-methylpropyl)-1,3,4-thiadiazol-2-yl]-2,6-dimethoxybenzamide;
N-[5-(1,1-diethylpropyl)-1,3,4-thiadiazol-2-yl]-2,6-dimethoxybenzamide;
N-[3-(1,1-dimethylpropyl)-5-isoxazolyl]-2,6-dimethoxybenzamide;
N-[3-(1,1-diethylhexyl)-5-isoxazolyl]-2,6-dimethoxybenzamide;
N-[5-(1-ethylheptyl)-1,3,4-thiadiazol-2yl]-2,6-dimethoxybenzamide;
N-[3-(1,1-diethylheptyl)-5-isoxazolyl]-2,6-dimethoxybenzamide;
N-[3-(1-ethyl-1-methylbutyl)-5-isoxazolyl]-2,6-dimethoxybenzamide;
N-[3-(1,1-diethylbutyl)-5-isoxazolyl-2,6-dimethoxybenzamide;
N-[5-(1-methylbutyl)-1,3,4-thiadiazol-2-yl]-2,6-dimethoxybenzamide;
N-[5-(1-ethyl-1-methylhexyl)-1,3,4-thiadiazol-2-yl]-2,6-dimethoxybenzamide;
N-[3-(1,1-dimethyltetradecyl)-5-isoxazolyl]-2,6-dimethoxybenzamide;
N-[5-(1,2-dimethylbutyl)-1,3,4-thiadiazol-2-yl]-2,6-dimethoxybenzamide;
N-[5-(1,1-diethylhexyl)-3-isoxazolyl]-2,6-dimethoxybenzamide;
N-[5-(1,1,2-trimethylbutyl)-1,3,4-thiadiazol-2-yl]-2,6-dimethoxybenzamide;
N-[3-(1,1,2,2-tetramethylbutyl)-5-isoxazolyl]-2,6-dimethoxybenzamide;
N-[3-(1-ethylhexyl)-5-isoxazolyl]-2,6-dimethoxybenzamide;
N-[5-(1-ethyl-1-methylpentyl)-3-isoxazolyl]-2,6-dimethoxybenzamide;
N-[5-(1,1-diethyldecyl)-1,3,4-thiadiazol-2-yl]-2,6-dimethoxybenzamide;
N-[5-(1-*iso*propylcyclohexyl)-3-isoxazolyl]-2,6-dimethoxybenzamide;
N-[5-(1-methylcyclopropyl)-1,3,4-thiadiazol-2-yl]-2,6-dimethoxybenzamide;
N-[5-(1-ethylcycloheptyl)-1,3,4-thiadiazol-2-yl]-2,6-dimethoxybenzamide;
N-[3-(1-*iso*propylcyclopentyl)-5-isoxazolyl]-2,6-dimethoxybenzamide;

Other specific compounds embraced by this invention include:
N-[5-(1,1-diethyl-2-methylthioethyl)-1,3,4-thiadiazol-2-yl]-2,6-diethoxybenzamide;
N-[5-(2,2-diethylbutyl)-1,3,4-thiadiazol-2-yl]-2,6-diethoxybenzamide;
N-[5-(1,2-diethyl-3-isopropoxypropyl]-1,3,4-thiadiazol-2-yl]-2,6-dimethoxybenzamide;

N-[3-(1,1-dimethyl-2-cycloheptylethyl)-5-isoxazolyl]-2,6-di-*n*-propoxybenzamide;
N-[3-(2,2-diethylhexyl)-5-isoxazolyl]-2,6-dimethoxybenzamide;
N-[5-(1,1-diethyl-2-phenylethyl)-1,3,4-thiadiazol-2-yl]-2,6-dimethoxybenzamide;
N-[3-(1,1-diethylhexyl)-5-isoxazolyl]-2,6-diethoxybenzamide;
N-[5-(1,1-diethyl-5-fluoropentyl]-1,3,4-thiadiazol-2-yl]-2,6-di-*n*-propoxybenzamide;
N-[3-(1,1-diethyl-2-methoxyethyl)-5-isoxazolyl]-2,6-dimethoxybenzamide;
N-[5-(1,1-diethylpentyl)-4H-1,2,4-triazol-3-yl]-2,6-dimethoxybenzamide;
N-[5-(1-ethyl-1-methylbutyl)-1,3,4-thiadiazol-2-yl]-2,6-diethoxybenzamide;
N-[3-(1-ethyl-1-phenylethyl)-5-isoxazolyl]-2,6-diethoxybenzamide;
N-[5-(1-ethyl-1-methyl-3-chloropropyl)-1,3,4-thiadiazol-2-yl]-4-methoxybenzamide;
N-[5-(1-ethyl-1-methyl-3-methoxypropyl)-1,3,4-thiadiazol-2-yl]-2,4,6-triethoxybenzamide;
N-[3-(1-methyl-1-benzyloxyethyl)-5-isoxazolyl]-2,6-dimethoxybenzamide;
N-[3-(1,1-di-*n*-propyl-5-phenylpentyl)-5-isoxazolyl]-2,6-diethoxybenzamide;
N-[5-(1-ethylcyclobutyl)-1,3,4-thiadiazol-2-yl]-2,6-diethoxybenzamide;
N-[3-(1,1-diethyl-3-butenyl)-5-isoxazolyl]-2,6-diethoxybenzamide;
N-[5-(1-methylcyclopropyl)-1,3,4-thiadiazol-2-yl]-2,6-diethoxybenzamide;
N-[5-(1-ethylcycloheptyl)-1,3,4-thiadiazol-2-yl]-2,6-di-*n*-propoxybenzamide;
N-[3-(1,1-diethyl-5-chloropentyl)-5-isoxazolyl]-2,6-dimethoxybenzamide;
N-[3-(1-*n*-propylcyclobutyl)-5-isoxazolyl]-2,6-diethoxybenzamide;
N-[3-(1-*iso*propylcyclopentyl)-5-isoxazolyl]-2-ethylthio-6-methoxybenzamide;
N-[3-(2-methyl-3-(2-methyl-1-propenyl)cyclobutyl)-5-isoxazolyl]-2,6-diethoxybenzamide;
N-[5-(1,1-dimethyl-3-pentenyl)-1,3,4-thiadiazol-2-yl]-2-methoxy-6-*n*-propoxybenzamide;
N-[3-(1,1-dimethylpropyl)-1H-pyrazol-5-yl]-2,6-diethoxybenzamide;
N-[3-(1-ethyl-1-methylpropyl)-5-isoxazolyl]-2-fluoro-6-ethoxybenzamide;
N-[3-(1,1-di-*n*-propylpentyl)-1H-pyrazol-5-yl-2-methoxy-6-ethoxybenzamide;
N-[5-(1,1-dimethylpropyl)-1,3,4-thiadiazol-2-yl]benzamide;
N-[5-(1-ethylcyclopentyl)-1,3,4-thiadiazol-2-yl]-2,6-diethoxybenzamide;
N-[5-(1-methyl-1-*n*-propylpentyl)-1,3,4-thiadiazol-2-yl]-2-methyl-6-methylthiobenzamide;
N-[3-(1-ethyl-1-methylbutyl)-5-isoxazolyl]-2-bromo-6-methoxybenzamide;
N-[5-(1-ethyl-1-methyldecyl)-1,2,4-thiadiazol-2-yl]-2,6-dimethoxy-4-trifluoromethylbenzamide;
N-[3-(*tert.*-butyl)-5-isothiazolyl]-2,6-diethoxybenzamide;
N-[3-(1,1-diethylpentyl)-5-isoxazolyl]-2,4,6-trimethylbenzamide;
N-[3-(1,1-diethylhexyl)-5-isoxazolyl]-4-iodobenzamide;
N-[3-(1,2,2-trimethyl-1-ethylpropyl)-1,2,4-thiadiazol-5-yl]-3-ethylthio-6-methoxybenzamide;
N-[3-(1-ethyl-1-methylpropyl)-1,2,4-thiadiazol-5-yl]-2,6-di-*n*-propoxybenzamide;
N-[3-(1-ethyl-1-*n*-propylhexyl)-1,2,4-oxadiazol-5-yl]-2,6-idethoxybenzamide;
N-[3-(1-ethylcycloheptyl)-1,2,4-oxadiazol-5-yl]-2,4,5-trimethoxybenzamide;
N-[3-(1-ethyl-3-phenylpropyl)-1,2,4-oxadiazol-5-yl]-2,6-di(methylthio)benzamide;
N-[3-(1-methyl-1-ethyl-3-hexynyl)-1,2,4-oxadiazol-5-yl]-2,6-dimethoxybenzamide;
N-[5(4)-(1-ethyl-1-methylpropyl)-2-imidazolyl]-2,6-dimethoxybenzamide;
N-[5(4)-(1,1-dimethyl-5-phenylpentyl)-2-imidazolyl]-2,6-di(ethylthio)benzamide;
N-[5(4)-(2,2-diethyl-2-cyclobutylethyl)2-imidazolyl]-2,6-dimethoxy-4-(trifluoromethyl)benzamide;
N-[5-(4)-(1-(3-butenyl)cyclohexyl)-2-imidazolyl]-2-chloro-4-methoxy-6-ethoxybenzamide;
N-[6-(1-ethyl-1-methylpropyl)pyridazin-3-yl]-2,6-di-*n*-propoxybenzamide; and
N-[6-(1-ethyl-3-cycloheptylpropyl)pyridazin-3-yl]-2,6-di-*n*-butoxybenzamide.

In certain aspects of the invention it may be desired to increase the water solubility of the benzamide derivatives by forming agronomically-acceptable salts. The nature of such salts will be immediately apparent to those skilled in the art, and it would be otiose to list them here. Suffice it to say, that Group IA alkali metal, particularly sodium, salts are prepared. They may be prepared by reacting a benzamide derivative of formula (I) with a strong base such as sodium hydride in an ethereal solvent such as tetrahydrofuran or diethyl ether. The salification may be accomplished at temperatures between 0 and 50°C, conveniently at room temperature.

The preparation of various benzamide derivatives provided by the invention is illustrated by the following detailed Examples.

### Example 1

N-[3-(1-Ethyl-1-methylpropyl)-5-isoxazolyl]-2,6-dimethoxybenzamide

A) Preparation of 5-amino-3-(1-ethyl-1-methylpropyl)isoxazole

A 16.5 Kg portion of methyl 2-ethylbutyrate was reacted with 60 Kg of *n*-butyl lithium, diisopropylamine, and 19.1 Kg of methyl iodide to provide 17.4 Kg of methyl 2-ethyl-2-methylbutyrate. A 7.5 Kg portion of the ester thus formed was reacted with 3.25 Kg of acetonitrile and 5.03 g of sodium hydride in 33 liters of tetrahydrofuran to provide 1-ethyl-1-methylpropyl cyanomethyl ketone. The ketone thus formed was reacted with 4.35 Kg of hydroxylamine hydrochloride and 2.54 Kg of sodium hydroxide in 44 liters of water to provide 5.65 Kg of 5-amino-3-(1-ethyl-1-methylpropyl)isoxazole.

B) Preparation of 2,6-dimethoxybenzoyl chloride

A 8.5 Kg portion of 2,6-dimethoxybenzoic acid was dissolved in 60 liters of toluene and the solution was stirred at ambient temperature while 6.8 liters of thionyl chloride was added dropwise over forty-five minutes. Following the addition, the reaction mixture next was cooled to room temperature and the solvent was removed by evaporation under reduced pressure, washed with 25 liters of Pet. ether cooled and filtered to provide a crude solid product. The product was stirred for one hour with 25 liters of fresh Pet. ether, and then cooled to 10°C. and filtered to provide 8.94 Kg of 2,6-dimethoxybenzoyl chloride.

C) Synthesis of N-[3-(1-ethyl-1-methylpropyl)-5-isoxazolyl]-2,6-dimethoxybenzamide

To a stirred solution of 3.36 Kg of 5-amino-3-(1-ethyl-1-methylpropyl)isoxazole in 65 liters of toluene was added portion-wise over thirty minutes 4.015 Kg of 2,6-dimethoxybenzoyl chloride. The reaction mxiture was heated to reflux and stirred for forty-eight hours. It was then cooled to room temperature and concentrated to a volume of about 25 liters by evaporation of the solvent under reduced pressure. The product precipitated and was collected by filtration, washed with fresh toluene, and air dried to provide 6.084 Kg of N-[3-(1-ethyl-1-methylpropyl)-5-isoxazolyl]-2,6-dimethoxybenzamide. M.P. 172—174°C. Yield 91%.

Analysis calculated for $C_{18}H_{24}N_2O_4$

| | | | |
|---|---|---|---|
| Theory: | C, 65.04; | H, 7.28; | N, 8.43; |
| Found: | C, 64.79; | H, 7.02; | N, 8.28. |

Example 2

N-[3-(1,1-dimethylethyl)-5-isoxazolyl]-2,6-di-*n*-propoxybenzamide

A) Preparation of 2,6-di-*n*-propoxybenzoyl chloride

Methyl 2,6-dihydroxybenzoate was reacted with *n*-propyl iodide in the presence of sodium hydride to provide methyl 2,6-di-*n*-propoxybenzoate. The ester thus formed was saponified by reaction with 40% aqueous potassium hydroxide in ethanol to afford 2,6-di-*n*-propoxybenzoic acid. A 20 g portion of the acid was then reacted with 30 g of thionyl chloride in 100 ml of benzene at reflux for five hours. Removal of the solvent by evaporation under reduced pressure and purification of the product by distillation provided 4.88 g of 2,6-di-*n*-propoxybenzoyl chloride. B.P. 135—140°C at 0.2 torr (26.6 Pa).

B) To a stirred solution of 2.8 g of 5-amino-3-(1,1-dimethylethyl)isoxazole in 40 ml of tetrahydrofuran containing 4.5 ml of triethylamine was added dropwise over ten minutes a solution of 4.88 g of 2,6-di-*n*-propoxybenzoyl chloride in 10 ml of tetrahydrofuran. Following the addition, the reaction mixture was heated at reflux for seventy-two hours. The reaction mixture was then cooled to room temperature and the solvent was removed by evaporation under reduced pressure. The residue was dissolved in a mixture of dichloromethane and water, and the organic layer was separated, washed with fresh water, dried, and the solvent was removed by evaportion to provide an oil. The oil was crystallized from Skelly B and diethyl ether to provide 750 mg of N-[3-(1,1-dimethylethyl)-5-isoxazolyl]-2,6-di-*n*-propoxybenzamide. M.P. 85—87°C. Yield 10%.

Analysis calculated for $C_{20}H_{28}N_2O_4$

| | | | |
|---|---|---|---|
| Theory: | C, 66.64; | H, 7.83; | N, 7.77. |
| Found: | C, 67.65; | H, 7.78; | N, 7.32. |

Example 3

N-[5-(1-ethylcyclohexyl)-3-isoxazolyl]-2,6-dimethoxybenzamide

A) Preparation of 3-amino-5-(1-ethylcyclohexyl)isoxazole

Acetonitrile was reacted with 1-ethyl-1-methoxycarbonylcyclohexane in the presence of sodium hydride to provide 1-ethyl-1-(2-cyanoacetyl)cyclohexane. A 55 g portion of the latter compound was dissolved in 200 ml of diethyl ether containing 20.5 g of absolute methanol, and the solution was stirred and cooled to about 5°C. Hydrogen chloride gas was next bubbled through the reaction mixture for forty-five minutes, after which time the reaction mixture was stored at 0°C for twelve hours. Removal of the reaction solvent by evaporation under reduced pressure provided a yellow solid, which was dissolved in 300 ml of fresh absolute methanol and treated with 97 g of triethylamine and 22 g of hydroxylamine hydrochloride. The reaction mixture was heated at 50°C for three hours, and then cooled to room temperature and diluted with 25 ml of concentrated hydrochloric acid. The acidic reaction mixture was heated at 50°C for twelve hours, and then cooled to room temperature and concentrated to dryness by evaporation of the solvent under reduced pressure. The residue thus obtained was dissolved in water, and the aqueous mixture was made alkaline by the addition of 20% sodium hydroxide. The product was extracted into diethyl ether, which was then washed with water, dried, and the solvent was removed by evaporation. Distillation of the product afforded 14 g of 3-amino-5-(1-ethylcyclohexyl)isoxazole. B.P. 135—140°C at 0.1—0.05 torr (13.3—6.65 Pa).

B) A solution of 14 g of 3-amino-5-(1-ethylcyclohexyl)isoxazole and 14.4 g of 2,6-dimethoxybenzoyl chloride in 100 ml of toluene was heated to reflux and stirred for eighteen hours. The reaction mixture then was cooled to room temperature and the solvent was removed by evaporation under reduced pressure to provide a solid. The solid was dissolved in 200 ml of dichloromethane, washed with dilute sodium hydroxide and with brine, dried, and the solvent was removed by evaporation. The product was crystallized from dichloromethane and diethyl ether to provide 15.5 g of N-[5-(1-ethylcyclohexyl)-3-isoxazolyl]-2,6-dimethoxybenzamide. M.P. 179—181°C. Yield 60%.

Analysis calculated for $C_{20}H_{26}N_2O_4$
Theory: C, 67.02; H, 7.31; N, 7.82;
Found: C, 66.82; H, 7.02; N, 7.54.

The following exemplary compounds were prepared by reacting an amino-isoxazole with a 2,6-dialkoxy benzoyl halide derivative according to the general methods of Examples 1—3 to provide the corresponding N-isoxazolyl-2,6-dialkyoxybenzamide.

Example 4

N-[3-(1,1-dimethyl-2-chloroethyl)-5-isoxazolyl]-2,6-dimethoxybenzamide

M.P. 172—173°C. Yield 33%.
Analysis calculated for $C_{16}H_{19}ClN_2O_4$
Theory: C, 56.72; H, 5.65; N, 8.27; Cl, 10.46;
Found: C, 56.49; H, 5.66; N, 8.08; Cl, 10.52.

Example 5

N-[3-(2,2-dimethylpropyl)-5-isoxazolyl]-2,6-dimethoxybenzamide

M.P. 152—154°C. Yield 24%.
Analysis calculated for $C_{17}H_{22}N_2O_4$
Theory: C, 64.13; H, 6.97; N, 8.80;
Found: C, 64.07; H, 6.76; N, 8.62.

Example 6

N-[3-(1,1-dimethylethyl)-5-isoxazolyl]-2,6-dimethoxybenzamide

M.P. 172—173°C. Yield 66%.
Analysis calculated for $C_{16}H_{20}N_2O_4$
Theory: C, 63.14; H, 6.62; N, 9.20;
Found: C, 62.90; H, 6.52; N, 8.94.

Example 7

N-[5-(1,1-dimethylethyl)-3-isoxazolyl]-2,6-dimethoxybenzamide

M.P. 182—183°C. Yield 27%.
Analysis calculated for $C_{16}H_{20}N_2O_4$
Theory: C, 63.14; H, 6.62; N, 9.20;
Found: C, 63.14; H, 6.64; N, 9.07.

Example 8

N-[3-(1,1-dimethylethyl)-5-isoxazolyl]-2-methoxy-6-*iso*propoxybenzamide

M.P. 126—128°C. Yield 3.5%.
Analysis calculated for $C_{18}H_{24}N_2O_4$
Theory: C, 65.24; H, 7.00; N, 8.45;
Found: C, 65.00; H, 6.80; N, 8.39.

Example 9

N-[3-(1,1-dimethylpropyl)-5-isoxazolyl]-2,6-dimethoxybenzamide

M.P. 140—142°C. Yield 50%.
Analysis calculated for $C_{17}H_{22}N_2O_4$
Theory: C, 64.13; H, 6.97; N, 8.80;
Found: C, 63.86; H, 6.71; N, 9.05.

Example 10

N-[3-(1,1-dimethylpentyl)-5-isoxazolyl]-2,6-dimethoxybenzamide

M.P. 132—133°C. Yield 31%.
Analysis calculated for $C_{19}H_{26}N_2O_4$
Theory: C, 65.88; H, 7.57; N, 8.09;
Found: C, 65.88; H, 7.42; N, 7.86.

Example 11

N-[3-(2-cyclohexyl-1,1-dimethylethyl)-5-isoxazolyl]-2,6-dimethoxybenzamide

M.P. 146—148°C. Yield 21%.
Analysis calculated for $C_{22}H_{30}N_2O_4$
Theory: C, 68;37; H, 7.82; N, 7.25;
Found: C, 68.12; H, 7.57; N, 6.99.

Example 12

N-[3-(1-cyclohexyl-1-methylethyl)-5-isoxazolyl]-2,6-dimethoxybenzamide

M.P. 158—160°C. Yield 91%.
Analysis calculated for $C_{21}H_{28}N_2O_4$
Theory: C, 67.72; H, 7.58; N, 7.52;
Found: C, 67.56; H, 7.37; N, 7.56.

Example 13

N-[3-(1,1-dimethyl-2-phenylethyl)-5-isoxazolyl]-2,6-dimethoxybenzamide

M.P. 108—110°C. Yield 9%.
Analysis calculated for $C_{22}H_{25}N_2O_4$
Theory: C, 69.46; H, 6.36; N, 7.36;
Found: C, 69.28; H, 6.53; N, 7.12.

Example 14

N-[3-(1-ethyl-1-methylbutyl)-5-isoxazolyl]-2,6-dimethoxybenzamide

M.P. 149—151°C. Yield 41%.
Analysis calculated for $C_{19}H_{26}N_2O_4$
Theory: C, 65.88; H, 7.57; N, 8.09;
Found: C, 65.59; H, 7.35; N, 7.87.

Example 15

N-[3-(1,1-diethylpropyl)-5-isoxazolyl]-2,6-dimethoxybenzamide

M.P. 163—165°C. Yield 13%.

Example 16

N-[3-(1,1-dimethyl-3-butenyl)-5-isoxazolyl]-2,6-dimethoxybenzamide

M.P. 160—162°C. Yield 22%.
Analysis calculated for $C_{18}H_{22}N_2O_4$
Theory: C, 65.44; H, 6.71; N, 8.48;
Found: C, 65.23; H, 6.50; N, 8.39.

Example 17

N-[3-(1-methylcyclohexyl)-5-isoxazolyl]-2,6-dimethoxybenzamide

M.P. 161—163°C. Yield 54%.
Analysis calculated for $C_{19}H_{24}N_2O_4$
Theory: C, 66.26; H, 7.02; N, 8.13;
Found: C, 66.06; H, 6.80; N, 8.28.

Example 18

N-[3-(1,1-dimethyltetradecyl)-5-isoxazolyl]-2,6-dimethoxybenzamide

M.P. 57—59°C. Yield 9%.
Analysis calculated for $C_{28}H_{44}N_2O_4$
Theory: C, 71.15; H, 9.38; N, 5.93;
Found: C, 71.34; H, 9.15; N, 5.81.

Example 19

N-[3-(1-ethylcyclohexyl)-5-isoxazolyl]-2,6-dimethoxybenzamide

M.P. 177—179°C. Yield 34%.
Analysis calculated for $C_{20}H_{26}N_2O_4$
Theory: C, 67.02; H, 7.31; N, 7.82;
Found: C, 66.74; H, 7.07; N, 7.90.

Example 20

N-[3-(1,1,3-trimethylbutyl)-5-isoxazolyl]-2,6-dimethoxybenzamide

M.P. 146—148°C. Yield 12%.

Analysis calculated for $C_{19}H_{26}N_2O_4$

Theory: C, 65.88; H, 7.57; N, 8.09;

Found: C, 65.70; H, 7.50; N, 7.87.

Example 21

N-[3-(1-methylcyclopentyl)-5-isoxazolyl]-2,6-dimethoxybenzamide

M.P. 128—130°C. Yield 10%

Analysis calculated for $C_{18}H_{22}N_2O_4$

Theory: C, 65.44; H, 6.71; N, 8.48;

Found: C, 65.24; H, 6.59; N, 8.22.

Example 22

N-[5-(1,1-dimethylbutyl)-3-isoxazolyl]-2,6-dimethoxybenzamide

M.P. 133—135°C. Yield 48%

Analysis calculated for $C_{18}H_{24}N_2O_4$

Theory: C, 65.04; H, 7.28; N, 8.43;

Found: C, 65.25; H, 7.01; N, 8.19.

Example 23

N-[3-(1,1,2,2-tetramethylpropyl)-5-isoxazolyl]-2,6-dimethoxybenzamide

M.P. 174—175°C.

Analysis calculated for $C_{19}H_{26}N_2O_4$

Theory: C, 65.88; H, 7.57; N, 8.09;

Found: C, 65.97; H, 7.32; N, 8.33.

Example 24

N-[3-(1-ethylpropyl)-5-isoxazolyl]-2,6-dimethoxybenzamide

M.P. 157—159°C. Yield 53%.

Analysis calculated for $C_{17}H_{22}N_2O_4$

Theory: C, 64.13; H, 6.97; N, 8.80;

Found: C, 63.87; H, 6.77; N, 8.56.

Example 25

N-[5-(1-ethyl-1-methylpropyl)-3-isoxazolyl]-2,6-dimethoxybenzamide

M.P. 165—166°C. Yield 49%.

Analysis calculated for $C_{18}H_{24}N_2O_4$

Theory: C, 65.04; H, 7.28; N, 8.43;

Found: C, 64.94; H, 7.01; N, 8.21.

Example 26

N-[3-(1,1-dimethylethyl)-5-isoxazolyl]-2,6-dimethoxybenzamide

M.P. 123—125°C. Yield 11%.

Analysis calculated for $C_{18}H_{24}N_2O_4$

Theory: C, 65.04; H, 7.28; N, 8.43;

Found: C, 64.50; H, 7.04; N, 7.89.

Example 27

N-[3-(2-methoxy-1,1-dimethylethyl)-5-isoxazolyl]-2,6-dimethoxybenzamide

M.P. 201—203°C. Yield 19%.

Analysis calculated for $C_{17}H_{22}N_2O_5$

Theory: C, 61.06; H, 6.63; N, 8.38;

Found: C, 61.50; H, 6.36; N, 8.51.

Example 28

N-[3-(1,1-dimethylbutyl)-5-isoxazolyl]-2,6-dimethoxybenzamide

M.P. 141—143°C. Yield 9%.

Analysis calculated for $C_{18}H_{24}N_2O_4$

Theory: C, 65.04; H, 7.28; N, 8.43;

Found: C, 64.79; H, 7.04; N, 8.26.

Example 29

N-[3-(1-propylcyclohexyl)-5-isoxazolyl]-2,6-dimethoxybenzamide

M.P. 202—204°C. Yield 38%.

Analysis calculated for $C_{21}H_{28}N_2O_4$

Theory: C, 67.72; H, 7.58; N, 7.52;

Found: C, 67.48; H, 7.58; N, 7.56.

Example 30

N-[3-(1-methyl-1-(2,4-dichlorophenoxy)ethyl)-5-isoxazolyl]-2,6-dimethoxybenzamide

M.P. 154—156°C. Yield 25%.

Analysis calculated for $C_{21}H_{20}Cl_2N_2O_5$

Theory: C, 55.89; H, 4.47; N, 6.21; Cl, 15.71;

Found: C, 56.09; H, 4.46; N, 6.01; Cl, 15.45.

Example 31

N-[3-(1-methyl-1-phenylethyl)-5-isoxazolyl]-2,6-dimethoxybenzamide

M.P. 185—187°C. Yield 30%.

Analysis calculated for $C_{21}H_{22}N_2O_4$

Theory: C, 68.84; H, 6.05; N, 7.65;

Found: C, 69.04; H, 5.93; N, 7.44.

Example 32

N-[3-(1-methyl-1-phenylpropyl)-5-isoxazolyl]-2,6-dimethoxybenzamide

M.P. 183—185°C. Yield 29%.

Analysis calculated for $C_{22}H_{24}N_2O_4$

Theory: C, 69.46; H, 6.36; N, 7.36;

Found: C, 69.26; H, 6.13; N, 7.54.

Example 33

N-[3-(1-methyl-1-(phenylmethoxy)ethyl]-5-isoxazolyl-2,6-dimethoxybenzamide

M.P. 123—125°C. Yield 13%.

Analysis calculated for $C_{22}H_{24}N_2O_5$

Theory: C, 66.65; H, 6.10; N, 7.07;

Found: C, 66.84; H, 5.88; N, 6.86.

Example 34

N-[3-(1-ethylcycloheptyl)-5-isoxazolyl]-2,6-dimethoxybenzamide

M.P. 163—165°C. Yield 32%.

Analysis calculated for $C_{21}H_{28}N_2O_4$

Theory: C, 67.72; H, 7.58; N, 7.52;

Found: C, 67.64; H, 7.78; N, 7.25.

Example 35

N-[3-(1-cyclohexyl-1-methylpropyl)-5-isoxazolyl]-2,6-dimethoxybenzamide

M.P. 173—175°C. Yield 22%.

Analysis calculated for $C_{22}H_{30}N_2O_4$

Theory: C, 68.37; H, 7.82; N, 7.25;

Found: C, 68.29; H, 7.53; N, 7.27.

Example 36

N-[3-(1-methoxy-1-methylethyl)-5-isoxazolyl]-2,6-dimethoxybenzamide

M.P. 232—234°C. Yield 37%.

Analysis calculated for $C_{16}H_{20}N_2O_5$

Theory: C, 59.99; H, 6.29; N, 8.74;

Found: C, 59.87; H, 6.07; N, 8.73.

By following the general procedures of Examples 1—3, an amino-isoxazole was reacted with suitably substituted benzoyl halide to give the following N-isoxazolyl benzamides.

Example 37

N-[3-(1-ethyl-1-methylpropyl)-5-isoxazolyl]-2-fluoro-6-methoxybenzamide

M.P. 133—135°C. Yield 26%.

Analysis calculated for $C_{17}H_{21}N_2O_3F$

Theory: C, 63.74; H, 6.61; N, 8.74;

Found: C, 63.97; H, 6.48; N, 8.70.

Example 38
N-[5-(1,1-dimethylethyl)-3-isoxazolyl]-2,6-dichlorobenzamide
M.P. 249—250°C.
Analysis calculated for $C_{14}H_{14}Cl_2N_2O_2$
Theory: C, 53.69; H, 4.51; N, 8.94;
Found: C, 53.89; H, 4.54; N, 8.77.

Example 39
N-[3-(1,1-dimethylethyl)-5-isoxazolyl]-2,6-dichlorobenzamide
M.P. 235—237°C. Yield 22%.
Analysis calculated for $C_{14}H_{14}Cl_2N_2O_2$
Theory: C, 53.69; H, 4.51; N, 8.94; Cl, 22.64;
Found: C, 53.74; H, 4.56; N, 8.96; Cl, 22.83.

Example 40
N-[3-(1,1-dimethylethyl)-5-isoxazolyl]-2,6-difluorobenzamide
M.P. 153—154°C.
Analysis calculated for $C_{14}H_{14}F_2N_2O_2$
Theory: C, 60.00; H, 5.04; N, 10.00;
Found: C, 59.91; H, 4.83; N, 10.16.

Example 41
N-[3-(1-ethyl-1-methylpropyl)-5-isoxazolyl]-2-chloro-6-methoxybenzamide
M.P. 173—174°C. Yield 42%.
Analysis calculated for $C_{17}H_{21}ClN_2O_3$
Theory: C, 60.62; H, 6.28; N, 8.32;
Found: C, 60.74; H, 6.02; N, 8.54.

Example 42
N-[3-(1-ethyl-1-methylpropyl)-5-isoxazolyl]-2,4,6-trimethoxybenzamide
M.P. 150—152°C. Yield 40%.
Analysis calculated for $C_{19}H_{26}N_2O_5$
Theory: C, 62.97; H, 7.23; N, 7.73;
Found: C, 63.01; H, 7.05; N, 7.72.

Example 43
N-[5-(1-ethyl-1-methylpropyl)-3-isoxazolyl]-2,4,6-trimethoxybenzamide
M.P. 165—170°C. Yield 41%.
Analysis calculated for $C_{19}H_{26}N_2O_5$
Theory: C, 62.97; H, 7.23; N, 7.73;
Found: C, 62.94; H, 6.99; N, 7.94.

Example 44
N-[3-(1-ethyl-1-methoxymethylpropyl)-5-isoxazolyl]-2,6-dimethoxybenzamide

Example 45
N-[3-(1,1-diethyl-2-propenyl)-5-isoxazolyl]-2,6-dimethoxybenzamide

Example 46
N-[3-(1,1-dimethylethyl)-5-isoxazolyl]-2,4-dimethoxybenzamide
M.P. 166—168°C. Yield 53%.
Analysis calculated for $C_{16}H_{20}N_2O_4$
Theory: C, 63.14; H, 6.62; N, 9.20;
Found: C, 63.38; H, 6.71; N, 9.01.

Example 47
N-[3-(1,1-dimethylethyl)-5-isoxazolyl]-3,5-dimethylbenzamide
M.P. 121—123°C. Yield 45%.
Analysis calculated for $C_{16}H_{20}N_2O_2$
Theory: C, 70.56; H, 7.40; N, 10.29;
Found: C, 70.82; H, 7.25; N, 10.21.

Example 48

N-[3-propyl-5-isoxazolyl]-2,6-dimethoxybenzamide

M.P. 124—126°C. Yield 29%.

Analysis calculated for $C_{15}H_{18}N_2O_4$

Theory: C, 62.06; H, 6.25; N, 9.65;

Found: C, 62.34; H, 6.46; N, 9.55.

Example 49

N-(3-propyl-5-isoxazolyl)-2,6-dimethylbenzamide

M.P. 120—122°C.

Analysis calculated for $C_{15}H_{18}N_2O_2$

Theory: C, 69.74; H, 7.02; N, 10.84;

Found: C, 69.98; H, 6.82; N, 10.58.

Example 50

N-[3-(1,1-dimethylethyl)-5-isoxazolyl]-3,4-dimethoxybenzamide

M.P. 164—166°C. Yield 37%.

Analysis calculated for $C_{16}H_{20}N_2O_4$

Theory: C, 63.14; H, 6.62; N, 9.20;

Found: C, 63.27; H, 6.41; N, 9.12.

Example 51

N-[3-(1,1-dimethylethyl)-5-isoxazolyl]-3,5-dimethoxybenzamide

M.P. 115—117°C. Yield 49%.

Analysis calculated for $C_{16}H_{20}N_2O_4$

Theory: C, 63.14; H, 6.62; N, 9.20;

Found: C, 63.40; H, 6.37; N, 9.30.

Example 52

N-[3-(1-methylethyl)-5-isoxazolyl]-2,6-dimethoxybenzamide

M.P. 143—144°C. Yield 52%.

Analysis calculated for $C_{15}H_{18}N_2O_4$

Theory: C, 62.49; H, 5.59; N, 9.72;

Found: C, 62.20; H, 5.46; N, 9.51.

Example 53

N-[3-(1,1-dimethylethyl)-5-isoxazolyl]-2,4,6-trimethylbenzamide

M.P. 177—179°C. Yield 22%.

Analysis calculated for $C_{17}H_{22}N_2O_2$

Theory: C, 71.30; H, 7.74; N, 9.78;

Found: C, 71.34; H, 7.45; N, 9.78.

Example 54

N-[3-(1-ethyl-1-(methoxymethyl)propyl)-5-isoxazolyl]-2,6-dimethoxybenzamide

M.P. 167—168°C. Yield 26%.

Example 55

N-[3-(1-ethyl-1-methylpentyl)-5-isoxazolyl]-2,6-dimethoxybenzamide

M.P. 150—152°C. Yield 34%.

Example 56

N-[3-(1,1-dimethylethyl)-5-isoxazolyl]-2,6-dimethylbenzamide

M.P. 179—181°C. Yield 32%.

Analysis calculated for $C_{16}H_{20}N_2O_2$

Theory: C, 70.56; H, 7.40; N, 10.29;

Found: C, 70.35; H, 7.19; N, 10.02.

Example 57

N-[3-(2,2-dimethyl-3-[2-methyl-1-propenyl]-cyclopropyl)-5-isoxazolyl]-2,6-dimethoxybenzamide

M.P. 92—94°C. Yield 8%.

Analysis calculated for $C_{21}H_{24}N_2O_4$

Theory: C, 68.09; H, 7.07; N, 7.56;

Found: C, 68.16; H, 6.83; N, 7.42.

Example 58

N-[3-(1-ethyl-1-methoxypropyl)-5-isoxazolyl]-2,6-dimethoxybenzamide

M.P. 174—176°C. Yield 7%.

Example 59

N-[3-(1,1-dimethylethyl)-5-isoxazolyl]-2,6-diethylbenzamide

M.P. 173—175°C. Yield 7%.

Analysis calculated for $C_{18}H_{24}N_2O_2$

| | | | |
|---|---|---|---|
| Theory: | C, 71.97; | H, 8.05; | N, 9.33; |
| Found: | C, 72.20; | H, 8.24; | N, 9.21. |

Example 60

N-[3-(1,1-dimethylethyl)-5-isoxazolyl]-2,4,6-trimethoxybenzamide

M.P. 115—118°C. Yield 26%.

Analysis calculated for $C_{17}H_{22}N_2O_5$

| | | | |
|---|---|---|---|
| Theory: | C, 61.07; | H, 6.63; | N, 8.38; |
| Found: | C, 60.88; | H, 6.76; | N, 8.12. |

Example 61

N-[5-(1-ethyl-1-methylpropyl)-1,3,4-thiadiazol-2-yl]-2,6-dimethoxybenzamide

A) Preparation of 2-amino-5-(1-ethyl-1-methylpropyl)-1,3,4-thiadiazole

A solution containing 13.0 g of 2-ethyl-2-methylbutyric acid and 9.1 g of thiosemicarbazide in 125 ml of dioxane was stirred and heated to 90°C. To the stirred reaction mixture was added dropwise over thirty minutes 15.3 g of phosphorous oxychloride. Following complete addition, the reaction mixture was heated at 90°C for six hours. The mixture was then cooled to about 30°C and added to 100 g of ice. The aqueous mixture was made alkaline by the addional ammonium hydroxide, and the alkaline solution was extracted several times with ethyl acetate. The extracts were combined, washed with water, dried, and the solvent was removed by evaporation under reduced pressure to afford 17.0 g of 2-amino-5-(1-ethyl-1-methylpropyl)-1,3,4-thiadiazole.

M.P. 138—140°C.

B) To a stirred solution of 9.2 g of the thiadiazole from above in 100 ml of tetrahydrofuran containing 4.0 g of pyridine were added in one portion 11.0 g of 2,6-dimethoxybenzoyl chloride. The reaction mixture was heated at reflux for three hours, and then cooled to about 30°C and filtered. The solvent was removed from the filtrate by evaporation under reduced pressure to provide a solid which, when crystallized from 2B ethanol afforded 6.3 g of N-[5-(1-ethyl-1-methylpropoyl)-1,3,4-thiadiazol-2-yl]-2,6-dimethoxybenzamide.

M.P. 208—210°C. Yield 36%.

Analysis calculated for $C_{17}H_{23}N_3O_3S$

| | | | |
|---|---|---|---|
| Theory: | C, 58.43; | H, 6.63; | N, 12.02; |
| Found: | C, 58.34; | H, 6.58; | N, 11.79. |

Example 62

N-[5-(1-ethyl-1-methylpropyl)-1,3,4-thiadiazol-2-yl]-benzamide

To a stirred suspension of 3.3 g of 2-amino-5-(1-ethyl-1-methylpropyl)-1,3,4-thiadiazole in 30 ml of tetrahydrofuran were added in one portion 2.8 g of benzoyl chloride. The reaction mixture was stirred at room temperature while a solution of 1.6 g of pyridine in 20 ml of tetrahydrofuran was added dropwise over thirty minutes. Following complete addition, the reaction mixture was heated at reflux for three hours. The mixture was then filtered to remove the pyridine hydrochloride, and the filtrate was washed several times with 1 N hydrochloric acid solution. The organic layer was separated and the solvent was removed by evaporation under reduced pressure to provide a yellow gum. The gum was crystallized from ethanol and water to afford 1.85 g of N-[5-(1-ethyl-1-methylpropyl)-1,3,4-thiadiazol-2-yl]benzamide.

M.P. 98—100°C. Yield 35%.

Analysis calculated for $C_{15}H_{19}N_3OS$

| | | | | |
|---|---|---|---|---|
| Theory: | C, 62.25; | H, 6.62; | N, 14.52; | S, 11.08; |
| Found: | C, 62.01; | H, 6.39; | N, 14.27; | S, 11.22. |

Example 63

N-[5-(1-ethyl-1-methylpropyl)-1,3,4-thiadiazol-2-yl]-2,6-diethylbenzamide

A) Preparation of 2,6-diethylbenzoyl chloride

2,6-Diethylcyanobenzene was prepared by converting 2,6-diethylaniline to a diazonium salt and then reacting the diazonium salt with copper cyanide. Hydrolysis of 2,6-diethylcyanobenzene was effected by reaction with sodium hydroxide in ethylene glycol to provide 2,6-diethylaminocarbonyl benzene. The latter compound was reacted with phosphoric acid to provide 2,6-diethylbenzoic acid. Reaction of the benzoic acid with thionyl chloride afforded 2,6-diethylbenzoylchloride as an oil.

B) A solution of 1.85 g of 2-amino-5-(1-ethyl-1-methylpropyl)-1,3,4-thiadiazole and 2.21 g of 2,6-diethylbenzoyl chloride in 50 ml of toluene was heated at reflux for sixteen hours and then cooled and the solvent was removed by evaporation under reduced pressure to provide a solid residue. The solid was crystallized from 2B ethanol to give 1.25 g of N-[5-(1-ethyl-1-methylpropyl)-1,3,4-thiadiazol-2-yl]-2,6-diethylbenzamide.

M.P. 186—188°C. Yield 36%.

Analysis calculated for $C_{19}H_{27}N_3OS$

| | | | | |
|---|---|---|---|---|
| Theory: | C, 66.05; | H, 7.88; | N, 12.16; | S, 9.28; |
| Found: | C, 66.18; | H, 7.82; | N, 11.87; | S, 9.16. |

Example 64

N-[5-(1,1-dimethyl-2-(methylthio)ethyl)-1,3,4-thiadiazol-2-yl]-2,6-dimethoxybenzamide

A) Preparation of 2-amino-5-[1,1-dimethyl-2-(methylthio)ethyl]-1,3,4-thiadiazole

Lithium diisopropylamide was prepared by reacting 51.0 g of diisopropylamine with 227 ml of *n*-butyl lithium in 350 ml of tetrahydrofuran at −5°C. To the stirred reaction mixture were added dropwise over thirty minutes 22.0 g of isobutyric acid. Following the addition, the reaction mixture was warmed to 25°C and stirred for one hour. The mixture was then again cooled to −5°C, and 24.1 g of chloromethyl methylsulfide were added dropwise. The reaction mixture was allowed to warm to 25°C and was stirred at that temperature for twelve hours. The excess solvent was next removed by evaporation under reduced pressure, and the residue was added to 50 g of ice containing 50 ml of 1 N hydrochloric acid solution. The aqueous acid mixture was extracted several times with diethyl ether, and the extracts were combined, washed with water, dried, and the solvent was removed by evaporation to give 22.0 g of 2,2-dimethyl-methylthiopropionic acid as an oil.

A 9.0 g portion of the acid thus formed was dissolved in 120 ml of dioxane containing 5.5 g of thiosemicarbazide, and the reaction mixture was heated at 90°C for thirty minutes, and then a 10.1 g portion of phosphorous oxychloride was added dropwise over ten minutes to the reaction mixture. Following complete addition, the mixture was heated at 90°C for twelve hours. After cooling the reaction mixture to room temperature, the solvent was decanted and the solid precipitate was dissolved in warm water. The aqueous mixture was made alkaline to pH 8 by the addition of ammonium hydroxide, and then the alkaline solution was extracted with ethyl acetate. The extracts were combined, washed with water, dried, and the solvent was removed by evaporation under reduced pressure to provide 4.2 g of 2-amino-5-[1,1-dimethyl-2-(methylthio)ethyl]-1,3,4-thiadiazole.

M.P. 117—120°C.

B) A 3.0 g portion of the thiadiazole thus prepared was reacted with 3.4 g of 2,6-dimethoxybenzoyl chloride in 30 ml of tetrahydrofuran containing 1.3 g of pyridine. The reaction was carried out at 106°C for sixteen hours. After cooling the reaction mixture to room temperature, the solvent was removed by evaporation under reduced pressure to leave the product as an oil. The oil was purified first by chromatography over silica gel using diethyl ether as an eluant, and then by crystallization from ethyl acetate to provide 1.94 g of N-[5-(1,1-dimethyl-2-(methylthio)ethyl]-1,3,4-thiadiazol-2-yl]-2,6-dimethoxybenzamide.

M.P. 165—167°C. Yield 35%.

Analysis calculated for $C_{16}H_{21}N_3O_3S_2$

| | | | | |
|---|---|---|---|---|
| Theory: | C, 52.29; | H, 5.76; | N, 11.43; | S, 17.45; |
| Found: | C, 52.38; | H, 5.47; | N, 11.20; | S, 17.40. |

By following the general procedures of Examples 61—64, an appropriately substituted 2-amino-1,3,4-thiadiazole was reacted with a benzoyl halide derivative to provide the following exemplary N-(1,3,4-thiadiazol-2-yl)benzamides.

Example 65

N-[5-(1-cyclohexyl-1-methylethyl)-1,3,4-thiadiazol-2-yl]-2,6-dimethoxybenzamide.

M.P. 241—243°C. Yield 38%.

Analysis calculated for $C_{20}H_{27}N_3O_3S$

| | | | |
|---|---|---|---|
| Theory: | C, 61.67; | H, 6.99; | N, 10.79; |
| Found: | C, 61.68; | H, 6.76; | N, 10.77. |

Example 66

N-[5-(1,1-diethylpropyl)-1,3,4-thiadiazol-2-yl]-2,6-dimethoxybenzamide.

M.P. 216—218°C. Yield 31%.

Analysis calculated for $C_{18}H_{25}N_3O_3S$

| | | | |
|---|---|---|---|
| Theory: | C, 59.48; | H, 6.93; | N, 11.56; |
| Found: | C, 59.65; | H, 6.73; | N, 11.37. |

Example 67

N-[5-(2,2-dimethylpropyl)-1,3,4-thiadiazol-2-yl]-2,6-dimethoxybenzamide

M.P. 188—190°C. Yield 43%.

Analysis calculated for $C_{16}H_{21}N_3O_3S$

Theory: C, 57.29; H, 6.31; N, 12.53;

Found: C, 57.27; H, 6.10; N, 12.31.

Example 68

N-[5-(2-methoxy-1,1-dimethylethyl) -1,3,4-thiadiazol-2-yl]-2,6-dimethoxybenzamide

M.P. 172—174°C. Yield 56%.

Analysis calculated for $C_{16}H_{21}N_3O_4S$

Theory: C, 54.68; H, 6.02; N, 11.96;

Found: C, 54.56; H, 5.95; N, 11.72.

Example 69

N-[5-(1,1-dimethyl-2-phenylethyl)-1,3,4-thiadiazol-2-yl]-2,6-dimethoxybenzamide

M.P. 167—169°C. Yield 29%.

Analysis calculated for $C_{21}H_{23}N_3O_3S$

Theory: C, 63.45; H, 5.83; N, 10.57; S, 8.07;

Found: C, 63.71; H, 5.82; N, 10.71; S, 8.05.

Example 70

N-[5-(2-cyclohexyl-1,1-dimethylethyl)-1,3,4-thiadiazol-2-yl]-2,6-dimethoxybenzamide

M.P. 191—193°C. Yield 77%.

Analysis calculated for $C_{21}H_{29}N_3O_3S$

Theory: C, 62.50; H, 7.24; N, 10.41; S, 7.95;

Found: C, 62.63; H, 7.22; N, 10.43; S, 7.99.

Example 71

N-[5-(1,1-dimethylhexyl)-1,3,4-thiadiazol-2-yl]-2,6-dimethoxybenzamide

M.P. 120—122°C. Yield 66%.

Analysis calculated for $C_{19}H_{27}N_3O_3S$

Theory: C, 60.45; H, 7.21; N, 11.13; S, 8.49;

Found: C, 60.64; H, 7.00; N, 11.27; S, 8.75.

Example 72

N-[5-(1-ethyl-1-methylpropyl)-1,3,4-thiadiazol-2-yl]-2-methoxy-3,6-dichlorobenzamide

M.P. 194—196°C. Yield 6%.

Analysis calculated for $C_{16}H_{19}Cl_2N_3O_2S$

Theory: C, 49.49; H, 4.93; N, 10.82;

Found: C, 49.69; H, 5.10; N, 11.04.

Example 73

N-[5-(1-ethylpentyl)-1,3,4-thiadiazol-2-yl]-2,6-dimethoxybenzamide

M.P. 120—122°C. Yield 69%.

Analysis calculated for $C_{18}H_{25}N_3O_3S$

Theory: C, 59.48; H, 6.93; N, 11.56;

Found: C, 59.74; H, 6.90; N, 11.45.

Example 74

N-[5-(1-methylcyclohexyl)-1,3,4-thiadiazol-2-yl]-2,6-dimethoxybenzamide

M.P. 211—213°C. Yield 47%.

Analysis calculated for $C_{11}H_{23}N_3O_3S$

Theory: C, 59.81; H, 6.41; N, 11.63;

Found: C, 60.03; H, 6.13; N, 11.83.

Example 75

N-[5-(1-methylpropyl)-1,3,4-thiadiazol-2-yl]-2,6-dimethoxybenzamide

M.P. 137—140°C. Yield 27%.

Analysis calculated for $C_{15}H_{19}N_3O_3S$

Theory: C, 56.06; H, 5.96; N, 13.07;

Found: C, 56.27; H, 6.03; N, 12.85.

Example 76

N-[5-(1-ethyl-1-methylbutyl)-1,3,4-thiadiazol-2-yl]-2,6-dimethoxybenzamide

M.P. 145—146°C. Yield 15%.

Analysis calculated for $C_{18}H_{25}N_3O_3S$

Theory: C, 59.48; H, 6.93; N, 11.56;

Found: C, 59.33; H, 7.03; N, 11.49.

Example 77

N-[5-(cyclohexylmethyl) -1,3,4-thiadiazol-2-yl]-2,6-dimethoxybenzamide

M.P. 152—154°C. Yield 54%.

Analysis calculated for $C_{18}H_{23}N_3O_3S$

Theory: C, 59.81; H, 6.41; N, 11.63; S, 8.87;

Found: C, 59.87; H, 6.40; N, 11.34; S, 8.62.

Example 78

N-[5-(2,2-dimethylbutyl)-1,3,4-thiadiazol-2-yl]-2,6-dimethoxybenzamide

M.P. 168—169°C. Yield 42%.

Analysis calculated for $C_{17}H_{23}N_3O_3S$

Theory: C, 58.43; H, 6.63; N, 12.02;

Found: C, 58.70; H, 6.79; N, 11.77.

Example 79

N-[5-(1-methylcyclopropyl)-1,3,4-thiadiazol-2-yl]-2,6-dimethoxybenzamide

M.P. 197—198°C. Yield 41%.

Analysis calculated for $C_{15}H_{17}N_3O_3S$

Theory: C, 56.41; H, 5.37; N, 13.16;

Found: C, 56.19; H, 5.25; N, 12.99.

Example 80

N-[5-(1-methylcyclopentyl)-1,3,4-thiadiazol-2-yl] -2,6-dimethoxybenzamide

M.P. 218—220°C. Yield 22%.

Analysis calculated for $C_{17}H_{21}N_3O_3S$

Theory: C, 58.77; H, 6.09; N, 12.09;

Found: C, 58.98; H, 6.34; N, 12.09.

Example 81

N-[5-(2,2-dichloro-1-methylcyclopropyl)-1,3,4-thiadiazol-2-yl]-2,6-dimethoxybenzamide

M.P. 235—236°C. Yield 63%.

Analysis calculated for $C_{15}H_{15}Cl_2N_3O_3S$

Theory: C, 46.40; H, 3.89; N, 10.82;

Found: C, 46.66; H, 3.64; N, 10.60.

Example 82

N-[5-(1,2-dimethylpropyl)-1,3,4-thiadiazol-2-yl]-2,6-dimethoxybenzamide

M.P. 169—171°C. Yield 50%.

Analysis calculated for $C_{16}H_{21}N_3O_3S$

Theory: C, 57.29; H, 6.31; N, 12.53;

Found: C, 57.29; H, 6.02; N, 12.37.

Example 83

N-[5-(1,1-dimethyl-3-butenyl)-1,3,4-thiadiazol-2-yl]-2,6-dimethoxybenzamide

M.P. 170—172°C. Yield 38%.

Analysis calculated for $C_{17}H_{21}N_3O_3S$

Theory: C, 58.77; H, 6.09; N, 12.09; S, 9.23;

Found: C, 58.75; H, 5.89; N, 11.91; S, 8.98.

Example 84

N-[5-(1-ethyl-1-methylpropyl)-1,3,4-thiadiazol-2-yl]-2-chlorobenzamide

M.P. 233—234°C. Yield 49%.

Analysis calculated for $C_{15}H_{18}ClN_3OS$

Theory: C, 55.63; H, 5.60; N, 12.98;

Found: C, 55.40; H, 5.36; N, 12.81.

Example 85

N-[5-(1-ethyl-1-methylpropyl)-1,3,4-thiadiazol-2-yl]-2-methoxybenzamide

M.P. 114—115°C. Yield 25%.

Analysis calculated for $C_{16}H_{21}N_3O_2S$

| | | | |
|---|---|---|---|
| Theory: | C, 60.16; | H, 6.63; | N, 13.16; |
| Found: | C, 59.96; | H, 6.42; | N, 13.05. |

Example 86

N-[5-(1-ethyl-1-methylpropyl)-1,3,4-thiadiazol-2-yl]-2,6-dimethylbenzamide

M.P. 191—192°C. Yield 32%.

Analysis calculated for $C_{17}H_{23}N_3OS$

| | | | |
|---|---|---|---|
| Theory: | C, 64.32; | H, 7.30; | N, 13.24; |
| Found: | C, 64.47; | H, 7.41; | N, 13.46. |

Example 87

N-[5-(1-ethyl-1-methylpropyl)-1,3,4-thiadiazol-2-yl]-2-(methylthio)benzamide

M.P. 145—147°C. Yield 62.1%.

Analysis calculated for $C_{16}H_{21}N_3OS_2$

| | | | | |
|---|---|---|---|---|
| Theory: | C, 57.28; | H, 6.31; | N, 12.53; | S, 19.11; |
| Found: | C, 56.99; | H, 6.06; | N, 12.50; | S, 19.35. |

Example 88

N-[5-(1-ethylcyclohexyl)-1,3,4-thiadiazol-2-yl]-2,6-dimethoxybenzamide

M.P. 222—224°C. Yield 25%.

Analysis calculated for $C_{19}H_{25}N_3O_3S$

| | | | |
|---|---|---|---|
| Theory: | C, 60.78; | H, 6.71; | N, 11.19; |
| Found: | C, 60.63; | H, 6.85; | N, 10.92. |

Example 89

N-[5-(1,1-diethylbutyl)-1,3,4-thiadiazol-2-yl]-2,6-dimethoxybenzamide

M.P. 210—212°C. Yield 61%.

Analysis calculated for $C_{19}H_{27}N_3O_3S$

| | | | |
|---|---|---|---|
| Theory: | C, 60.45; | H, 7.21; | N, 11.13; |
| Found: | C, 60.47; | H, 6.94; | N, 10.97. |

Example 90

N-[5-(1,1,2-trimethylpropyl)-1,3,4-thiadiazol-2-yl]-2,6-dimethoxybenzamide

M.P. 197—199°C. Yield 22%.

Analysis calculated for $C_{17}H_{23}N_3O_3S$

| | | | | |
|---|---|---|---|---|
| Theory: | C, 58.43; | H, 6.63; | N, 12.02; | S, 9.18; |
| Found: | C, 58.66; | H, 6.43; | N, 12.02; | S, 9.03. |

Example 91

N-[5-(1-ethylcyclopentyl)-1,3,4-thiadiazol-2-yl]-2,6-dimethoxybenzamide

M.P. 226—228°C. Yield 62%.

Analysis calculated for $C_{18}H_{23}N_3O_3S$

| | | | | |
|---|---|---|---|---|
| Theory: | C, 59.81; | H, 6.41; | N, 11.63; | S, 8.87; |
| Found: | C, 59.91; | H, 6.16; | N, 11.71; | S, 9.08. |

Example 92

N-[5-(1-ethyl-1-methylpropyl)-1,3,4-thiadiazol-2-yl]-2,6-dichlorobenzamide

M.P. 276—277°C. Yield 39%.

Analysis calculated for $C_{15}H_{17}Cl_2N_3OS$

| | | | | | |
|---|---|---|---|---|---|
| Theory: | C, 50.28; | H, 4.78; | N, 11.73; | S, 8.95; | Cl 19.79; |
| Found: | C, 50.52; | H, 4.54; | N, 11.56; | S, 8.69; | Cl, 20.03. |

Example 93

N-(5-methyl-1,3,4-thiadiazol-2-yl)-2,6-dimethoxybenzamide

M.P. 187—188°C. Yield 85%.

Analysis calculated for $C_{12}H_{13}N_3O_3S$

| | | | |
|---|---|---|---|
| Theory: | C, 51.60; | H, 4.69; | N, 15.04; |
| Found: | C, 51.72; | H, 4.50; | N, 15.05. |

Example 94

N-[5-(methoxymethyl)-1,3,4-thiadiazol-2-yl]-2,6-dimethoxybenzamide

M.P. 164—166°C. Yield 51%.

Analysis calculated for $C_{13}H_{15}N_3O_4S$

| | | | | |
|---|---|---|---|---|
| Theory: | C, 50.48; | H, 4.89; | N, 13.58; | S, 10.37; |
| Found: | C, 50.63; | H, 4.74; | N, 13.37; | S, 10.39. |

Example 95

N-[5-(1-methyl-1-propylbutyl)-1,3,4-thiadiazol-2-yl]-2,6-dimethoxybenzamide

M.P. 161—163°C. Yield 40%.

Analysis calculated for $C_{19}H_{27}N_3O_3S$

| | | | | |
|---|---|---|---|---|
| Theory: | C, 60.45; | H, 7.21; | N, 11.13; | S, 8.49; |
| Found: | C, 60.66; | H, 7.03; | N, 10.85; | S, 8.27. |

Example 96

N-[5-(1,1,2,2-tetramethylpropyl)-1,3,4-thiadiazol-2-yl]-2,6-dimethoxybenzamide

M.P. 245—247°C. Yield 51%.

Analysis calculated for $C_{18}H_{25}N_3O_3S$

| | | | | |
|---|---|---|---|---|
| Theory: | C, 59.48; | H, 6.93; | N, 11.56; | S, 8.82; |
| Found: | C, 59.58; | H, 6.70; | N, 11.44; | S, 8.93. |

Example 97

[5-(1-ethyl-1-methylpropyl)-1,3,4-thiadiazol-2-yl]-2,6-dimethoxy-4-(trifluoromethyl)benzamide

M.P. 252—254°C. Yield 20%.

Analysis calculated for $C_{18}H_{22}F_3N_3O_3S$

| | | | | | |
|---|---|---|---|---|---|
| Theory: | C, 51.79; | H, 5.31; | N, 10.07; | S, 7.68; | F, 13.65; |
| Found:. | C, 51.52; | H, 5.07; | N, 9.97; | S, 7.84; | F, 13.70. |

Example 98

N-[5-(2-chloro-1,1-dimethylethyl)-1,3,4-thiadiazol-2-yl]-2,6-dimethoxybenzamide

M.P. 202—204°C. Yield 69%.

Analysis calculated for $C_{15}H_{18}ClN_3O_3S$

| | | | | |
|---|---|---|---|---|
| Theory: | C, 50.63; | H, 5.06; | N, 11.81; | S, 9.00; |
| Found: | C, 50.86; | H, 5.14; | N, 11.90; | S, 8.59. |

Example 99

N-[5-(1,1-dimethylpentyl)-1,3,4-thiadiazol-2-yl]-2,6-dimethoxybenzamide

M.P. 160—162°C. Yield 30%.

Analysis calculated for $C_{18}H_{25}N_3O_3S$

| | | | | |
|---|---|---|---|---|
| Theory: | C, 59.48; | H, 6.93; | N, 11.56; | S, 8.82; |
| Found: | C, 59.69; | H, 6.77; | N, 11.34; | S, 8.81. |

Example 100

N-(5-cyclohexyl-1,3,4-thiadiazol-2-yl)-2,6-dimethoxybenzamide

M.P. 206—208°C. Yield 55%.

Analysis calculated for $C_{17}H_{21}N_3O_3S$

| | | | | |
|---|---|---|---|---|
| Theory: | C, 58.77; | H, 6.09; | N, 12.09; | S, 9.23; |
| Found: | C, 58.48; | H, 6.30; | N, 11.85; | S, 9.42. |

Example 101

N-[5-(1,1-dimethylbutyl)-1,3,4-thiadiazol-2-yl]-2,6-dimethoxybenzamide

M.P. 167—169°C. Yield 45%.

Analysis calculated for $C_{17}H_{23}N_3O_3S$

| | | | | |
|---|---|---|---|---|
| Theory: | C, 58.43; | H, 6.63; | N, 12.02; | S, 9.18; |
| Found: | C, 58.65; | H, 6.79; | N, 12.25; | S, 8.87. |

Example 102

N-(5-cyclobutyl-1,3,4-thiadiazol-2-yl)-2,6-dimethoxybenzamide

M.P. 195—197°C. Yield 65%.

Analysis calculated for $C_{15}H_{17}N_3O_3S$

| | | | | |
|---|---|---|---|---|
| Theory: | C, 56.41; | H, 5.37; | N, 13.16; | S, 10.04; |
| Found: | C, 56.13; | H, 5.18; | N, 12.89; | S, 9.96. |

Example 103

N-[5-(1-ethyl-1-methylpropyl)-1,3,4-thiadiazol-2-yl]-4-methoxybenzamide

M.P. 138—140°C. Yield 63%.
Analysis calculated for $C_{16}H_{21}N_3O_2S$
Theory: C, 60.16; H, 6.63; N, 13.16; S, 10.04;
Found: C, 59.90; H, 6.47; N, 13.10; S, 9.82.

Example 104

N-[5-(1-ethyl-1-methylpropyl)-1,3-4-thiadiazol-2-yl]-2,4,6-trimethoxybenzamide

M.P. 183.5°C. Yield 65%.
Analysis calculated for $C_{18}H_{25}N_3O_4S$
Theory: C, 56.97; H, 6.64; N, 11.07; S, 8.45;
Found: C, 57.15; H, 6.63; N, 10.86; S, 8.38.

Example 105

N-[5-(1-propylcyclohexyl)-1,3,4-thiadiazol-2-yl]-2,6-dimethoxybenzamide

M.P. 190—192°C. Yield 56%.
Analysis calculated for $C_{20}H_{27}N_3O_3S$
Theory: C, 61.67; H, 6.99; N, 10.79; S, 8.23;
Found: C, 61.46; H, 6.76; N, 10.53; S, 8.44.

Example 106

N-[5-(1,1,2-trimethyl-2-butenyl)-1,3,4-thiadiazol-2-yl]-2,6-dimethoxybenzamide

M.P. 215—218°C. Yield 60%.
Analysis calculated for $C_{18}H_{23}N_3O_3S$
Theory: C, 59.81; H, 6.41; N, 11.63; S, 8.87;
Found: C, 59.54; H, 6.14; N, 11.55; S, 8.80.

Example 107

N-[5-(1,1,2-trimethylbutyl)-1,3,4-thiadiazol-2-yl]-2,6-dimethoxybenzamide

M.P. 181—183°C. Yield 69%.
Analysis calculated for $C_{18}H_{25}N_3O_3S$
Theory: C, 59.48; H, 6.93; N, 11.56; S, 8.82;
Found: C, 59.46; H, 6.61; N, 11.36; S, 8.32.

Example 108

N-[5-(1,1,3-trimethylbutyl)-1,3,4-thiadiazol-2-yl]-2,6-dimethoxybenzamide

M.P. 195—197°C. Yield 66%.
Analysis calculated for $C_{18}H_{25}N_3O_3S$
Theory: C, 59.48; H, 6.93; N, 11.56;
Found: C, 59.40; H, 6.77; N, 11.58.

Example 109

N-[5-(1,1-dimethylpropyl)-1,3,4-thiadiazol-2-yl]-2,6-dimethoxybenzamide

M.P. 227—229°C. Yield 67%.
Analysis calculated for $C_{16}H_{21}N_3O_3S$
Theory: C, 57.29; H, 6.31; N, 12.53; S, 9.56;
Found: C, 57.09; H, 6.03; N, 12.27; S, 9.76.

Example 110

N-[5-phenylmethyl-1,3,4-thiadiazol-2-yl]-2,6-dimethoxybenzamide

M.P. 190—192°C. Yield 80%.
Analysis calculated for $C_{18}H_{17}N_3O_3S$
Theory: C, 60.83; H, 4.82; N, 11.82; S, 9.02;
Found: C, 60.78; H, 4.86; N, 12.05; S, 8.88.

Example 111

N-[5-(1,1-dimethylethyl)-1,3,4-thiadiazol-2-yl]-2,6-dimethoxybenzamide

M.P.
Analysis calculated for $C_{15}H_{19}N_3O_3S$
Theory: C, 56.05; H, 5.96; N, 12.07;
Found: C, 55.81; H, 5.98; N, 12.10.

Example 112

N-[5-(1-ethyl-1,2,2-trimethylpropyl)-1,3,4-thiadiazol-2-yl]-2,6-dimethoxybenzamide

M.P. 254—256°C. Yield 56%.

Analysis calculated for $C_{19}H_{27}N_3O_3S$

Theory: C, 60.45; H, 7.21; N, 11.13; S, 8.49;

Found: C, 60.33; H, 7.02; N, 10.95; S, 8.80.

Example 113

N-(5-cyclopentyl-1,3,4-thiadiazol-2-yl)-2,6-dimethoxybenzamide

M.P. 180—181°C. Yield 72%.

Analysis calculated for $C_{16}H_{19}N_3O_3S$

Theory: C, 57.64; H, 5.74; N, 12.60;

Found: C, 57.70; H, 5.87; N, 12.37.

Example 114

N-[3-(1,1-dimethylethyl)-1H-pyrazol-5-yl]-2,6-dimethoxybenzamide

A) Preparation of 3-(1,1-dimethylethyl)-5-amino-1H-pyrazole

A suspension of 9.6 g of sodium hydride in 300 ml of tetrahydrofuran was stirred at 60°C while a mixture of 23.2 g of methyl trimethylacetate and 8.2 g of acetonitrile was added in one portion. The reaction mixture was heated at reflux for five hours, and then cooled to room temperature and concentrated by evaporation of the solvent under reduced pressure. The product thus formed was dissolved in water and washed with dichloromethane. The aqueous layer was made acidic with 1 N hydrochloric acid, and the acidic solution was extracted with fresh dichloromethane. The organic extracts were combined, washed with water, dried, and the solvent was removed by evaporation to give 14.0 g of cyanomethyl *tert.*-butyl ketone.

The ketone thus formed was dissolved in 150 ml of ethanol containing 32 g of hydrazine. The reaction mixture was heated at reflux for twelve hours, and then cooled to room temperature. Removal of the solvent by evaporation under reduced pressure provided a solid residue, which when triturated with 250 ml of petroleum ether, filtered and air dried, was identified as 12.5 g of 3-*tert.*-butyl-5-amino-1H-pyrazole.

M.P. 72—74°C.

B. Acylation of the amino pyrazole with 2,6-dimethoxybenzoyl chloride.

To a stirred solution of 1.39 g of 3-*tert.*-butyl-5-amino-1H-pyrazole in 50 ml of benzene were added in one portion 2.01 g of 2,6-dimethoxybenzoyl chloride. The reaction mixture was heated at reflux for sixteen hours. The reaction mixture was then cooled, filtered, and the solvent was removed from the filtrate by evaporation. The residue was crystallized from ethyl acetate to provide 550 mg of N-[3-(1,1-dimethylethyl)-1H-pyrazol-5-yl]2,6-dimethoxybenzamide

M.P. 176—178°C. Yield 18%.

Mass Spec. $M^+$ Theory, 303, Found 304.

NMR ($DMSOd_6$); $\delta$ 1.35 (s, 9H, t-Bu); $\delta$ 3.76 (s, 6H, $CH_3O$—) $\delta$ 5.7—7.4 (m, 5H, aromatic); $\delta$ 10.9 (s, 1H, amide NH).

Analysis calculated for $C_{16}H_{21}N_3O_4$

Theory: C, 63.35; H, 6.98; N, 13.85;

Found: C, 57.39; H, 6.34; N, 14.41.

The following N-pyrazolyl benzamides were prepared by the general procedure of Example 114.

Example 115

N-[3-(1-ethyl-1-methylpropyl)-1H-pyrazol-5-yl]-2,6-dimethoxybenzamide

M.P. 222—223°C. Yield 38%.

Analysis calculated for $C_{18}H_{25}N_3O_3$

Theory: C, 65.23; H, 7.60; N, 12.68;

Found: C, 65.08; H, 7.61; N, 12.50.

Example 116

N-[3-(1,1-dimethylbutyl)-1H-pyrazol-5-yl]-2,6-dimethoxybenzamide

M.P. 211—213°C. Yield 27%.

Mass Spec. $M^+$ Theory, 330, Found, 331

NMR ($CDCl_3$); $\delta$ 0.7—1.6 (m, 13H, 1,1-dimethylbutyl); $\delta$ 3.81 (s, 6H, $CH_3O$); $\delta$ 6.51—6.77 (m, 3H, benzoyl aromatic); $\delta$ 7.2—7.5 (m, 2H, pyrazole aromatic); $\delta$ 7.8—8.1 (broad s, 1H, amide NH).

Analysis calculated for $C_{18}H_{24}N_3O_3$

Theory: C, 65.23; H, 7.60; N, 12.68;

Found: C, 65.34; H, 6.79; N, 8.44.

Example 117

N-[3-(1,1-dimethylpropyl)-1H-pyrazol-5-yl]-2,6-dimethoxybenzamide

M.P. 235—237°C. Yield 37%.

Analysis calculated for $C_{17}H_{22}N_3O_3$

Theory: C, 64.33; H, 7.30; N, 13.24;

Found: C, 64.20; H, 7.03; N, 12.99.

Example 118

N-[5-(1-ethyl-1-methylpropyl)-4H-1,2,4-triazol-3-yl]-2,6-dimethoxybenzamide

A) Preparation of 5-(1-ethyl-1-methylpropyl)-3-amino-4H-1,2,4-triazole

To a stirred solution of 13.1 g of potassium hydroxide in 40 ml of water were added dropwise a solution of 8.4 g of dicyandiamide in 50 ml of acetone. The reaction mixture was next cooled to 5°C, and then 14.0 g of 2-ethyl-2-methyl butyryl chloride were added dropwise over ten minutes. Following the addition, the reaction mixture was stirred at about 5°C for fifteen minutes, and then diluted to 600 ml by the addition of water. The aqueous mixture was made acidic to pH 5.5 with glacial acetic acid, whereupon a white precipitate formed. The precipitate was collected by filtration, washed with water and air dried to give 5.45 g of N-(2-ethyl-2-methylbutyryl)-dicyandiamide.

The product thus formed was suspended in 35 ml of water and stirred while a solution of 1.1 g of hydrazine in 25 ml of 2-ethoxyethanol was added in one portion. The reaction mixture was boiled for forty-five minutes, and then was cooled to room temperature and stirred for twelve hours. The precipitate which had formed was collected by filtration and was dried at 90°C for two hours to provide 2.65 g of 1-[5-(1-ethyl-1-methylpropyl)-4H-1,2,4-triazol-3-yl]urea.

The pyrazolyl urea thus formed was added to 30 ml of water containing 3.0 g of sodium hydroxide. The reaction mixture was heated at reflux for twelve hours, and then made strongly acidic (pH 2.0) with nitric acid. The precipitate which formed was collected by filtration while the mixture was hot. After air drying the precipitate, it was identified as 2.65 g of 5-(1-ethyl-1-methylpropyl)-3-amino-4H-1,2,4-triazole, as the nitric acid salt.

M.P. 131°C.

The salt thus formed was dissolved in water and diluted with ammonia to pH 8. The solvent was then removed by evaporation and the residue was triturated with 25 ml of acetonitrile to give 1.1 g of 5-(1-ethyl-1-methylpropyl)-3-amino-4H-1,2,4-triazole.

B) A solution of 1.1 g of the triazole and 1.43 g of 2.6-dimethoxybenzoyl chloride in 75 ml of toluene was heated at reflux for sixteen hours. The reaction mixture was cooled to room temperature and the solvent was removed by evaporation under reduced pressure to give a gum. The gum was chromatographed over a silica gel column, eluting with 50% ethyl acetate in hexane. The fractions shown by thin layer chromatographic analysis to contain the product were combined and the solvent was evaporated to provide 175 mg of N-[5-(1-ethyl-1-methylpropyl)-4H-1,2,4-triazol-3-yl]-2,6-dimethoxybenzamide.

M.P. 279—280°C. Yield 8%.

Analysis calculated for $C_{17}H_{24}N_4O_3$

Theory: C, 61.45; H, 7.23; N, 16.87;

Found: C, 60.94; H, 7.14; N, 15.99.

Mass Spec: $M^+$ Theory, 332, Found, 333.

Example 119

N-[3-(1,1-dimethylethyl)-5-isothiazolyl]-2,6-dimethoxybenzamide

A) Preparation of 3-(1,1-dimethylethyl)-5-aminoisothiazole

A mixture of 50.0 g of cyanomethyl *tert.*-butyl ketone in 250 ml of ethanol containing 200 ml of ammonia was heated at 150°C for sixteen hours. The reaction mixture was cooled to room temperature and the solvent was removed by evaporation under reduced pressure. The residue was dissolved in 330 ml of dichloromethane and diluted with 0.7 g of potassium hydroxide and 52 ml of hydrogen sulfide. The reaction mixture was heated at 80°C for twenty-four hours, and then cooled and concentrated to dryness by evaporation of the solvent. The product was identified as 71 g of 2,2-dimethyl-3-amino-3-butenyl thiocarboxamide.

An 800 mg portion of the product thus obtained was dissolved in 25 ml of ethanol containing 5 ml of 30% hydrogen peroxide. The reaction mixture was stirred for twenty minutes, and then concentrated to dryness by evaporation to provide (3-(1,1-dimethyl-ethyl)-5-aminoisothiazole.

B) A solution of the 5-amino-isothiazole and 1.1 g of 2,6-dimethoxybenzoyl chloride in 50 ml of toluene was heated at reflux for two hours, during which time a precipitate formed. The precipitate was collected by filtration and dried to give 340 mg of N-[3-(1,1-dimethylethyl)-5-isothiazolyl]-2,6-dimethoxybenzamide.

M.P. 266—267°C. Yield 30%.

Analysis calculated for $C_{16}H_{20}N_2O_3S$

Theory: C, 59.98; H, 6.29; N, 8.74;

Found: C, 59.71; H, 6.15; N, 8.74.

Example 120

N-[3-(1-ethyl-1-methylpropyl)-5-isothiazolyl]-2,6-dimethoxybenzamide was prepared according to Example 120.

M.P. 243—244°C. Yield 70%.

Analysis calculated for $C_{18}H_{24}N_2O_3S$

Theory: C, 62.04; H, 6.94; N, 8.04;

Found: C, 62.21; H, 6.73; N, 8.24.

Example 121

N-[6-(1,1-dimethylethyl)pyridazin-3-yl]-2,6-dimethoxybenzamide

A) Preparation of 3-amino-6-(1,1-dimethylethyl)pyridazine

A mixture of 5.8 g. of 3-chloro-6-(1,1-dimethylethyl)pyridazine in 100 ml. of liquid ammonia was heated in a bomb at 200°C. for twenty-four hours. The reaction mixture was cooled to room temperature and filtered, and the solvent was removed from the filtrate by evaporation under reduced pressure to provide a black oil. The oil was chromatographed over silica gel, eluting with ethyl acetate and benzene. The appropriate fractions were combined and the solvent was removed therefrom by evaporation to give 2.08 g. of 3-amino-6-(1,1-dimethylethyl)pyridazine. M.P. 125—132°C.

Analysis calculated for $C_8H_{13}N_3$

Theory: C, 63.54; H, 8.67; N, 27.79;

Found: C, 63.74; H, 8.49; N, 27.53.

B) A solution of 600 mg. of 2,6-dimethoxybenzoyl chloride in 100 ml. of toluene containing 500 mg. of 3-amino-6-(1,1-dimethylethyl)pyridazine was heated at reflux under a nitrogen atmosphere for twenty-one hours. The reaction mixture was cooled to room temperature and the precipitate which formed was collected by filtration. The solid product was chromatographed over silica gel, eluting with ethyl acetate. Thin layer chromatographic analysis of the major product suggested that a minor amount of bis acylated product has been formed. The mixture was dissolved in 10 ml. of ethanol and 10 ml. of 2 N sodium hydroxide, and the alkaline solution was heated at reflux for two hours. The reaction mixture was cooled and acidified by the addition of 1 N hydrochloric acid. The precipitate which formed was collected by filtration and recrystallized from hexane to provide 49 mg. of N-[6-(1,1-dimethylethyl)pyridazin-3-yl]-2,6-dimethoxybenzamide.

M.P. 163—165°C. Yield 5%.

Analysis calculated for $C_{17}H_{21}N_3O_3$

Theory: C, 64.74; H, 6.71; N, 13.32;

Found: C, 64.95; H, 6.41; N, 13.28.

Mass Spec. $M^+$ Theory 315; Found 315.

Several additional compounds embraced by this invention have been prepared by reaction of an appropriately substituted benzoyl halide with a aryl amine. Typical examples follow.

Example 122

N-5-(1-ethyl-1-methylpropyl)-1,3,4-thiadiazol-2-yl]-2,6-di(methylthio)benzamide

M.P. 194—195°C. Yield 42%.

Analysis calculated for $C_{17}H_{23}N_3OS_3$

Theory: C, 53.51; H, 6.08; N, 11.01; S, 25.21;

Found: C, 53.65; H, 6.12; N, 10.80; S, 25.40.

Example 123

N-[5-(1-ethyl-1-methylpropyl)-1,3,4-thiadiazol-2-yl]-2-methoxy-6-methylthiobenzamide

M.P. 184—185°C. Yield 44.7%.

Analysis calculated for $C_{17}H_{23}N_3O_2S_2$

Theory: C, 55.86; H, 6.34; N, 11.50; S, 17.54;

Found: C, 55.93; H, 6.17; N, 11.23; S, 17.37.

Example 124

N-[5-(1-*n*-propylcyclopentyl)-1,3,4-thiadiazol-2-yl]-2,6-dimethoxybenzamide

M.P. 181—183°C. Yield 56.5%.

Analysis calculated for $C_{19}H_{25}N_3O_3S$

Theory: C, 60.78; H, 6.71; N, 11.09; S, 8.54;

Found: C, 61.08; H, 6.76; N, 11.40; S, 8.29.

Example 125

N-[5-(1-(1-methylethenyl)cyclohexyl)-1,3,4-thiadiazol-2-yl]-2,6-dimethoxybenzamide

M.P. 226—228°C. Yield 53%.

Analysis calculated for $C_{20}H_{25}N_3O_3S$

Theory: C, 61.99; H, 6.50; N, 10.84; S, 8.27;

Found: C, 62.20; H, 6.52; N, 10.55; S, 8.04.

Example 126

N-[-(1-*iso*propylcyclohexyl)-1,3,4-thiadiazol-2-yl]-2,6-dimethoxybenzamide

M.P. 224—226°C. Yield 53.6%.

Analysis calculated for $C_{20}H_{27}N_3O_3S$

Theory: C, 61.67; H, 6.99; N, 10.79; S, 8.23;

Found: C, 61.47; H, 6.75; N, 10.53; S, 8.21.

Example 127

N-[5-(1-ethyl-1,2-dimethyl-2-propenyl)-1,3,4-thiadiazol-2-yl]-2,6-dimethoxybenzamide

M.P. 207—209°C. Yield 10%.

Analysis calculated for $C_{18}H_{23}N_3O_3S$

Theory: C, 59.81; H, 6.41; N, 11.63; S, 8.87;

Found: C, 59.65; H, 6.40; N, 11.56; S, 9.02.

Example 128

N-[5-(1-ethyl-1-methylpropyl)-1,3,4-thiadiazol-2-yl]-2-methoxy-4-trifluoromethyl-6-methylthiobenzamide

M.P. 224—225°C. Yield 26.2%.

Analysis calculated for $C_{18}H_{22}F_3N_3O_2S_2$

Theory: C, 49.87; H, 5.12; N, 9.69; F, 13.15; S, 14.79;

Found: C, 49.65; H, 4.92; N, 9.90; F, 13.40; S, 14.81.

Example 129

N-[5-(1-methylethyl)-1,3,4-thiadiazol-2-yl]-2,6-dimethoxybenzamide.

M.P. 164—166°C. Yield 29%.

Analysis calculated for $C_{14}H_{17}N_3O_3S$

Theory: C, 54.72; H, 5.54; N, 13.68; S, 10.42;

Found: C, 54.65; H, 5.52; N, 13.62; S, 10.90.

Example 130

N-(5-Cyclopropyl-1,3,4-thiadiazol-2-yl)-2,6-dimethoxybenzamide

M.P. 194—196°C. Yield 52%.

Analysis calculated for $C_{14}H_{15}N_3O_3S$

Theory: C, 55.07; H, 4.95; N, 13.76; S, 10.50;

Found: C, 55.37; H, 5.18; N, 13.66; S, 10.29.

Example 131

N-(3-ethyl-5-isoxazolyl)-2,6-dimethoxybenzamide

M.P. 132—4°C. Yield 57%.

Example 132

N-(3-methyl-5-isoxazolyl)-2,6-dimethoxybenzamide

M.P. 155—158°C. Yield 70%.

Analysis calculated for $C_{13}H_{14}N_2O_4$

Theory: C, 59.54; H, 5.38; N, 10.68; O, 24.40;

Found: C, 59.85; H, 5.67; N, 10.40; O, 24.63.

Example 133

N-(3-*n*-hexyl-5-isoxazolyl)-2,6-dimethoxybenzamide

M.P. 119—121°C.

Analysis calculated for $C_{18}H_{22}N_2O_4$

Theory: C, 65.04; H, 7.28; N, 8.43;

Found: C, 64.92; H, 7.43; N, 8.16.

Example 134

N-(3-ethyl-5-isoxazolyl)-2,6-dimethoxybenzamide

M.P. 132—134°C. Yield 56.6%

Analysis calculated for $C_{14}H_{16}N_2O_4$

Theory: C, 60.86; H, 5.84; N, 10.14;

Found: C, 60.77; H, 5.81; N, 10.03.

Example 135

N-[5-(1-ethyl-1-methylpropyl)-1,3,4-oxadiazol-2-yl]-2,6-dimethoxybenzamide.

M.P. 190—192°C. Yield 16%

Analysis calculated for $C_{17}H_{23}N_3O_3$

Theory: C, 61.36; H, 6.91; N, 12.61;

Found: C, 61.99; H, 6.84; N, 13.29.

Example 136

N-[3-(1,1-dimethylethyl)-1,2,4-thiadiazol-5-yl]-2,6-dimethoxybenzamide

M.P. 180—182°C. Yield 65.7%

Analysis calculated for $C_{15}H_{19}N_3O_3S$

Theory: C, 56.06; H, 5.96; N, 13.07; S, 9.98;

Found: C, 56.07; H, 5.72; N, 12.89; S, 10.25.

Example 137

N-[3-(1,1-dimethylethyl)-1,2,4-oxadiazol-5-yl]-2,6-dimethoxybenzamide

M.P. 207—209°C.

Analysis calculated for $C_{15}H_{19}N_3O_4$

Theory: C, 59.01; H, 6.27; N, 13.76;

Found: C, 59.18; H, 6.49; N, 13.85.

Example 138

N-[3-(1-ethyl-1-methylpropyl)-5-isoxazolyl]-2,3,5-triodobenzamide

M.P. 167—168°C.

Analysis calculated for $C_{16}H_{17}I_3N_2O_2$

Theory: C, 29.56; H, 2.64; N, 4.31;

Found: C, 29.80; H, 2.79; N, 4.29.

Example 139

N-[3-(1-ethyl-1-methylpropyl)-5-isoxazolyl]-2,6-dimethylthiobenzamide

M.P. 114—116°C. Yield 21%

Example 140

N-[3-(1-methoxymethyl-1-methylpropyl)-5-isoxazolyl]-2,6-dimethoxybenzamide

M.P. 164—166°C. Yield 34%

Example 141

N-[3-(1-ethyl-1-methylpropyl)-4-methyl-5-isoxazolyl]-2,6-dimethoxybenzamide

M.P. 179—180°C. Yield 37%

Example 142

N-[5-(1-ethyl-1-methylpropyl)-1,3,4-oxadiazol-2-yl]-2,6-dimethoxybenzamide

M.P. 190—192°C. Yield 2%

Example 143

N-[5-(1-methylcyclobutyl)-1,3,4-thiadiazol-2-yl]-2,6-dimethoxybenzamide

M.P. 218—220°C. Yield 50%

Example 144

N-[5-(1,1,2-trimethyl-2-propenyl)-1,3,4-thiadiazol-2-yl]-2,6-dimethoxybenzamide

M.P. 236—238°C. Yield 66%

Example 145

3-Bromo-N-[5-(1-ethyl-1-methylpropyl)-1,3,4-thiadiazol-2-yl]-2,6-dimethoxybenzamide

M.P. 163—5°C. Yield 35%

Example 146

N-[5-(1,1-dimethyl-2-propenyl)-1,3,4-thiadiazol-2-yl]-2,6-dimethoxybenzamide

M.P. 225—8°C. Yield 52%

Example 147
N-(5-cycloheptyl-1,3,4-thiadiazol-2-yl)-2,6-dimethoxybenzamide
M.P. 213—5°C. Yield 80%

Example 148
N-[5-(1-ethyl-1-mthylpentyl)-1,3,4-thiadiazol-2-yl]-2,6-dimethoxybenzamide
M.P. 141—3°C. Yield 49%

Example 149
N-[5-(1-ethyl-1-propylbutyl)-1,3,4-thiadiazol-2-yl]-2,6-dimethoxybenzamide
M.P. 216—8°C. Yield 48%

Example 150
N-[5-(1-ethyl-1,3-dimethylbutyl)-1,3,4-thiadiazol-2-yl]-2,6-dimethoxybenzamide
M.P. 166—8°C. Yield 18%

Example 151
N-[5-(1-ethyl-1-methylhexyl)-1,3,4-thiadiazol-2-yl]-2,6-dimethoxybenzamide
M.P. 107—9°C. Yield 37%

Example 152
N-[5-(1-ethyl-1,2-dimethylpropyl)-1,3,4-thiadiazol-2-yl]-2,6-dimethoxybenzamide
M.P. 205—7°C. Yield 29%

Example 153
N-[5-(1,1-dimethyl-2-methoxypropyl)-1,3,4-thiadiazol-2-yl]-2,6-dimethoxybenzamide
M.P. 202—3°C. Yield 25%

Example 154
N-[5-(1-methylthio-1-methylpropyl)-1,3,4-thiadiazol-2-yl]-2,6-dimethoxybenzamide
M.P. 223—4°C. Yield 52%

Example 155
N-[5-(1-ethyl-1,2-dimethylbutyl)-1,3,4-thiadiazol-2-yl]-2,6-dimethoxybenzamide
M.P. 197—9°C. Yield 15%

Example 156
N-[6-(1-ethyl-1 -methylpropyl)pyridazin-3-yl]-2,6-dimethoxybenzamide
M.P. 145—7°C. Yield 68%

Example 157
N-[6-(1ethylcyclohexyl)pyridazin-3-yl]-2,6-dimethoxybenzamide

Example 158
N-[5-(1,1-dimethoxymethyl)ethyl-3-isoxazolyl]-2,6-dimethoxybenzamide
M.P. 164—6°C. Yield 77%

Example 159
N-[5-(1,1-bisacetoxymethyl)ethyl-1,3,4-thiadiazol-2-yl]-2,6-dimethoxybenzamide
M.P. 115—125°C.(S) Yield 3%

Example 160
N-[5-(2-methyl-1,3-dithian-2-yl)-1,3,4-thiadiazol-2-yl]-2,6-dimethoxybenzamide
M.P. 259°C. Yield 2%

Example 161
N-[5-(2,2-dichloro-1,1-dimethylethyl)-1,3,4-thiadiazol-2-yl]-2,6-dimethoxybenzamide
M.P. 211—3°C. Yield 33%

Example 162
N-[5-(1,3-dithian-2-yl)-1,3,4-thiadiazol-2-yl]-2,6-dimethoxybenzamide
M.P. 200—208°C. Yield 60%

Example 163

N-[5(4)-(1,1-dimethylethyl)-2-imidazolyl]-2,6-dimethoxybenzamide

A) Preparation of 2-amino-5-(1,1-dimethylethyl)imidazole

To a stirred solution of 6.8 g of aminomethyl-(1,1-dimethylethyl)ketone, as the hydrochloride salt, in 30 ml of water were added portionwise 5.0 g of cyanamide. The pH of the reaction mixture was adjusted to 6.0 with 1N sodium hydroxide, and then the mixture was heated to 90°C and stirred for thirty-five minutes. The reaction mixture was next cooled to room temperature, diluted with 100 ml. of water, and extracted several times with diethyl ether. The aqueous layer was made alkaline with ammonium hydroxide, and again extracted several times with diethylether. The extracts from the alkaline mixture were combined and concentrated to dryness to provide a solid residue. The residue thus obtained was dissolved in 20 ml of 6N hydrochloric acid and heated at reflux for sixteen hours. The reaction mixture was cooled, concentrated to an oil by evaporation of the solvent, and the oil was dissolved in water and made alkaline to pH 8.5. The alkaline mixture was extracted several times with diethyl ether. The etheral extracts were combined, washed with water, dried, and the solvent was removed by evaporation under reduced pressure to provide 0.5 g of 5-(1,1-dimethylethyl)-2-amino-imidazole.

NMR ($DMSOd_6$): $\delta$ 1.17 (s, 9H, *t*-butyl) $\delta$ 5.47 ($NH_2$, NH), $\delta$ 6.1 (s, 1H, aromatic).

B) A mixture of 0.5 g of the 2-aminoimidazole thus prepared and 0.72 g of 2,6-dimethoxybenzoyl chloride in 50 ml of benzene was heated at reflux for sixteen hours. The reaction mixture was then cooled and the solvent was removed by evaporation under reduced pressure. The product was dissolved in 25 ml of ethanol, and again the solvent was removed by evaporation under reduced pressure to provide an oil. The oil was purified by preparative thin layer chromatography to afford 25 mg of N-[5-(4)-(1,1-dimethylethyl)-2-imidazolyl]-2,6-dimethoxybenzamide.

M.P. 170—173°C.

NMR ($DMSOd_6$) $\delta$ 1.2 (s, 9H, *t*-butyl); $\delta$ 3.74 (s, 6H, methoxy); $\delta$ 6.38—7.41 (m, 5H, aromatic).

Example 164

N-[5-(1-Ethyl-1-methylpropyl)-1,3,4-thiadiazol-2-yl]-2,6-dimethoxybenzenecarbothioamide

N-[5-(1-Ethyl-1-methylpropyl)-1,3,4-thiadiazol-2-yl]-2,6-dimethoxybenzamide (5 g; 0.014 mole) was suspended in dioxane (75 ml). Phosphorus pentasulfide (4.7 g; 0.21 mole) was added to the suspension and the reaction mixture refluxed for 3 hours under a nitrogen atmosphere. The mixture was then cooled and filtered. The mother liquor was then poured into water (200 ml) and the yellow precipitate which formed upon stirring (1 hour) was filtered.

The material thus obtained was purified using high pressure liquid chromatography.

M.P. 202—4°C. Yield 17.6%

Example 165

N-[3-(1-Ethyl-1-methylpropyl)-5-isoxazolyl]-2,6-dimethoxybenzenecarbothioamide

N-[3-(1-Ethyl-1-methylpropyl)-5-isoxazolyl]-2,6-dimethoxybenzamide (6.0 g; 0.018 mole) and Lawesson's Reagent (7.28,g; 0.018 mole) were suspended in toluene (150 ml) and the stirred mixture heated to reflux. The mixture initially formed a clear yellow solution and then, as the reaction proceeded, turned orange in color, at which time the solution was cooled to room temperature and the toluene evaporated off *in vacuo*. Methylene chloride was added to the solid precipitate which formed and the mixture was filtered. Evaporation of the filtrate *in vacuo* yielded a reddish orange viscous oil. This oil was placed on a column of dry-packed silica gel (500 g) and the column eluted with methylene chloride. The first component to elute was an isomer derived from Lawesson's reagent. The next component was the title product which was recovered as a yellow liquid. The desired product fractions were combined, solvent evaporated *in vacuo*, whereupon the title product crystallized out as a yellow solid, M.P. 110—2°C, yield 4.1 g.

Example 166

N-[5-(1,1-Dimethoxymethyl)ethyl-3-isoxazolyl]-2,6-dimethoxybenzamide, sodium salt

N-[5-(1,1-Dimethoxymethyl)ethyl-3-isoxazolyl]-2,6-dimethoxybenzamide (3.64 g; 0.01 mole) was dissolved in tetrahydrofuran (75 ml) at room temperature under a nitrogen atmosphere. To the solution was added sodium hydride (0.432 g; 0.009 mole) portionwise with stirring. The reaction mixture was stirred until the title product precipitated as a white solid.

After standard work-up and drying *in vacuo*, (the material was hygroscopic) there was obtained 2.25 grams of the sodium salt. The melting point of the hydrated product was 82—4°C.

Example 167

N-[5-(1-Ethyl-1-methylpropyl)-3-isoxazolyl]-2,6-dimethoxybenzamide, sodium salt

The title product was similarly prepared using as solvent a 1:1 mixture of methylene dichloride and dry diethyl ether.

M.P. 218—220°C. Yield 83%

The benzamide derivatives of formula I have been found to display useful herbicidal activity against a variety of weed species commonly occurring in areas utilized for growing desired crops such as the cereal grains and the like. The selective herbicidal activity of the compounds has been analyzed in a number of standard greenhouse and open field tests. One such test was a broad spectrum greenhouse test carried out by filling square plastic pots with a sterilized sandy loam soil and planting seeds of tomato, large crabgrass and pigweed. Each pot was fertilized with 158 mg of a 23—21—17 fertilizer four days before treatment with test compound.

The test compounds were formulated for application by dissolving each compound in a solution comprising 100 ml of acetone and 100 ml of ethanol plus 1.174 g of Toximul R and 0.783 g of Toximul S. (Toximul R and Toximul S are proprietary blends of anionic and nonionic surfactants manufactured by Stepan Chemical Company, Northfield, Illinois). Each test compound was dissolved in the diluent at the rate of 20 mg per 2 ml of solvent, and then the solution was diluted to 8 ml with deionized water. The formulated compounds were applied to the planted pots at an effective rate of 16.8 kg/ha (15 pounds per acre).

Test compounds were applied postemergence to some planted pots and preemergence to others. The postemergence applications were made by spraying the solution containing the test compound over the emerged plants about twelve days after the seeds were planted. Preemergence applications were sprayed on the soil one day after the seeds were planted.

Following application of the test compounds the pots were placed in a greenhouse and watered as necessary. Observations were conducted about 10—13 days following application of the test compounds, and untreated control plants were used as standards in each observation. The degree of herbicidal activity of the test compounds were determined by rating the treated plants on a scale of 1—5. On this scale, "1" indicates no plant injury; "2" is slight injury; "3" is moderate plant injury; "4" is severe injury and "5" is death of the plant or no seedling emergence. The type of plant injury sustained by the plants was tabulated using the following code letters:

A = abscission of leaves
B = burned
C = chlorosis
D = death
E = epinasty
F = formative effects
G = dark green
I = increased plant growth
L = local necrosis
N = no germination
P = purple pigmentation
R = reduced germination
S = stunting
U = unclassified injury

Compounds causing plant injuries rated 4 or 5 are considered very active, while compounds rated 2 or 3 are considered moderately active.

Table I below presents the herbicidal activity of typical benzamides of the invention when evaluated in a broad spectrum screen as described above.

TABLE I

| Compound of Example No. | Preemergence Tomato | Preemergence Large Crabgrass | Preemergence Pigweed | Postemergence Tomato | Postemergence Large Crabgrass | Postemergence Pigweed |
|---|---|---|---|---|---|---|
| 1 | 5N | 5N | 5N | 4BS | 4Bs | 4BS |
| 2 | 1 | 1 | 5N | 1 | 1 | 1 |
| 3 | 4RS | 3S | 4RS | 1 | 1 | 1 |
| 4 | 1 | 1 | 5N | 5D | 1 | 4CBS |
| 5 | 1 | 1 | 3RS | 4CBS | 4BS | 5D |
| 9 | 5N | 2CBS | 5N | 2CBS | 1 | 1 |
| 10 | 3RS | 3RS | 4RS | 3GS | 1 | 1 |
| 14 | 5N | 5N | 5N | 4BS | 2BS | 4BS |
| 16 | 5N | 4RS | 5N | 4BS | 3CBS | 4CBS |
| 17 | 5N | 4S | 5N | 3SB | 2SB | 3SB |
| 18 | 2S | 2S | 3S | 5D | 2S | 4SB |
| 21 | 1 | 1 | 5N | 1 | 1 | 1 |
| 23 | 5N | 5N | 1 | 2S | 1 | 1 |
| 24 | 5N | 5N | 5N | 4CFS | 2BS | 3FS |
| 25 | 5N | 5N | 5N | 5D | 1 | 5D |
| 29 | 5N | 5N | 5N | 3FS | 1 | 2FS |
| 33 | 1 | 1 | 5N | 1 | 1 | 1 |
| 37 | 1 | 5D | 1 | 1 | 2CBS | 1 |
| 54 | 5N | 5N | 5N | 4CB | 1 | 4CB |
| 55 | 1 | 5N | 5N | 4FS | 2S | 3FS |
| 57 | 1 | 4RS | 4RS | 1 | 1 | 1 |
| 58 | 5N | 5N | 5N | 2FS | 1 | 1 |
| 61 | 5N | 4RS | 5N | 4BS | 1 | 4BS |
| 64 | 4RS | 1 | 5N | 2FS | 1 | 1 |
| 67 | 1 | 2S | 5N | 1 | 1 | 1 |
| 69 | 1 | 1 | 1 | 1 | 2BS | 2BS |
| 71 | 5N | 1 | 5N | 4CBS | 2PBS | 4BS |
| 73 | 4RS | 2RS | 4RS | 4BS | 2PBS | 4BS |
| 74 | 5N | 5N | 5N | 3BS | 2BS | 3BS |

TABLE I (Cont'd.)

| Compounds of Example No. | Preemergence | | | Postemergence | | |
|---|---|---|---|---|---|---|
| | Tomato | Large Crabgrass | Pigweed | Tomato | Large Crabgrass | Pigweed |
| 75 | 4RS | 1 | 5N | 2CBS | 1 | 1 |
| 76 | 5N | 5N | 5N | 4BS | 2BS | 5D |
| 79 | 3S | 4S | 3S | 1 | 1 | 1 |
| 80 | 5N | 3RS | 5N | 1 | 1 | 1 |
| 82 | 5N | 1 | 5N | 2CS | 1 | 1 |
| 86 | 1 | 1 | 1 | 2CBS | 2BS | 3BS |
| 90 | 5N | 5N | 5N | 4CFS | 2BS | 3FS |
| 95 | 5N | 5N | 5N | 4BS | 3BS | 4FS |
| 98 | 1 | 1 | 5N | 1 | 1 | 1 |
| 99 | 1 | 1 | 5N | 1 | 1 | 1 |
| 105 | 4RS | 1 | 4RS | 1 | 1 | 3RS |
| 107 | 4RS | 3RS | 4RS | 2S | 2BS | 2FS |
| 108 | 3RS | 2S | 5N | 1 | 1 | 2BS |
| 109 | 5N | 3RS | 5N | 4FS | 2PBS | 4FS |
| 116 | 5N | 4RS | 4RS | 3GFS | 2BS | 1 |
| 117 | 3RS | 2RS | 4RS | 4BS | 2BS | 3BS |
| 121 | 5N | 5N | 5N | 4CBS | 2BS | 5D |
| 131 | 1 | 1 | 1 | 1 | 1 | 1 |
| 133 | 1 | 1 | 1 | 1 | 1 | 1 |
| 163 | 5N | 5N | 5N | 3CBS | 1 | 2CS |

A similar greenhouse study utilizing seven seed species was carried out to further evaluate preemergence and postemergence herbicidal activity of the benzamide derivatives of this invention. The compounds to be evaluated were formulated according to the procedure outlined above, except that about 4 g/100 ml of the compound were dissolved in the surfactant-containing solvent, and about 1 part of the organic solution was diluted with 12 parts of water before application to seeded containers. The compounds were applied at the effective rate of 9 kg/ha (8 lbs/acre). Typical results of such evaluation are presented in Table II below.

TABLE II

| Compound of Example Number | Preemergence Corn | Preemergence Large Crabgrass | Preemergence Pigweed | Preemergence Foxtail | Preemergence Velvet Leaf | Preemergence Morningglory | Preemergence Zinnia | Postemergence Corn | Postemergence Large Crabgrass | Postemergence Pigweed | Postemergence Foxtail | Postemergence Velvet Leaf | Postemergence Morningglory | Postemergence Zinnia |
|---|---|---|---|---|---|---|---|---|---|---|---|---|---|---|
| 1 | 5 | 5 | 5 | 5 | 5 | 4 | 5 | 1 | 3 | 3 | 2 | 1 | 2 | 3 |
| 2 | 1 | 1 | 4 | 1 | 1 | 2 | 3 | 1 | 1 | 1 | 1 | 1 | 1 | 2 |
| 4 | 1 | 2 | 5 | 2 | 2 | 3 | 5 | 1 | 1 | 1 | 1 | 1 | 1 | 1 |
| 6 | 1 | 1 | 5 | 1 | 1 | 1 | 2 | 2 | 1 | 1 | 1 | 1 | 1 | 2 |
| 8 | 1 | 1 | 3 | 1 | 1 | 2 | 1 | 1 | 1 | 1 | 1 | 1 | 1 | 1 |
| 9 | 3 | 4 | 5 | 4 | 1 | 1 | 4 | 1 | 1 | 1 | 1 | 1 | 1 | 1 |
| 10 | 2 | 5 | 5 | 3 | 3 | 2 | 4 | 1 | 1 | 3 | 1 | 1 | 2 | 3 |
| 11 | 1 | 3 | 5 | 2 | 1 | 2 | 1 | 1 | 1 | 2 | 1 | 1 | 1 | 3 |
| 12 | 2 | 4 | 5 | 3 | 3 | 3 | 2 | 2 | 1 | 3 | 1 | 2 | 2 | 3 |
| 13 | 3 | 4 | 5 | 4 | 1 | 1 | 1 | 1 | 1 | 3 | 1 | 1 | 2 | 2 |
| 14 | 4 | 5 | 5 | 5 | 4 | 5 | 5 | 2 | 1 | 4 | 1 | 2 | 3 | 3 |
| 16 | 3 | 4 | 5 | 4 | 3 | 3 | 3 | 2 | 1 | 1 | 1 | 1 | 2 | 3 |
| 17 | 2 | 4 | 5 | 4 | 3 | 2 | 2 | 2 | 1 | 3 | 2 | 2 | 2 | 3 |
| 19 | 5 | 5 | 5 | 5 | 5 | 5 | 5 | 3 | 3 | 4 | 2 | 3 | 3 | 3 |
| 22 | 3 | 4 | 5 | 2 | 2 | 4 | 4 | 1 | 1 | 3 | 1 | 1 | 2 | 3 |
| 23 | 2 | 5 | 5 | 4 | 3 | 3 | 3 | 1 | 1 | 3 | 1 | 1 | 1 | 2 |
| 24 | 3 | 4 | 5 | 3 | 3 | 3 | 5 | 1 | 1 | 2 | 1 | 2 | 1 | 2 |
| 26 | 1 | 3 | 5 | 2 | 2 | 1 | 3 | 1 | 1 | 2 | 1 | 1 | 1 | 2 |
| 30 | 2 | 1 | 1 | 2 | 1 | 1 | 2 | 1 | 1 | 1 | 1 | 1 | 1 | 1 |

TABLE II (Continued)

| Compound of Example Number | Preemergence | | | | | | | Postemergence | | | | | | |
|---|---|---|---|---|---|---|---|---|---|---|---|---|---|---|
| | Corn | Large Crabgrass | Pigweed | Foxtail | Velvet Leaf | Morningglory | Zinnia | Corn | Large Crabgrass | Pigweed | Foxtail | Velvet Leaf | Morningglory | Zinnia |
| 33 | 1 | 1 | 2 | 1 | 3 | 1 | 5 | 1 | 1 | 1 | 1 | 1 | 1 | 1 |
| 37 | 2 | 3 | 2 | 2 | 1 | 1 | 1 | 2 | 1 | 1 | 1 | 2 | 2 | 2 |
| 41 | 1 | 1 | 4 | 1 | 1 | 1 | 1 | 1 | 1 | 3 | 1 | 1 | 1 | 3 |
| 47 | 2 | 1 | 1 | 3 | 2 | 1 | 3 | 1 | 1 | 1 | 1 | 1 | 1 | 1 |
| 48 | 1 | 2 | 2 | 2 | 1 | 1 | 1 | 1 | 1 | 1 | 1 | 1 | 1 | 1 |
| 49 | 1 | 3 | 2 | 2 | 1 | 1 | 1 | — | — | — | — | — | — | — |
| 50 | 1 | 1 | 2 | 1 | 1 | 1 | 1 | 1 | 1 | 1 | 1 | 1 | 1 | 1 |
| 51 | 1 | 1 | 1 | 1 | 1 | 1 | 1 | 1 | 1 | 1 | 1 | 1 | 2 | 2 |
| 52 | 1 | 1 | 1 | 1 | 1 | 2 | 1 | 2 | 1 | 1 | 1 | 1 | 1 | 1 |
| 53 | 2 | 1 | 1 | 1 | 1 | 1 | 1 | 2 | 1 | 1 | 1 | 1 | 1 | 1 |
| 54 | 4 | 5 | 5 | 5 | 5 | 5 | 5 | 1 | 1 | 1 | 1 | 1 | 1 | 1 |
| 55 | 2 | 5 | 5 | 5 | 5 | 3 | 3 | 1 | 2 | 4 | 1 | 3 | 2 | 3 |
| 56 | 1 | 1 | 1 | 1 | 3 | 1 | 1 | 1 | 1 | 1 | 1 | 1 | 1 | 1 |
| 57 | 1 | 1 | 2 | 2 | 1 | 1 | 1 | 1 | 1 | 1 | 1 | 1 | 1 | 1 |
| 58 | 2 | 5 | 5 | 5 | 4 | 5 | 5 | 1 | 3 | 3 | 2 | 1 | 2 | 1 |
| 59 | 1 | 1 | 3 | 1 | 1 | 1 | 1 | 1 | 1 | 1 | 1 | 1 | 1 | 1 |
| 61 | 4 | 5 | 5 | 5 | 5 | 5 | 5 | 2 | 2 | 4 | 1 | 1 | 2 | 3 |
| 62 | 1 | 3 | 5 | 2 | 2 | 1 | 2 | 2 | 3 | 4 | 1 | 2 | 1 | 1 |
| 63 | 1 | 2 | 4 | 1 | 1 | 2 | 3 | 1 | 1 | 2 | 1 | 1 | 2 | 2 |
| 64 | 1 | 2 | 5 | 1 | 1 | 2 | 2 | 1 | 1 | 2 | 1 | 1 | 1 | 2 |
| 66 | 3 | 4 | 5 | 4 | 3 | 2 | 3 | 2 | 1 | 3 | 1 | 1 | 2 | 2 |

TABLE II (Continued)

| Compound of Example Number | Preemergence | | | | | | | Postemergence | | | | | | |
|---|---|---|---|---|---|---|---|---|---|---|---|---|---|---|
| | Corn | Large Crabgrass | Pigweed | Foxtail | Velvet Leaf | Morningglory | Zinnia | Corn | Large Crabgrass | Pigweed | Foxtail | Velvet Leaf | Morningglory | Zinnia |
| 67 | 1 | 3 | 5 | 2 | 1 | 2 | 2 | 1 | 1 | 1 | 1 | 1 | 1 | 1 |
| 68 | 1 | 2 | 5 | 2 | 2 | 2 | 5 | 1 | 1 | 3 | 1 | 1 | 1 | 2 |
| 69 | 1 | 1 | 2 | 1 | 1 | 2 | 2 | 1 | 1 | 3 | 1 | 1 | 2 | 2 |
| 70 | 1 | 1 | 1 | 1 | 1 | 1 | 1 | 1 | 2 | 2 | 1 | 1 | 1 | 1 |
| 75 | 1 | 1 | 4 | 1 | 1 | 1 | 3 | 1 | 1 | 1 | 1 | 1 | 2 | 1 |
| 77 | 1 | 1 | 2 | 1 | 1 | 1 | 1 | 1 | 1 | 1 | 1 | 1 | 1 | 1 |
| 78 | 1 | 1 | 3 | 1 | 1 | 1 | 1 | 1 | 1 | 1 | 1 | 1 | 1 | 1 |
| 79 | 1 | 1 | 1 | 1 | 1 | 2 | 1 | 1 | 1 | 1 | 1 | 2 | 2 | 2 |
| 80 | 1 | 4 | 5 | 3 | 1 | 2 | 5 | 2 | 1 | 1 | 1 | 1 | 2 | 2 |
| 81 | 1 | 1 | 3 | 1 | 1 | 1 | 1 | 1 | 1 | 1 | 1 | 1 | 1 | 1 |
| 82 | 1 | 4 | 5 | 2 | 2 | 3 | 4 | 1 | 1 | 3 | 1 | 1 | 1 | 1 |
| 83 | 2 | 4 | 5 | 3 | 2 | 3 | 4 | 1 | 1 | 1 | 1 | 1 | 1 | 1 |
| 84 | 1 | 1 | 4 | 1 | 1 | 1 | 1 | 1 | 1 | 1 | 1 | 1 | 1 | 1 |
| 85 | 1 | 1 | 1 | 1 | 1 | 1 | 1 | 1 | 1 | 1 | 1 | 1 | 1 | 1 |
| 86 | 1 | 1 | 4 | 1 | 1 | 1 | 1 | 1 | 1 | 1 | 1 | 1 | 1 | 1 |
| 87 | 1 | 3 | 3 | 2 | 3 | 2 | 3 | 1 | 1 | 1 | 1 | 1 | 1 | 1 |
| 88 | 3 | 5 | 5 | 4 | 3 | 3 | 3 | 1 | 1 | 3 | 1 | 1 | 1 | 2 |
| 92 | 1 | 1 | 3 | 1 | 1 | 1 | 1 | 1 | 1 | 1 | 1 | 1 | 2 | 1 |

TABLE II (Continued)

| Compound of Example Number | Preemergence | | | | | | | Postemergence | | | | | | |
|---|---|---|---|---|---|---|---|---|---|---|---|---|---|---|
| | Corn | Large Crabgrass | Pigweed | Foxtail | Velvet Leaf | Morningglory | Zinnia | Corn | Large Crabgrass | Pigweed | Foxtail | Velvet Leaf | Morningglory | Zinnia |
| 94 | 1 | 1 | 1 | 1 | 1 | 1 | 1 | 1 | 1 | 1 | 1 | 1 | 1 | 1 |
| 95 | 3 | 4 | 5 | 4 | 3 | 3 | 2 | 1 | 2 | 4 | 2 | 3 | 3 | 3 |
| 97 | 1 | 3 | 5 | 2 | 1 | 1 | 3 | 1 | 1 | 1 | 1 | 1 | 1 | 1 |
| 99 | 1 | 4 | 5 | 1 | 1 | 1 | 1 | 1 | 1 | 3 | 1 | 1 | 1 | 2 |
| 100 | 1 | 1 | 1 | 1 | 1 | 1 | 1 | 1 | 1 | 1 | 1 | 2 | 2 | 1 |
| 101 | 1 | 4 | 4 | 1 | 1 | 3 | 2 | 2 | 1 | 2 | 1 | 1 | 1 | 2 |
| 102 | 1 | 2 | 3 | 1 | 1 | 1 | 2 | 1 | 1 | 1 | 1 | 1 | 1 | 1 |
| 103 | 1 | 4 | 1 | 1 | 1 | 1 | 1 | 2 | 1 | 3 | 1 | 1 | 3 | 5 |
| 104 | 1 | 4 | 3 | 3 | 2 | 2 | 1 | 1 | 1 | 1 | 1 | 1 | 2 | 1 |
| 106 | 1 | 1 | 1 | 1 | 1 | 1 | 1 | 1 | 1 | 1 | 1 | 1 | 1 | 2 |
| 110 | 1 | 1 | 1 | 1 | 1 | 1 | 1 | 1 | 1 | 1 | 1 | 1 | 1 | 1 |
| 113 | 2 | 1 | 2 | 1 | 1 | 1 | 1 | 2 | 1 | 1 | 1 | 1 | 1 | 1 |
| 114 | 1 | 4 | 5 | 3 | 2 | 3 | 5 | 1 | 1 | 1 | 1 | 1 | 1 | 1 |
| 115 | 2 | 4 | 4 | 3 | 2 | 4 | 3 | 1 | 1 | 1 | 2 | 1 | 1 | 2 |
| 116 | 1 | 4 | 4 | 2 | 2 | 2 | 1 | 1 | 1 | 1 | 1 | 1 | 1 | 2 |
| 118 | 1 | 2 | 5 | 1 | 2 | 2 | 2 | 1 | 1 | 1 | 1 | 1 | 1 | 1 |
| 125 | 1 | 2 | 2 | 1 | 1 | 1 | 1 | 1 | 1 | 1 | 1 | 1 | 1 | 1 |
| 127 | 1 | 5 | 4 | 1 | 3 | 2 | 2 | 1 | 1 | 3 | 1 | 1 | 1 | 2 |
| 128 | 1 | 1 | 2 | 1 | 1 | 1 | 1 | 1 | 1 | 1 | 1 | 1 | 1 | 1 |
| 130 | 1 | 1 | 1 | 1 | 1 | 1 | 1 | 1 | 1 | 1 | 1 | 1 | 1 | 1 |
| 136 | 1 | 1 | 1 | 1 | 1 | 1 | 1 | 1 | 3 | 1 | 1 | 1 | 1 | 2 |
| 137 | 1 | 2 | 2 | 1 | 1 | 1 | 1 | 1 | 1 | 1 | 1 | 1 | 1 | 1 |

The herbicidal activity of a number of the benzamide derivatives of the invention was evaluated at various application rates in a multiple-species greenhouse test. Several additional weed and crop species were utilized to determine the herbicidal selectivity of the compounds. The compounds were formulated as described above, and applied preemergence to seeded flats. The results for several compounds of the invention are presented below in Table III.

TABLE III

| Compound of Example No. | Rate of Application kg/ha | (Lbs/Acre) | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wildoat | Velvetleaf | Jimson Weed | Morningglory | Zinnia |
|---|---|---|---|---|---|---|---|---|---|---|---|---|---|---|---|---|---|---|---|---|---|---|
| | | | Preemergence | | | | | | | | | | | | | | | | | | | |
| 1 | 1.1 | (1) | 4 | 1 | 4 | 1 | 2 | 5 | 3 | 5 | 5 | 5 | 5 | 5 | 5 | 5 | 5 | 3 | 4 | 5 | 3 | 5 |
| | 0.6 | (0.5) | 2 | 1 | 3 | 1 | 5 | 5 | 3 | 2 | 5 | 4 | 5 | 5 | 5 | 5 | 5 | 2 | 3 | 5 | 2 | 5 |
| | 0.28 | (0.25) | 1 | 1 | 2 | 1 | 5 | 5 | 5 | 4 | 4 | 4 | 5 | 5 | 5 | 5 | 4 | 1 | 2 | 5 | 2 | 3 |
| 3 | 1.1 | (1) | 1 | 1 | 2 | 1 | 1 | 5 | 2 | 3 | 4 | 4 | 5 | 4 | 3 | 4 | 1 | 1 | 3 | 1 | 1 | 2 |
| | 0.6 | (0.5) | 1 | 1 | 1 | 1 | 1 | 5 | 1 | 2 | 3 | 3 | 5 | 3 | 3 | 4 | 1 | 1 | 2 | 1 | 1 | 2 |
| | 0.28 | (0.25) | 1 | 1 | 1 | 1 | 1 | 5 | 1 | 1 | 1 | 1 | 4 | 1 | 2 | 5 | 1 | 1 | 2 | 1 | 1 | 2 |
| 4 | 1.1 | (1) | 1 | 1 | 1 | 1 | 4 | 5 | 1 | 1 | 2 | 1 | 5 | 1 | 4 | 5 | 1 | 1 | 1 | 4 | 1 | 5 |
| | 0.6 | (0.5) | 1 | 1 | 1 | 1 | 4 | 5 | 1 | 1 | 3 | 1 | 5 | 1 | 3 | 4 | 1 | 1 | 1 | 4 | 1 | 4 |
| | 0.28 | (0.25) | 1 | 1 | 1 | 1 | 1 | 4 | 1 | 1 | 1 | 1 | 5 | 1 | 2 | 3 | 2 | 1 | 1 | 3 | 1 | 1 |
| 5 | 4.5 | (4) | 1 | 1 | 1 | 1 | 1 | 5 | 1 | 1 | 1 | 2 | 5 | 1 | 2 | 5 | 1 | 1 | 1 | 3 | 1 | 1 |
| | 1.1 | (1) | 1 | 1 | 1 | 1 | 1 | 3 | 1 | 1 | 1 | 1 | 5 | 1 | 1 | 3 | 1 | 1 | 1 | 2 | 2 | 2 |
| 6 | 4.5 | (4) | 2 | 2 | 4 | 3 | 4 | 5 | 1 | 2 | 4 | 1 | 5 | 2 | 5 | 5 | 4 | 1 | 2 | 5 | 4 | 5 |
| | 2.2 | (2) | 2 | 2 | 2 | 1 | 2 | 5 | 1 | 1 | 4 | 1 | 5 | 1 | 3 | 5 | 1 | 1 | 1 | 4 | 1 | 5 |
| | 1.1 | (1) | 1 | 1 | 2 | 1 | 1 | 4 | 1 | 2 | 4 | 1 | 5 | 1 | 3 | 4 | 1 | 1 | 1 | 2 | 1 | 4 |
| | 0.6 | (0.5) | 1 | 1 | 2 | 1 | 1 | 3 | 1 | 2 | 1 | 1 | 5 | 1 | 3 | 2 | 1 | 1 | 2 | 1 | 1 | 3 |
| | 0.28 | (0.25) | 1 | 1 | 1 | 1 | 1 | 4 | 1 | 1 | 1 | 1 | 5 | 1 | 3 | 2 | 1 | 1 | 1 | 2 | 1 | 2 |

TABLE III (Continued)

| Compound of Example No. | Rate of Application kg/ha | (Lbs/Acre) | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wildoat | Velvetleaf | Jimson Weed | Morningglory | Zinnia |
|---|---|---|---|---|---|---|---|---|---|---|---|---|---|---|---|---|---|---|---|---|---|---|
| | | | Preemergence | | | | | | | | | | | | | | | | | | | |
| 9 | 4.5 | (4) | 2 | 1 | 3 | 1 | 3 | 5 | 2 | 2 | 4 | 4 | 5 | 3 | 5 | 5 | 3 | 1 | 2 | 5 | 3 | 5 |
| | 1.1 | (1) | 1 | 1 | 2 | 1 | 2 | 5 | 2 | 2 | 2 | 3 | 5 | 3 | 5 | 5 | 2 | 1 | 1 | 4 | 2 | 4 |
| | 0.6 | (0.5) | 1 | 1 | 1 | 1 | 2 | 5 | 2 | 1 | 2 | 3 | 5 | 2 | 4 | 5 | 1 | 1 | 2 | 3 | 1 | 3 |
| | 0.14 | (0.125) | 1 | 1 | 1 | 1 | 1 | 5 | 1 | 1 | 1 | 1 | 5 | 1 | 1 | 5 | 1 | 1 | 1 | 1 | 1 | 1 |
| 11 | 4.5 | (4) | 1 | 1 | 1 | 1 | 1 | 3 | 1 | 1 | 1 | 1 | 3 | 1 | 4 | 3 | 2 | 1 | 1 | 2 | 1 | 2 |
| | 2.2 | (2) | 1 | 1 | 1 | 1 | 1 | 2 | 1 | 2 | 1 | 2 | 5 | 1 | 4 | 3 | 1 | 1 | 1 | 2 | 1 | 2 |
| | 1.1 | (1) | 1 | 1 | 1 | 1 | 1 | 1 | 1 | 1 | 1 | 1 | 4 | 1 | 3 | 2 | 1 | 1 | 1 | 1 | 1 | 1 |
| 13 | 4.5 | (4) | 2 | 1 | 2 | 1 | 1 | 4 | 1 | 3 | 1 | 3 | 5 | 3 | 2 | 3 | 2 | 1 | 1 | 1 | 1 | 1 |
| | 2.2 | (2) | 1 | 1 | 2 | 1 | 1 | 4 | 1 | 2 | 2 | 2 | 4 | 3 | 1 | 2 | 2 | 1 | 1 | 3 | 1 | 1 |
| | 1.1 | (1) | 1 | 1 | 1 | 1 | 1 | 3 | 1 | 1 | 2 | 1 | 3 | 1 | 1 | 1 | 1 | 1 | 1 | 1 | 1 | 1 |
| 14 | 1.1 | (1) | 2 | 2 | 3 | 1 | 5 | 5 | 2 | 5 | 5 | 5 | 5 | 5 | 5 | 5 | 5 | 1 | 3 | 5 | 3 | 4 |
| | 0.6 | (0.5) | 2 | 1 | 2 | 1 | 1 | 3 | 1 | 2 | 3 | 3 | 5 | 3 | 4 | 3 | 4 | 1 | 2 | 3 | 2 | 4 |
| | 0.28 | (0.25) | 1 | 1 | 2 | 1 | 2 | 2 | 3 | 1 | 2 | 3 | 5 | 3 | 5 | 2 | 2 | 1 | 2 | 4 | 1 | 2 |
| 15 | 1.1 | (1) | 1 | 1 | 1 | 1 | 1 | 3 | 1 | 1 | 4 | 3 | 5 | 2 | 5 | 4 | 1 | 1 | 2 | 4 | 2 | 5 |
| | 0.6 | (0.5) | 1 | 1 | 1 | 1 | 1 | 4 | 1 | 2 | 4 | 1 | 5 | 2 | 4 | 3 | 1 | 1 | 2 | 2 | 1 | 2 |
| | 0.28 | (0.25) | 1 | 1 | 1 | 1 | 1 | 3 | 1 | 1 | 5 | 1 | 5 | 1 | 4 | 5 | 1 | 1 | 2 | 5 | 1 | 2 |

TABLE III (Continued)

| Compound of Example No. | Rate of Application kg/ha | (Lbs/Acre) | Preemergence Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wildoat | Velvetleaf | Jimson Weed | Morningglory | Zinnia |
|---|---|---|---|---|---|---|---|---|---|---|---|---|---|---|---|---|---|---|---|---|---|---|
| 16 | 1.1 | (1) | 1 | 1 | 2 | 1 | 3 | 5 | 1 | 3 | 5 | 4 | 5 | 4 | 5 | 5 | 4 | 1 | 2 | 5 | 1 | 4 |
| | 0.6 | (0.5) | 1 | 1 | 2 | 1 | 5 | 5 | 1 | 1 | 2 | 2 | 5 | 1 | 4 | 5 | 1 | 1 | 1 | 5 | 1 | 2 |
| | 0.28 | (0.25) | 1 | 1 | 1 | 1 | 4 | 4 | 1 | 1 | 1 | 1 | 5 | 1 | 4 | 5 | 1 | 1 | 1 | 2 | 1 | 1 |
| 17 | 1.1 | (1) | 1 | 2 | 3 | 2 | 3 | 5 | 1 | 1 | 5 | 4 | 5 | 4 | 4 | 4 | 2 | 2 | 2 | 3 | 2 | 1 |
| | 0.6 | (0.5) | 2 | 1 | 3 | 1 | 3 | 3 | 1 | 3 | 4 | 3 | 5 | 4 | 5 | 5 | 2 | 2 | 3 | 2 | 2 | 1 |
| | 0.28 | (0.25) | 2 | 2 | 3 | 2 | 5 | 5 | 1 | 1 | 1 | 3 | 5 | 4 | 3 | 4 | 1 | 1 | 1 | 2 | 1 | 1 |
| 21 | 1.1 | (1) | 1 | 1 | – | 1 | 1 | 2 | 1 | 3 | 2 | 2 | 5 | 2 | 3 | 5 | 1 | 1 | 1 | 2 | 1 | 1 |
| | 0.6 | (0.5) | 1 | 2 | – | 1 | 2 | 5 | 1 | 1 | 1 | 1 | 5 | 1 | 1 | 2 | 1 | 1 | 1 | 1 | 1 | 1 |
| | 0.28 | (0.25) | 1 | 1 | – | 1 | 1 | 1 | 1 | 1 | 1 | 1 | 4 | 1 | 1 | 2 | 1 | 1 | 1 | 1 | 1 | 1 |
| 23 | 1.1 | (1) | 1 | 1 | 2 | 1 | 1 | 5 | 3 | 3 | 5 | 5 | 5 | 5 | 4 | 4 | 4 | 2 | 2 | – | 3 | 3 |
| | 0.6 | (0.5) | 2 | 1 | 1 | 1 | 1 | 5 | 1 | 3 | 5 | 4 | 5 | 3 | 3 | 3 | 3 | 2 | 3 | – | 1 | 2 |
| | 0.28 | (0.25) | 2 | 1 | 1 | 1 | 1 | 5 | 2 | 2 | 3 | 4 | 5 | 3 | 2 | 2 | 2 | 2 | 1 | – | 1 | 2 |
| | 0.14 | (0.125) | 1 | 1 | 1 | 1 | 1 | 5 | 1 | 1 | 1 | 1 | 5 | 4 | 2 | 5 | 3 | 1 | 2 | 2 | 2 | 4 |
| 24 | 1.1 | (1) | 1 | 1 | 1 | 1 | 1 | 4 | 1 | 1 | 1 | 1 | 5 | 1 | 3 | 4 | 1 | 1 | 1 | 3 | 1 | 4 |
| | 0.6 | (0.5) | 1 | 1 | 1 | 1 | 1 | 4 | 1 | 3 | 2 | 1 | 5 | 2 | 4 | 4 | 1 | 1 | 1 | – | 1 | 4 |
| | 0.28 | (0.25) | 1 | 2 | 1 | 1 | 1 | 3 | 1 | 2 | 2 | 1 | 5 | 2 | 3 | 5 | 2 | 1 | 1 | – | 1 | 4 |

TABLE III (Continued)

| Compound of Example No. | Rate of Application kg/ha | (Lbs/Acre) | Preemergence Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wildoat | Velvetleaf | Jimson Weed | Morningglory | Zinnia |
|---|---|---|---|---|---|---|---|---|---|---|---|---|---|---|---|---|---|---|---|---|---|---|
| 26 | 4.5 | (4) | 1 | 2 | 2 | 1 | 1 | 5 | 1 | 1 | 1 | 1 | 5 | 2 | 4 | 3 | 1 | 1 | 1 | 1 | 1 | 2 |
| | 2.2 | (2) | 1 | 1 | 1 | 1 | 1 | 4 | 1 | 1 | 1 | 1 | 5 | 2 | 3 | 2 | 1 | 1 | 1 | 2 | 1 | 2 |
| | 1.1 | (1) | 1 | 1 | 1 | 1 | 1 | 2 | 1 | 1 | 1 | 1 | 2 | 2 | 3 | 1 | 1 | 1 | 1 | 1 | 1 | 2 |
| 27 | 4.5 | (4) | 1 | 2 | 3 | 1 | 3 | 5 | 2 | 4 | 4 | 2 | 5 | 1 | 5 | 5 | 2 | 1 | 2 | 4 | 2 | 5 |
| | 2.2 | (2) | 1 | 1 | 4 | 1 | 3 | 5 | 1 | 2 | 4 | 1 | 5 | 1 | 5 | 5 | 1 | 1 | 2 | 5 | 3 | 3 |
| | 1.1 | (1) | 1 | 1 | 1 | 1 | 5 | 5 | 1 | 2 | 3 | 1 | 5 | 1 | 5 | 5 | 1 | 1 | 1 | 2 | 2 | 5 |
| | 0.6 | (0.5) | 1 | 1 | 2 | 1 | 4 | 4 | 1 | 3 | 1 | 1 | 5 | 1 | 3 | 4 | 1 | 1 | 2 | 3 | 1 | 4 |
| 29 | 1.1 | (1) | 2 | 1 | 1 | 1 | 1 | 5 | 2 | 4 | 2 | 4 | 5 | 4 | 3 | 4 | 3 | 2 | 3 | 2 | 1 | 2 |
| | 0.6 | (0.5) | 2 | 1 | 1 | 1 | 1 | 2 | 1 | 3 | 1 | 3 | 5 | 3 | 2 | 4 | 2 | 2 | 3 | 2 | 1 | 2 |
| | 0.28 | (0.25) | 2 | 1 | 1 | 1 | 1 | 2 | 1 | 2 | 1 | 3 | 4 | 3 | 2 | 3 | 1 | 2 | 2 | 1 | 1 | 1 |
| 31 | 1.1 | (1) | 1 | 1 | 2 | 1 | 1 | 4 | 1 | 2 | 1 | 2 | 4 | 2 | 2 | 3 | 1 | 1 | 1 | 3 | 1 | 1 |
| | 0.6 | (0.5) | 1 | 1 | 1 | 1 | 1 | 4 | 1 | 1 | 1 | 1 | 2 | 1 | 1 | 3 | 1 | 1 | 1 | 1 | 1 | 1 |
| | 0.28 | (0.25) | 1 | 1 | 1 | 1 | 1 | 1 | 1 | 1 | 1 | 1 | 1 | 1 | 2 | 2 | 1 | 1 | 1 | 1 | 1 | 1 |
| 33 | 2.2 | (2) | 1 | 1 | 1 | 1 | 1 | 4 | 1 | 1 | 1 | 1 | 4 | 1 | 2 | 3 | 2 | 1 | 1 | 1 | 1 | 1 |
| | 1.1 | (1) | 1 | 1 | 1 | 1 | 1 | 3 | 1 | 1 | 1 | 1 | 4 | 1 | 1 | 2 | 1 | 1 | 1 | 1 | 1 | 1 |

TABLE III (Continued)

| Compound of Example No. | Rate of Application kg/ha | (Lbs/Acre) | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wildoat | Velvetleaf | Jimsonweed | Morningglory | Zinnia |
|---|---|---|---|---|---|---|---|---|---|---|---|---|---|---|---|---|---|---|---|---|---|---|
| | | | Preemergence | | | | | | | | | | | | | | | | | | | |
| 34 | 1.1 | (1) | 2 | 1 | 3 | 1 | 2 | 4 | 1 | 1 | 1 | 2 | 5 | 4 | 2 | 4 | 4 | 1 | 3 | 3 | 2 | 2 |
| | 0.6 | (0.5) | 1 | 1 | 1 | 1 | 1 | 3 | 1 | 1 | 1 | 2 | 5 | 4 | 2 | 3 | 1 | 1 | 1 | 1 | 2 | 3 |
| 35 | 1.1 | (1) | 1 | 1 | 1 | 1 | 1 | 2 | 1 | 1 | 1 | 1 | 5 | 1 | 1 | 4 | 1 | 1 | 2 | 1 | 1 | 1 |
| | 0.6 | (0.5) | 1 | 1 | 1 | 1 | 1 | 1 | 1 | 1 | 1 | 1 | 2 | 1 | 1 | 3 | 1 | 1 | 1 | 1 | 1 | 1 |
| 36 | 1.1 | (1) | 1 | 1 | 1 | 1 | 1 | 4 | 1 | 1 | 1 | 1 | 1 | 1 | 1 | 2 | 1 | 1 | 1 | 1 | 1 | 1 |
| | 0.6 | (0.5) | 1 | 1 | 1 | 1 | 1 | 3 | 1 | 1 | 1 | 1 | 1 | 1 | 1 | 1 | 1 | 1 | 1 | 1 | 1 | 1 |
| 42 | 1.1 | (1) | 1 | 1 | 1 | 1 | 1 | 4 | 2 | 2 | 2 | 1 | 5 | 2 | 4 | 3 | 1 | 1 | 1 | 1 | 1 | 2 |
| | 0.6 | (0.5) | 1 | 1 | 1 | 1 | 1 | 4 | 1 | 1 | 1 | 1 | 5 | 1 | 1 | 3 | 1 | 1 | 1 | 1 | 1 | 3 |
| 43 | 4.5 | (4) | 1 | 1 | 1 | 1 | 1 | 4 | 1 | 2 | 1 | 1 | 5 | 3 | 1 | 4 | 2 | 1 | 1 | 1 | 1 | 1 |
| | 2.2 | (2) | 1 | 1 | 1 | 1 | 1 | 2 | 1 | 1 | 1 | 1 | 4 | 3 | 2 | 4 | 1 | 1 | 1 | 1 | 1 | 1 |

TABLE III (Continued)

| Compound of Example No. | Rate of Application kg/ha | (Lbs/Acre) | Preemergence: Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wildoat | Velvetleaf | Jimson Weed | Morningglory | Zinnia |
|---|---|---|---|---|---|---|---|---|---|---|---|---|---|---|---|---|---|---|---|---|---|---|
| 61 | 4.5 | (4) | 4 | 1 | 4 | 3 | 5 | 5 | 4 | 5 | 5 | 5 | 5 | 5 | 5 | 5 | 5 | 4 | 5 | 5 | 3 | 5 |
| | 0.6 | (0.5) | 2 | 1 | 4 | 2 | 4 | 5 | 3 | 2 | 2 | 3 | 5 | 4 | 4 | 5 | 4 | 1 | 2 | 4 | 2 | 3 |
| | 0.14 | (0.125) | 1 | 1 | 1 | 1 | 4 | 2 | 1 | 2 | 1 | 1 | 5 | 3 | 4 | 5 | 1 | 1 | 2 | 2 | 1 | 2 |
| 62 | 4.5 | (4) | 2 | 1 | 2 | 1 | 1 | 1 | 1 | 1 | 1 | 1 | 4 | 2 | 1 | 3 | 1 | 1 | 2 | 3 | 1 | 1 |
| | 2.2 | (2) | 1 | 1 | 1 | 1 | 1 | 1 | 1 | 1 | 1 | 1 | 2 | 2 | 1 | 2 | 1 | 1 | 1 | 2 | 1 | 1 |
| | 1.1 | (1) | 1 | 1 | 1 | 1 | 1 | 1 | 1 | 1 | 1 | 1 | 1 | 1 | 1 | 1 | 1 | 1 | 1 | 1 | 1 | 1 |
| 63 | 1.1 | (1) | 1 | 1 | 1 | 1 | 1 | 1 | 1 | 1 | 1 | 1 | 4 | 1 | 3 | 4 | 1 | 1 | 1 | 1 | 1 | 1 |
| | 0.6 | (0.5) | 1 | 1 | 1 | 1 | 1 | 1 | 1 | 2 | 1 | 2 | 1 | 1 | 1 | 4 | 1 | 1 | 1 | 2 | 1 | 1 |
| | 0.28 | (0.25) | 1 | 1 | 1 | 1 | 1 | 1 | 1 | 1 | 1 | 1 | 1 | 1 | 1 | 4 | 1 | 1 | 1 | 1 | 1 | 1 |
| 64 | 4.5 | (4) | 1 | 1 | 1 | 1 | 1 | 4 | — | 2 | 2 | 2 | 5 | 1 | 3 | 4 | 2 | 1 | 1 | 3 | 2 | 4 |
| | 2.2 | (2) | 1 | 1 | 1 | 1 | 1 | 3 | 1 | 1 | 2 | 1 | 5 | 1 | 2 | 3 | 1 | 1 | 1 | 3 | 1 | 2 |
| | 1.1 | (1) | 1 | 1 | 1 | 1 | 1 | 1 | 1 | 2 | 1 | 1 | 3 | 2 | 3 | 3 | 1 | 1 | 2 | 1 | 1 | 1 |
| 66 | 4.5 | (4) | 4 | 2 | 4 | 2 | 5 | 5 | 4 | 3 | 5 | 5 | 5 | 5 | 5 | 5 | 5 | 2 | 4 | 5 | 3 | 3 |
| | 1.1 | (1) | 2 | 2 | 3 | 2 | 3 | 5 | 3 | 2 | 2 | 4 | 5 | 4 | 3 | 5 | 4 | 2 | 2 | 1 | 1 | 3 |
| | 0.6 | (0.5) | 2 | 1 | 3 | 1 | 1 | 5 | 4 | 2 | 2 | 4 | 5 | 4 | 4 | 4 | 4 | 2 | 3 | 5 | 2 | 2 |
| | 0.14 | (0.125) | 1 | 2 | 1 | 1 | 1 | 2 | 1 | 2 | 1 | 1 | 5 | 3 | 2 | 5 | 1 | 1 | 1 | 1 | 1 | 1 |

TABLE III (Continued)

| Compound of Example No. | Rate of Application kg/ha | (Lbs/Acre) | Preemergence | | | | | | | | | | | | | | | | | | | |
|---|---|---|---|---|---|---|---|---|---|---|---|---|---|---|---|---|---|---|---|---|---|---|
| | | | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wildoat | Velvetleaf | Jimson Weed | Morningglory | Zinnia |
| 68 | 4.5 | (4) | 1 | 1 | 1 | 1 | 1 | 4 | 2 | 2 | 2 | 2 | 5 | 2 | 4 | 5 | 1 | 1 | 1 | 3 | 1 | 3 |
| | 2.2 | (2) | 1 | 1 | 1 | 1 | 1 | 4 | 1 | 1 | 1 | 1 | 5 | 1 | 3 | 3 | 1 | 1 | 1 | 2 | 1 | 2 |
| | 1.1 | (1) | 1 | 1 | 1 | 1 | 1 | 4 | 1 | 1 | 3 | 1 | 5 | 1 | 4 | 4 | 1 | 1 | 1 | 2 | 1 | 2 |
| | 0.6 | (0.5) | 1 | 1 | 1 | 1 | 1 | 1 | 1 | 1 | 1 | 1 | 5 | 1 | 2 | 4 | 1 | 1 | 1 | 1 | 1 | 2 |
| 73 | 4.5 | (4) | 1 | 2 | 3 | 1 | 3 | 3 | 1 | 3 | 3 | 3 | 5 | 4 | 5 | 5 | 2 | 2 | 1 | 5 | 2 | 4 |
| | 1.1 | (1) | 1 | 1 | 2 | 1 | 1 | 4 | 2 | 1 | 2 | 2 | 5 | 2 | 5 | 5 | 1 | 1 | 1 | 5 | 1 | 1 |
| | 0.6 | (0.5) | 1 | 1 | 1 | 1 | 1 | 1 | 1 | 1 | 3 | 1 | 5 | 1 | 3 | 5 | 1 | 1 | 1 | 3 | 1 | 1 |
| 74 | 1.1 | (1) | 1 | 1 | 2 | 1 | 3 | 5 | 3 | 2 | 3 | 3 | 5 | 3 | 3 | 5 | 2 | 3 | 3 | 4 | 2 | 1 |
| | 0.6 | (0.5) | 1 | 1 | 2 | 1 | 2 | 5 | 4 | 1 | 1 | 3 | 5 | 4 | 3 | 5 | 2 | 1 | 1 | 5 | 1 | 2 |
| | 0.28 | (0.25) | 2 | 1 | 1 | 1 | 1 | 5 | 1 | 1 | 5 | 2 | 5 | 1 | 2 | 5 | 1 | 1 | 1 | 4 | 1 | 1 |
| 76 | 1.1 | (1) | 1 | 1 | 2 | 2 | 4 | 4 | 1 | 2 | 5 | 5 | 5 | 5 | 5 | 5 | 4 | 2 | 2 | 4 | 2 | 3 |
| | 0.6 | (0.5) | 1 | 1 | 1 | 1 | 1 | 2 | 2 | 3 | 2 | 1 | 5 | 3 | 3 | 4 | 1 | 1 | 2 | 4 | 1 | 2 |
| | 0.28 | (0.25) | 1 | 1 | 1 | 1 | 1 | 2 | 1 | 1 | 3 | 1 | 4 | 1 | 3 | 2 | 1 | 1 | 1 | 3 | 1 | 1 |
| 83 | 1.1 | (1) | 1 | 1 | 1 | 1 | 4 | 4 | 1 | 1 | 2 | 2 | 5 | 1 | 3 | 5 | 1 | 1 | 1 | 5 | 1 | 3 |
| | 0.6 | (0.5) | 1 | 1 | 1 | 1 | 1 | 2 | 1 | 1 | 1 | 1 | 5 | 2 | 3 | 4 | 1 | 1 | 1 | 4 | 1 | 2 |
| | 0.28 | (0.25) | 1 | 1 | 1 | 1 | 1 | 2 | 1 | 1 | 1 | 1 | 5 | 2 | 2 | 3 | 1 | 1 | 1 | 1 | 1 | 1 |
| 89 | 4.5 | (4) | 1 | 1 | – | 1 | 1 | 1 | – | 2 | 1 | 1 | 4 | 1 | 2 | 3 | 1 | 1 | 1 | – | 1 | 1 |
| | 2.2 | (2) | 1 | 1 | – | 1 | 1 | 1 | 1 | 1 | 1 | 1 | 5 | 1 | 2 | 5 | 1 | 1 | 1 | – | 1 | 1 |
| | 1.1 | (1) | 1 | 1 | – | 1 | 1 | 1 | 2 | 1 | 1 | 1 | 1 | 1 | 1 | 4 | 1 | 1 | 1 | – | 1 | 1 |

TABLE III (Continued)

| Compound of Example No. | Rate of Application kg/ha | (Lbs/Acre) | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wildoat | Velvetleaf | Jimson Weed | Morningglory | Zinnia |
|---|---|---|---|---|---|---|---|---|---|---|---|---|---|---|---|---|---|---|---|---|---|---|
| | | | Preemergence | | | | | | | | | | | | | | | | | | | |
| 96 | 4.5 | (4) | 1 | 1 | 1 | 1 | 3 | 5 | 1 | 2 | 2 | 4 | 4 | 4 | 3 | 5 | 2 | 2 | 5 | 3 | 3 | 3 |
| | 2.2 | (2) | 2 | 1 | 1 | 1 | 3 | 5 | 1 | 2 | 1 | 4 | 3 | 4 | 3 | 4 | 2 | 2 | 2 | 2 | 1 | 2 |
| | 1.1 | (1) | 1 | 1 | 1 | 1 | 1 | 5 | 1 | 2 | 1 | 5 | 3 | 4 | 3 | 5 | 2 | 2 | 2 | 2 | 1 | 2 |
| | 0.6 | (0.5) | 1 | 1 | 3 | 1 | 2 | 4 | 1 | 2 | 1 | 3 | 5 | 3 | 4 | 4 | 2 | 2 | 2 | 3 | 2 | 2 |
| | 0.28 | (0.25) | 1 | 1 | 1 | 1 | 1 | 2 | 1 | 2 | 1 | 1 | 4 | 1 | 2 | 3 | 1 | 1 | 1 | 3 | 1 | 2 |
| 97 | 4.5 | (4) | 1 | 1 | 1 | 1 | 1 | 1 | 1 | 1 | 1 | 1 | 3 | 1 | 1 | 3 | 1 | 1 | 1 | 1 | 1 | 1 |
| | 2.2 | (2) | 1 | 1 | 1 | 1 | 1 | 1 | 1 | 1 | 1 | 1 | 2 | 1 | 1 | 3 | 1 | 1 | 1 | 1 | 1 | 1 |
| | 1.1 | (1) | 1 | 1 | 1 | 1 | 1 | 1 | 1 | 1 | 1 | 1 | 1 | 1 | 1 | 3 | 1 | 1 | 1 | 1 | 1 | 1 |
| 98 | 2.1 | (1) | 1 | 1 | 2 | 1 | 1 | 5 | 1 | 1 | 2 | 1 | 5 | 1 | 2 | 5 | 1 | 1 | 1 | 3 | 1 | 2 |
| | 0.6 | (0.5) | 1 | 1 | 1 | 1 | 1 | 4 | 1 | 1 | 1 | 1 | 5 | 2 | 2 | 4 | 1 | 1 | 1 | 3 | 1 | 2 |
| | 0.18 | (0.25) | 1 | 1 | 1 | 1 | 1 | 3 | 1 | 1 | 1 | 1 | 4 | 1 | 2 | 2 | 1 | 1 | 1 | 1 | 1 | 2 |
| 104 | 1.1 | (1) | 1 | 1 | 1 | 1 | 1 | 1 | 1 | 1 | 1 | 1 | 5 | 1 | 2 | 5 | 1 | 1 | 1 | 1 | 1 | 1 |
| | 0.6 | (0.5) | 1 | 1 | 1 | 1 | 1 | 1 | 1 | 1 | 1 | 1 | 3 | 1 | 1 | 3 | 1 | 1 | 1 | 1 | 1 | 1 |
| | 0.28 | (0.25) | 1 | 1 | 1 | 1 | 1 | 1 | 1 | 1 | 1 | 1 | 2 | 1 | 1 | 3 | 1 | 1 | 2 | 1 | 1 | 1 |
| 111 | 4.5 | (4) | 1 | 1 | 1 | 1 | 4 | 5 | 1 | 1 | 5 | 1 | 5 | 1 | 5 | 5 | 2 | 1 | 1 | 5 | 1 | 5 |
| | 2.2 | (2) | 1 | 1 | 1 | 1 | 4 | 5 | 1 | 1 | 3 | 1 | 5 | 1 | 5 | 5 | 1 | 1 | 1 | 5 | 1 | 2 |
| | 1.1 | (1) | 1 | 1 | 1 | 1 | 2 | 5 | 1 | 1 | 4 | 1 | 5 | 1 | 5 | 5 | 1 | 1 | 1 | 4 | 1 | 1 |

TABLE III (Continued)

| Compound of Example No. | Rate of Application kg/ha | (Lbs/Acre) | Preemergence Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wildoat | Velvetleaf | Jimson Weed | Morningglory | Zinnia |
|---|---|---|---|---|---|---|---|---|---|---|---|---|---|---|---|---|---|---|---|---|---|---|
| 112 | 1.1 | (1) | 1 | 1 | 2 | 1 | 3 | 4 | 1 | 1 | 2 | 4 | 5 | 5 | 5 | 5 | 2 | 3 | 2 | 5 | 2 | 1 |
| | 0.6 | (0.5) | 1 | 1 | 2 | 1 | 1 | 2 | 1 | 1 | 3 | 4 | 5 | 1 | 2 | 5 | 1 | 1 | 1 | 1 | 1 | 1 |
| | 0.28 | (0.25) | 1 | 1 | 1 | 1 | 1 | 1 | 3 | 1 | 1 | 2 | 5 | 3 | 3 | 5 | 1 | 2 | 1 | 3 | 1 | 1 |
| 114 | 2.2 | (2) | 1 | 1 | 1 | 1 | — | 5 | 1 | 2 | 2 | 1 | 5 | 1 | 3 | 3 | 1 | 1 | 1 | — | 1 | 2 |
| | 1.1 | (1) | 1 | 1 | — | 1 | 1 | 4 | 1 | 2 | 3 | 1 | 5 | 1 | 3 | 5 | 1 | 2 | 1 | — | 1 | 2 |
| | 0.6 | (0.5) | 1 | 1 | — | 1 | 1 | 4 | 1 | 3 | 1 | 1 | 4 | 1 | 2 | 3 | 1 | 1 | 1 | 2 | 1 | 2 |
| | 0.28 | (0.25) | 1 | 1 | — | 1 | 1 | 1 | 1 | 1 | 1 | 1 | 3 | 1 | 1 | 2 | 1 | 1 | 1 | 1 | 1 | 1 |
| 115 | 4.5 | (4) | 1 | 1 | — | 1 | 1 | 5 | 1 | 1 | 3 | 2 | 5 | 2 | 1 | 3 | 3 | 1 | 1 | — | 1 | 1 |
| | 2.2 | (2) | 1 | 1 | 1 | 1 | 1 | 1 | 1 | 1 | 1 | 1 | 4 | 1 | 1 | 1 | 1 | 1 | 1 | — | 1 | 2 |
| | 1.1 | (1) | 1 | 1 | — | 1 | 1 | 1 | 1 | 1 | 1 | 1 | 3 | 1 | 2 | 1 | 1 | 1 | 1 | — | 1 | 1 |
| 116 | 2.2 | (2) | 1 | 1 | 2 | 1 | 4 | 3 | 1 | 2 | 4 | 1 | 5 | 2 | 4 | 4 | 3 | 2 | 2 | 5 | 3 | 5 |
| | 1.1 | (1) | 1 | 1 | 1 | 1 | 5 | 4 | 1 | 1 | 3 | 1 | 3 | 3 | 3 | 3 | 1 | 1 | 1 | — | 1 | 2 |
| | 0.6 | (0.5) | 1 | 1 | 1 | 1 | 2 | 2 | 1 | 1 | 1 | 1 | 1 | 3 | 2 | 1 | 1 | 1 | 1 | — | 1 | 3 |
| | 0.28 | (0.25) | 1 | 1 | 1 | 1 | 1 | 1 | 1 | 1 | 2 | 1 | 3 | 1 | 2 | 2 | 1 | 3 | 1 | — | 2 | 1 |
| 118 | 1.1 | (1) | 1 | 1 | 2 | 1 | 4 | 5 | 2 | 2 | 1 | 1 | 5 | 2 | 3 | 5 | 1 | 1 | 2 | 1 | 1 | 4 |
| | 0.6 | (0.5) | 1 | 1 | 1 | 1 | 4 | 4 | 2 | 2 | 1 | 1 | 5 | 1 | 2 | 5 | 1 | 1 | 1 | 1 | 1 | 1 |
| | 0.28 | (0.25) | 1 | 1 | 1 | 1 | 1 | 4 | 1 | 1 | 1 | 1 | 5 | 1 | 1 | 2 | 1 | 1 | 1 | 1 | 1 | 1 |

TABLE III (Continued)

| Compound of Example No. | Rate of Application kg/ha | (Lbs/Acre) | Preemergence Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wildoat | Velvetleaf | Jimson Weed | Morningglory | Zinnia |
|---|---|---|---|---|---|---|---|---|---|---|---|---|---|---|---|---|---|---|---|---|---|---|
| 119 | 9 | (8) | 1 | – | – | – | – | – | – | – | – | – | – | 4 | – | 2 | 3 | – | 4 | – | 2 | 4 |
| | 4.5 | (4) | 1 | 1 | 1 | 1 | 1 | 1 | 1 | 1 | 1 | 1 | 1 | 1 | 1 | 2 | 1 | 1 | 1 | 1 | 1 | 2 |
| | 2.2 | (2) | 1 | 1 | 1 | 1 | 1 | 1 | 1 | 1 | 1 | 1 | 1 | 1 | 1 | 2 | 1 | 1 | 1 | 1 | 1 | 1 |
| 122 | 1.1 | (1) | 1 | 1 | 1 | 1 | 3 | 2 | 1 | 1 | 1 | 1 | 5 | 1 | 4 | 5 | 1 | 1 | 1 | 1 | 1 | 1 |
| | 0.6 | (0.5) | 1 | 1 | 1 | 1 | 1 | 2 | 1 | 1 | 1 | 1 | 5 | 1 | 3 | 5 | 1 | 1 | 1 | 1 | 1 | 1 |
| | 0.28 | (0.25) | 1 | 1 | 1 | 1 | 1 | 1 | 1 | 1 | 1 | 1 | 3 | 1 | 1 | 2 | 1 | 1 | 1 | 1 | 1 | 1 |
| 123 | 1.1 | (1) | 1 | 1 | 1 | 1 | 1 | 4 | 1 | 1 | 1 | 3 | 5 | 1 | 3 | 5 | 1 | 1 | 1 | 1 | 1 | 1 |
| | 0.6 | (0.5) | 1 | 1 | 1 | 1 | 1 | 1 | 1 | 1 | 1 | 1 | 5 | 1 | 1 | 3 | 1 | 1 | 1 | 1 | 1 | 1 |
| 124 | 1.1 | (1) | 1 | 1 | 3 | 1 | 2 | 4 | 1 | 3 | 4 | 2 | 5 | 5 | 4 | 4 | 4 | 1 | 3 | 4 | 1 | 1 |
| | 0.6 | (0.5) | 1 | 2 | 2 | 1 | 1 | 3 | 1 | 3 | 2 | 1 | 5 | 3 | 3 | 5 | 4 | 1 | 1 | 1 | 1 | 1 |
| 126 | 2.2 | (2) | 1 | 2 | 4 | 1 | 1 | 2 | 1 | 1 | 3 | 4 | 4 | 4 | 4 | 4 | 2 | 1 | 1 | 3 | 1 | 1 |
| | 1.1 | (1) | 1 | 1 | 2 | 1 | 1 | 1 | 1 | 1 | 3 | 3 | 5 | 4 | 5 | 5 | 3 | 1 | 2 | 2 | 1 | 1 |

As pointed out earlier, most of the compounds provided by this invention are potent herbicidal agents when applied at low rates to soil or plant surfaces pre- or post-emergence. Many of the compounds are even more potent when applied to soil and incorporated therein prior to planting of crop seeds. Pre-plant incorporation is particularly preferred when employing a benzamide which displays less than desirable potency or selectivity when surface applied. For example, the compound of Example 52, namely N-[3-(1-methylethyl)-5-isoxazolyl]-2,6-dimethoxybenzamide, failed to retard the vigor of tomato, large crabgrass or pigweed when applied both pre- and post-emergence at 16.8 kg/ha (15 pounds per acre). When the compound was applied at 16.8 kg/ha (15 pounds per acre) and incorporated into the soil prior to seeding, it totally retarded the growth of tomato, wild mustard and lambsquarter, and demonstrated significant herbicidal activity against pigweed, sugarbeets and Jimsonweed.

The herbicidal activity of several compounds provided herein has been determined following pre-plant incorporation of the benzamide derivative. The evaluations were carried out by first formulating an appropriate amount of test compound in 2.5 ml. of a fifty percent mixture of acetone and ethanol. The solution was diluted to 12.5 ml. by the addition of deionized water. The formulated test compound was sprayed onto five quarts of screened, autoclaved greenhouse potting soil. The test compound was incorporated into the soil mixture by tumbling the mixture in a modified cement mixer. The treated soil was transferred to greenhouse flats, and the flats were seeded to various weed and crop species. The seeded flats were maintained in a greenhouse, with sub-irrigation as needed. Plant injury ratings attributable to the test compound were made sixteen days post treatment and planting, utilizing a scale of 0 to 10, wherein 0 is no injury and 10 represents death of the plant species. The results of typical pre-plant soil incorporation studies are presented below in Table IV.

TABLE IV

Pre-Plant soil incorporation

| Compound of Example No. | Rate of application kg/ha | (lbs/Acre) | Alfalfa | Pigweed | Wild Mustard | Lambsquarter | Sugarbeets | Tomatoes | Jimsonweed |
|---|---|---|---|---|---|---|---|---|---|
| 46 | 16.8 | (15) | 0 | 5 | 8 | 8 | 6 | 6 | 5 |
| 48 | 16.8 | (15) | 0 | 5 | 10 | 10 | 6 | 5 | 0 |
| 52 | 16.8 | (15) | 0 | 6 | 10 | 10 | 7 | 10 | 5 |
| 65 | 16.8 | (15) | 6 | 10 | 10 | 10 | 6 | 6 | 5 |
| 78 | 16.8 | (15) | 6 | 10 | 10 | 10 | 10 | 5 | 3 |
| 85 | 9.0 | (8) | 0 | 4 | 10 | 9 | 4 | 5 | 2 |
| 100 | 16.8 | (15) | 2 | 8 | 10 | 10 | 9 | 8 | 6 |
| 106 | 16.8 | (15) | 7 | 10 | 10 | 10 | 8 | 6 | 6 |
| 110 | 16.8 | (15) | 0 | 9 | 10 | 8 | 8 | 10 | 9 |
| 120 | 4.5 | (4) | 0 | 3 | 10 | 6 | 3 | 3 | 0 |
| 132 | 9.0 | (8) | 0 | 7 | 10 | 10 | 4 | 3 | 1 |
| 134 | 16.8 | (15) | 0 | 9 | 10 | 10 | 9 | 8 | 5 |
| 135 | 16.8 | (15) | 0 | 2 | 10 | 6 | 4 | 6 | 0 |

Several of the preferred benzamides of the invention have been evaluated in a number of open field studies to determine efficacy, selectivity and crop tolerances. In a typical field trial, the benzamides were applied pre-emergence as an aqueous spray in soil seeded to cereal grains and having weed species present common with such crops. The studies routinely involved randomized blocks with four replicates. Observations were made for grain crop vigor, weed control, crop injury, crop emergence, and root injury.

One such field study was carried out in Great Britain to determine the efficacy and selectivity of preferred benzamides when surface applied pre-emergence to soil seeded to common wheat. Observations were made twenty-five days post-application to determine the vigor of the treated wheat plants compared to untreated controls, and to determine the amount of control of various weed species effected by the benzamides. The results of the field test are presented below in Table V. The data presented for crop vigor is the comparison of treated wheat to untreated wheat, the untreated controls being assigned a vigor rating of 100 percent. Weed control ratings are given in percentage of control compared to untreated control plots based upon visual inspection.

TABLE V

| Compound Applied | Rate kg/ha | Crop Vigor Rating | Blackgrass Control | Catchweed Bedstraw Control | Wild Chamomile Control | Ladysthumb Control | Chickweed Control |
|---|---|---|---|---|---|---|---|
| N-[3-(1-ethyl-1-methylbutyl)-5-isoxazolyl]-2,6-dimethoxybenzamide (Compound of Ex. 14) | 0.25 | 96 | 40 | 20 | 100 | 91 | 100 |
| | 0.50 | 93 | 68 | 8 | 100 | 100 | 100 |
| | 0.75 | 96 | 5 | 5 | 100 | 100 | 100 |
| | 1.0 | 93 | 73 | 27 | 100 | 100 | 100 |
| N-[3-(1-methylcyclo-hexyl)-5-isoxazolyl]-2,6-dimethoxybenzamide (Compound of Ex. 17) | 0.25 | 97 | 56 | 0 | 100 | 100 | 100 |
| | 0.50 | 98 | 59 | 3 | 100 | 100 | 100 |
| | 0.75 | 94 | 61 | 3 | 100 | 100 | 100 |
| | 1.0 | 93 | 54 | 5 | 100 | 100 | 100 |
| N-[3-(1-ethylcyclo-hexyl)-5-isoxazolyl]-2,6-dimethoxybenzamide (Compound of Ex. 19) | 0.25 | 96 | 83 | 18 | 100 | 94 | 100 |
| | 0.50 | 97 | 55 | 28 | 100 | 98 | 100 |
| | 0.75 | 96 | 83 | 25 | 100 | 100 | 100 |
| | 1.0 | 93 | 84 | 63 | 100 | 100 | 100 |
| N-[3-(1,1-dimethyl-ethyl)-5-isoxazolyl]-2,6-dimethoxybenzamide (Compound of Ex. 6) | 1.0 | 96 | 60 | 10 | 100 | 83 | 100 |
| N-[3-(1-ethyl-1-methyl-propyl)-5-ixoxazolyl]-2,6-dimethoxybenzamide (Compound of Ex. 1) | 1.0 | 92 | 66 | 93 | 100 | 100 | 100 |
| Untreated control | | 100 | 0 | 0 | 0 | 0 | 0 |

A field study was carried out in Brazil to evaluate the herbicidal activity and crop tolerances of certain preferred benzamides compared to untreated controls and to commercial herbicides trifluralin and metribuzin. The herbicides were applied as an aqueous spray and were pre-plant incorporated with a disc and harrow. Treated and untreated plots were individually seeded to peanuts, soybeans, cotton and corn. Observations were then made sixteen and thirty-six days post-treatment. Treated plots were visually compared with untreated controls to determine percent of crop emergence, percent of crop injury, percent of crop stand, and percent of root injury. Table VI which follows presents the effects of various herbicides on crops. Table VII presents the degree to which the various herbicides controlled weed species commonly encountered in the indicated crops.

TABLE VI

Peanuts

| Compound of Example No. | Rate kg/ha | % Crop Emergence at 16 Days | % Crop Injury at 16 Days | % Crop Injury at 36 Days | % Crop Stand at 16 Days | % Root Injury at 36 Days |
|---|---|---|---|---|---|---|
| 1 | 0.13 | 93 | 7 | 17 | 101 | 0 |
| | 0.25 | 83 | 20 | 27 | 87 | 0 |
| | 0.50 | 70 | 37 | 37 | 82 | 40 |
| | 1.0 | 70 | 40 | 53 | 83 | 23 |
| 6 | 1.0 | 73 | 20 | 3 | 83 | 0 |
| | 2.0 | 83 | 13 | 13 | 90 | 10 |
| 28 | 0.13 | 83 | 20 | 13 | 92 | 0 |
| | 0.25 | 93 | 13 | 17 | 102 | 0 |
| | 0.50 | 83 | 17 | 20 | 92 | 0 |
| | 1.0 | 80 | 30 | 30 | 105 | 10 |
| 61 | 0.13 | 93 | 7 | 0 | 92 | 0 |
| | 0.25 | 87 | 13 | 13 | 98 | 0 |
| | 0.50 | 77 | 17 | 20 | 84 | 0 |
| | 1.0 | 67 | 37 | 40 | 80 | 28 |
| Trifluralin | 0.96 | 80 | 27 | 20 | 80 | 0 |
| Metribuzin | 0.49 | 77 | 50 | 47 | 92 | 0 |
| Untreated Control | | 100 | 0 | 0 | 100 | 0 |

TABLE VI

Soybeans

| Compound of Example No. | Rate kg/ha | % Crop Emergence at 16 Days | % Crop Injury at 16 Days | % Crop Injury at 36 Days | % Crop Stand at 16 Days | % Root Injury at 36 Days |
|---|---|---|---|---|---|---|
| 1 | 0.13 | 53 | 37 | 30 | 51 | 0 |
| | 0.25 | 43 | 47 | 33 | 42 | 0 |
| | 0.50 | 37 | 57 | 53 | 26 | 0 |
| | 1.0 | 13 | 87 | 77 | 7 | 0 |
| 6 | 1.0 | 93 | 7 | 3 | 88 | 0 |
| | 2.0 | 97 | 0 | 0 | 103 | 0 |
| 28 | 0.13 | 87 | 10 | 10 | 81 | 0 |
| | 0.25 | 80 | 13 | 27 | 89 | 0 |
| | 0.50 | 60 | 40 | 27 | 46 | 0 |
| | 1.0 | 43 | 53 | 40 | 30 | 0 |
| 61 | 0.13 | 97 | 0 | 0 | 99 | 0 |
| | 0.25 | 87 | 17 | 13 | 77 | 0 |
| | 0.50 | 63 | 30 | 27 | 54 | 0 |
| | 1.0 | 37 | 60 | 47 | 31 | 0 |
| Trifluralin | 0.96 | 73 | 30 | 17 | 60 | 0 |
| Metribuzin | 0.49 | 90 | 13 | 7 | 87 | 0 |
| Untreated Control | | 100 | 0 | 0 | 100 | 0 |

TABLE VI

Cotton

| Compound of Example No. | Rate kg/ha | % Crop Emergence at 16 Days | % Crop Injury at 16 Days | % Crop Injury at 36 Days | % Crop Stand at 16 Days | % Root Injury at 36 Days |
|---|---|---|---|---|---|---|
| 1 | 0.13 | 83 | 17 | 17 | 96 | 0 |
| | 0.25 | 80 | 23 | 30 | 85 | 0 |
| | 0.50 | 70 | 33 | 30 | 80 | 0 |
| | 1.0 | 43 | 53 | 43 | 38 | 0 |
| 6 | 1.0 | 90 | 10 | 0 | 97 | 0 |
| | 2.0 | 87 | 13 | 3 | 104 | 0 |
| 28 | 0.13 | 97 | 3 | 0 | 112 | 0 |
| | 0.25 | 90 | 10 | 23 | 92 | 0 |
| | 0.50 | 80 | 27 | 13 | 97 | 0 |
| | 1.0 | 90 | 13 | 20 | 84 | 0 |
| 61 | 0.13 | 93 | 7 | 0 | 117 | 0 |
| | 0.25 | 73 | 17 | 7 | 97 | 0 |
| | 0.50 | 90 | 17 | 7 | 94 | 0 |
| | 1.0 | 80 | 17 | 23 | 94 | 0 |
| Trifluralin | 0.96 | 93 | 20 | 23 | 108 | 0 |
| Metribuzin | 0.49 | 37 | 73 | 67 | 24 | 0 |
| Control | | 100 | 0 | 0 | 100 | 0 |

TABLE VI

Corn

| Compound of Example No. | Rate kg/ha | % Crop Emergence at 16 Days | % Crop Injury at 16 Days | % Crop Injury at 36 Days | % Crop Stand at 16 Days | % Root Injury at 36 Days |
|---|---|---|---|---|---|---|
| 1 | 0.13 | 93 | 30 | 27 | 86 | 0 |
| | 0.25 | 90 | 33 | 30 | 88 | 0 |
| | 0.50 | 87 | 43 | 37 | 89 | 0 |
| | 1.0 | 77 | 57 | 60 | 85 | 17 |
| 6 | 1.0 | 97 | 3 | 3 | 94 | 0 |
| | 2.0 | 93 | 13 | 3 | 90 | 3 |
| 28 | 0.13 | 97 | 7 | 10 | 94 | 0 |
| | 0.25 | 83 | 33 | 17 | 84 | 0 |
| | 0.50 | 90 | 13 | 20 | 79 | 0 |
| | 1.0 | 90 | 27 | 27 | 91 | 0 |
| 61 | 0.13 | 97 | 17 | 3 | 95 | 0 |
| | 0.25 | 93 | 13 | 7 | 95 | 0 |
| | 0.50 | 87 | 30 | 13 | 86 | 0 |
| | 1.0 | 90 | 40 | 30 | 83 | 0 |
| Trifluralin | 0.96 | 77 | 47 | 37 | 74 | 3 |
| Metribuzin | 0.49 | 77 | 60 | 57 | 84 | 0 |
| Control | | 100 | 0 | 0 | 100 | 0 |

TABLE VII

| Compound of Example No. | Rate kg/ha | | | | | | |
|---|---|---|---|---|---|---|---|
| 1 | 0.13 | 7 | 0 | 7 | 17 | 0 | 10 |
| | 0.25 | 83 | 10 | 27 | 37 | 20 | 10 |
| | 0.50 | 90 | 17 | 57 | 60 | 27 | 0 |
| | 1.0 | 93 | 60 | 73 | 80 | 70 | 13 |
| 6 | 1.0 | 0 | 0 | 20 | 63 | 0 | 0 |
| | 2.0 | 7 | 0 | 30 | 37 | 0 | 0 |
| 28 | 0.13 | 0 | 0 | 20 | 7 | 7 | 3 |
| | 0.25 | 13 | 0 | 43 | 60 | 7 | 0 |
| | 0.50 | 53 | 3 | 27 | 33 | 0 | 0 |
| | 1.0 | 40 | 10 | 40 | 33 | 23 | 10 |
| 61 | 0.13 | 0 | 0 | 0 | 0 | 0 | 0 |
| | 0.25 | 0 | 0 | 0 | 0 | 0 | 0 |
| | 0.50 | 73 | 10 | 13 | 17 | 37 | 0 |
| | 1.0 | 73 | 30 | 30 | 85 | 37 | 0 |
| Trifluralin | 0.96 | 53 | 7 | 97 | 97 | 87 | 0 |
| Metribuzin | 0.49 | 30 | 27 | 70 | 87 | 83 | 70 |
| Control | | 0 | 0 | 0 | 0 | 0 | 0 |

A field study was carried out in Canada to determine the herbicidal efficacy and crop tolerance of varioius preferred benzamides alone and in combination with the commercial herbicide trifluralin. The herbicides were applied as an aqueous spray, and the field plots were seeded to the cereal grain barley and various weed species, after which the herbicides were soil incorporated utilizing a diamond harrow. Crop injury ratings were made visually on a scale of 0—10, with 0 being no injury and 10 being plant death. All observations reported were made twenty-six days post seeding and application, except crop yield data were determined eighty-four days following treatment. The percent of weed control of various weed species occurring in cereal crops such as barley were determined by visual comparison with untreated control plots. The results of the field test are present below in Table VIII.

TABLE VIII

Barley

| Compound of Example No. | Rate kg/ha | Crop Injury | % Crop Yield | % Weed Control White Mustard | % Weed Control Redroot Pigweed | % Weed Control Lambsquarter | % Weed Control Green Smartweed |
|---|---|---|---|---|---|---|---|
| Trifluralin | 0.7 | 0 | 119 | 0 | 95 | 40 | 0 |
| Control | | 0 | 100 | 0 | 0 | 0 | 0 |
| 6 | 1.1 | 0 | 143 | 95 | 85 | 40 | 20 |
| | 2.2 | 0 | 111 | 95 | 95 | 75 | 0 |
| 6 + Trifluralin | 1.1 + 0.7 | 0 | 119 | 90 | 95 | 40 | 0 |
| | 1.68 + 0.7 | 0 | 107 | 90 | 95 | 40 | 0 |
| | 2.2 + 0.7 | 0 | 99 | 100 | 95 | 70 | 20 |
| 27 | 1.1 | 0 | 147 | 100 | 90 | 50 | 20 |
| | 2.2 | 0 | 87 | 100 | 95 | 75 | 0 |
| 27 + Trifluralin | 0.56 + 0.7 | 0 | 123 | 95 | 90 | 20 | 0 |
| | 1.1 + 0.7 | 0 | 107 | 95 | 90 | 50 | 0 |
| | 1.68 + 0.7 | 0 | 135 | 100 | 95 | 40 | 0 |
| | 2.2 + 0.7 | 0 | 107 | 95 | 95 | 40 | 10 |
| 28 | 1.1 | 0 | 107 | 100 | 70 | 45 | 10 |
| | 2.2 | 0 | 103 | 100 | 95 | 75 | 0 |
| 28 + Trifluralin | 0.56 + 0.7 | 0 | 119 | 95 | 95 | 40 | 0 |
| | 1.1 + 0.7 | 0 | 111 | 95 | 75 | 50 | 0 |
| | 1.68 + 0.7 | 0 | 139 | 95 | 95 | 50 | 0 |
| | 2.2 + 0.7 | 0 | 119 | 100 | 95 | 40 | 10 |
| 101 | 1.1 | 0 | 111 | 95 | 80 | 30 | 0 |
| | 2.2 | 0 | 111 | 95 | 95 | 70 | 0 |
| 101 + Trifluralin | 0.56 + 0.7 | 0 | 119 | 95 | 90 | 40 | 0 |
| | 1.1 + 0.7 | 0 | 119 | 85 | 95 | 75 | 0 |
| | 1.68 + 0.7 | 0 | 131 | 95 | 95 | 50 | 0 |
| | 2.2 + 0.7 | 0 | 123 | 95 | 95 | 40 | 10 |

As can be seen from the data presented above, the benzamide derivatives of this invention exhibit a broad-spectrum of herbicidal activity. Some of the compounds also have demonstrated a certain amount of insecticidal activity. A further important embodiment of this invention is a herbicidal method for killing and controlling the growth of unwanted vegetation which comprises applying to the locus where vegetable control is desired a herbicidally effective amount of a benzamide of this invention. Since the compounds are capable of killing and retarding the growth of a number of weeds and undesirable plants which commonly grow in areas used for growing desired crops such as cereal grains and the like, the method of the invention is particularly applicable to practice in such crops. For example, the benzamides are very effective in killing and retarding the growth of weeds such as barnyardgrass, lambsquarter, yellow foxtail, fall panicum, wild buckwheat, crabgrass, mustard, pigweed, cocklebur, velvetleaf, jimsonweed, morningglory, ragweed, zinnia and a number of other vegetative species which are known to comprise unwanted weed and grass vegetation. While the compounds are toxic to such undesired weeds and grasses, they do not adversely affect the growth of desirable crops such as the cereal grains, for example wheat, barley, oats and rice. The compounds can also be employed in combating undesired vegetative growth in crops such as corn, soybeans, peanuts, cotton and related crops. This herbicidal selectivity is an important benefit to be realized in the practice of the invention. The compounds can also be utilized in the control of unwanted vegetation in non-crop land, for instance in a chemical fallow land program, particularly in fallow wheatland and the like.

The benzamide derivatives of the invention can also be employed in combination with any of several known and commonly used herbicides. For example, a preferred method of herbicidal control according to this invention employs a benzamide of the invention used in conjunction with one or more other herbicides, ideally one or more grass herbicides, for example a dinitroaniline herbicide such as trifluralin, butralin, dinitramine, nitralin, profluoralin, propanil, prodiamine, oryzalin, isopropalin, ethalfluralin, benefin, fluchloralin, pendimethalin, and the like. Other herbicides to be employed in combination with the present benzamides include alachlor, ametryn, amitrole, atrazine, bentazon, bifenox, butachlor, butam, buthidazole, chloramben, chlorbromuron, cyanazine, dichloroprop, dinoseb, fenac, linuron, methazole, metolachlor, metribuzin, nitrofen, pebulate, prometon, prometryn, propachlor, simazine, terbutryn, triallate, trichopyr, and the like.

The use of the benzamide derivatives of this invention in combination with other herbicides provides for an even broader range of weed control than generally can be realized with either the benzamides alone or the other herbicides such as trifluralin alone. Such combinations are particularly preferred when practicing the method in cereal grains.

The benzamide derivatives provided herein can also be applied in conjunction with agricultural chemicals which are utilized to control pests other than unwanted vegetative growth. They lend themselves well to combination with other agricultural chemicals such as insecticides, fungicides, nematicides, and other herbicides as may be desirable and convenient.

In carrying out the method of this invention, a benzamide derivative of the invention can be applied post-emergence to soil or to the foilage of plants whose growth is to be controlled, or pre-emergence to the locus where the growth of unwanted vegetation is to be controlled. A preferred herbicidal method comprises application of the benzamide derivative pre-emergence. The compounds can be applied to the surface of soil in which the growth of unwanted weeds and grasses is to be eliminated or retarded, or if desired the compounds can be incorporated into the soil, for example by use of conventional tillage equipment such as the disc, harrow or the like. Soil incorporation or surface application may be employed when the benzamide derivatives are utilized in combination with other herbicides such as trifluralin or the like. Also, pre-emergence soil incorporation is preferred for compounds having less than desired activity when surface applied but not incorporated.

The compounds of the invention are effective in selectively killing and controlling the growth of unwanted vegetation when applied at a herbicidally-effective rate. In carrying out the method of the invention, and in the case of using the benzamides as pre-emergence herbicides, the active ingredient will be applied to the locus where weed control is desired at a rate of about 0.06 to 16.8 kg/ha (0.05 to about 15 pounds per acre), and more desirably at a rate of about 0.11 to about 5.6 kg/ha (about 0.1 to about 5 pounds per acre). The most active compounds will generally be applied at about 0.11 to about 2.2 kg/ha (about 0.1 to about 2 pounds per acre). It will of course be understood by those having skill in the herbicidal art that such rates of application may vary, depending upon several factors, including the type of locus being treated, the particular benzamide being utilized, the particular weeds or grasses to be killed or controlled, whether the method is being practiced in cropland or fallow land, whether or not the herbicide is being incorporated into the soil, whether or not the benzamide is being utilized in conjunction with other agricultural chemicals, for instance other herbicides such as pendimethalin or simazine. Moreover, while the above-suggested rates are preferred for pre-emergent weed control, the compounds can be applied post-emergence at rates of about 1.1 to about 22 kg/ha (about 1 to about 20 pounds per acre), and more preferably at about 2.2 to about 11 kg/ha (about 2 to about 10 pounds per acre).

The herbicidal method provided by this invention can be carried out employing any of the benzamide herbicides defined herein, but as noted earlier, the method is preferably practiced employing an N-isoxazolyl or thiadiazolyl-2,6-dialkoxybenzamide. An especially preferred method employs a

benzamide selected from N - [3 - (1,1 - dimethylethyl) - 5 - isoxazolyl] - 2,6 - dimethoxybenzamide, N - [3 - (1 - ethyl - 1 - methylpropyl) - 5 - isoxazolyl] - 2,6 - dimethoxybenzamide, N - [5 - (1 - ethyl - 1 - methylpropyl) - 1,3,4 - thiadiazol - 2 - yl] - 2,6 - dimethoxybenzamide, N - [3 - (1 - ethylcyclohexyl) - 5 - isoxazolyl] - 2,6 - dimethoxybenzamide and N - [5 - (1 - ethyl - 1 - methylpropyl) - 3 - isoxazolyl] - 2,6 - dimethoxybenzamide. Such compounds can be employed alone at rates of about 0.11 to about 2.2 kg/ha (about 0.1 to about 2 pounds per acre), or if desired they can be employed in combination with other herbicides such as trifluralin, ametryn, alachlor or the like. When utilized in combination with other herbicides, the exact rate of application for each of the component herbicides will be determined by several factors, including the specific weeds and grasses to be controlled, the particular herbicides used in combination, the weed population, and related factors. Generally, the benzamides will be employed in combination with other herbicides in a ratio of about one to about five parts by weight of benzamide and about one to about five parts by weight of another herbicide. A preferred combination will contain the component herbicides in a weight ratio of about one to one. Such combination will be applied at a rate which is effective to control the unwanted vegetation to the desired degree. The respective herbicides can be applied separately or as a single mixture, for example, a tank mix or the like.

The benzamide derivatives of this invention ideally are suitably formulated for use as described above. Herbicidal formulations comprising a benzamide derivative of formula (I) or an agronomically-acceptable salt thereof, admixed with an agronomically-acceptable diluent, excipient or carrier therefor thus form a further important embodiment of the invention. Such compositions will typically contain from about 1 to about 95 percent by weight of active herbicide, and more generally about 10 to about 60 percent. The formulations may take the form of solid powders, dusts, granules; aqueous or non-aqueous sprays or drenches; concentrates, for example emulsifiable concentrates and the like; wettable powders, and any of the other forms commonly found in the agriculture art for herbicides.

Agronomically acceptable carriers, diluents and excipients commonly utilized to form powders, dusts, granules and the like include talc, diatomaceous earth, silica, pyrophyllite, attapulgite clays and the like. It is often desirable to formulate a compound of the invention as a granule for convenient pre-emergence application to soil. Such formulation generally is prepared by dissolving the benzamide herbicide in a suitable solvent such as kerosene or aromatic naphtha, and applying the solution to a carrier such as montmorillonite clay granules or the like. Another procedure which can be employed comprises simply dispersing an active ingredient in a dough composed of a suitable carrier, for instance damp clay, and then drying and grinding the clay to provide the formulated granules at the desired particle size.

The benzamide derivatives of formula (I) are also conveniently formulated as concentrated compositions which are diluted prior to application, for instance by the addition of water or other suitable diluent to make a dispersion, emulsion or the like. Typical concentrated formulations which are in the form of solids are wettable powders. Wettable powders comprise a finely-divided intimate mixture of an inert carrier or excipient, a benzamide derivative, and a suitable surfactant. Commonly utilized inert carriers which are agronomicaly acceptable include the diatomaceous earths, the various clays such as attapulgite clays or kaolin clays, or silica. Surfactants generally are present in abut 0.5 to about 10 percent by weight of the wettable powder, with the benzamide derivative being present in about 10 to about 60 percent by weight. Any of several known surfactants can be employed in the wettable powder formulations, including any of the sulfonated lignins, the condensed naphthalene-sulfonates, the alkylbenzenesulfonates, the alkyl sulfates, as well as the nonionic surfactants such as the ethylene oxide adducts of phenols. The wettable powders which are thus comprised generally are diluted with water or the like prior to application, so that the dispersion or final mixture contains from about 0.1 to about 5.0 percent by weight of the active benzamide herbicide. Such formulation is then applied as a spray or the like by conventional sprayers and other agricultural chemical applicators.

Another commonly utilized formulation type to be employed with the benzamide derivative are the emulsifiable concentrates. Such formulations are comprised of a benzamide herbicide admixed with a carrier such as a water immiscible organic solvent and an emulsifying agent, in an amount from 0.5 to 10 percent by weight of the emulsifiable concentrate. Water miscible cosolvents may be utilized in conjunction with the immiscible solvent to improve solubility of the benzamide. Commonly used solvents include toluene, xylene, chlorotoluene, benzene, methyl isobutyl ketone, cyclohexanone, naphtha and the like. Aqueous suspensions employing water as a diluent also are contemplated. Emulsifiers which can be employed include the common surfactants and blends of surfactants, for example the alkyl and aryl sulfonates, ethoxylated alkyl phenols, ethoxylated alkyl ethers, the nonoxynols, oxysorbics, allinols, allinates and other nonionic and anionic surfactants. The surfactant may comprise from 0.5 to 10 percent by weight of the suspension. Such emulsifiable concentrates, like the wettable powder formulations, are diluted prior to application, for instance by the addition of the appropriate amount of water to provide a mixture having the desired concentration of active ingredient.

As noted above, the benzamide derivatives of formula (I) are especially useful in combination with other herbicides in order to achieve the range and specificity of weed control desired. A typical method for employing a combination of benzamide herbicide and another herbicide such as trifluralin, terbutryn,

or the like, comprises mixing and diluting just prior to application the individual formulations of the respective individual herbicides. The mixing can be carried out, for example, in the tank of a conventional spraying apparatus. A typical combination of a benzamide of this invention with a herbicide such as trifluralin can be prepared by tank mixing at the site of application a wettable powder formulation of benzamide with an emulsifiable concentrate of trifluralin. Such tank-mixed combination will be applied to soil and if desired incorporated therein at a rate such that each active ingredient is present at about 0.6 to about 2.2 kg/ha (about 0.5 to about 2 pounds per acre). Such combination provides excellent pre-emergence control of a wide variety of unwanted vegetative species.

The following Examples further illustrate typical herbicidal compositions and their use as provided by this invention.

Example 168

Wettable Powder

| Ingredient | Concentration by Weight (Percent) |
|---|---|
| N-[3-(1-ethyl-1-methylpropyl)-5-isoxazolyl]-2,6-dimethoxybenzamide (the compound of Example 1) | 50 |
| Igepal CA—630, a polyoxyethylene octyl phenol nonionic wetting agent — GAF Corporation | 5 |
| Bardens Clay | 45 |

The benzamide herbicide is blended to uniformity with the adjuvants and milled to form a free flowing powder that will be wetted and suspensible in water at or near the site of application to form a sprayable mixture. The formulation is sprayed on the locus where vegetative control is desired at a volume so that the active ingredient is applied at about 0.11 to about 2.2 kg/ha (about 0.1 to about 2 pounds per acre).

Example 169

Wettable Powder

| Ingredient | Percent by Weight |
|---|---|
| N-[5-1-ethyl-1-methylpropyl)-1,3,4-thiadiazol-2-yl]-2,6-dimethoxybenzamide (the compound of Example 61) | 50 |
| Stepanol ME, a technical grade of sodium lauryl sulfate, Stepan Chemical Corp. | 4 |
| Reax 45L, a lignosulfonate dispersant, Westvaco Corporation | 6 |
| Zeolex-7, a precipitated hydrated silicate, J. M. Huber Corporation | 2 |
| Barden Clay, a hydrated aluminum silicate, J.M. Huber Corporation | 38 |

The ingredients are blended and pulverized to provide a free flowing powder that can be suspended in water for convenient spray application. The aqueous spray will be applied at about 5 to about 50 gallons per acre for an application rate of about 1.1 to about 5.6 kg/ha (about 1 to about 5 pounds per acre) of active benzamide.

Example 170

Aqueous Suspension

| Ingredient | Percent by Weight |
|---|---|
| N-[3-(1,1-dimethylethyl)-5-isoxazolyl]-2,6-dimethoxybenzamide (the compound of Example 6) | 43 |
| Polyfon H, an anionic lignosulfonate wetting agent and dispersant, Westvaco Corporation | 4 |
| Min-u-gel 200, a clay-type gelling agent, The Floridin Company | 2 |
| Antifoam C Dow Corning Corp. | 0.05 |
| Water | 50.95 |
| | 100.00% |

The ingredients are mixed and finely ground to uniformity to provide a suspension of active ingredient. The suspension is diluted with additional water and applied as a spray to the locus to be treated.

Example 171

Granular Formulation

| Ingredient | Percent by Weight |
|---|---|
| N-[5-(1,1-dimethyl-2-(methylthio)-ethyl)-1,3,4-thiadiazol-2-yl]-2,6-dimethoxybenzamide (the compound of Example 64) | 5 |
| Heavy aromatic naphtha | 5 |
| Florex 30/60, granular clay, Floridin Co. | 90 |

The benzamide is substantially dissolved in the heavy aromatic naphtha and sprayed onto clay granules of substantially uniform size, ideally less than about 1.0 cubic millimeter, preferably of the 30/60 size range. The granular formulation is surface applied to soil so that the active ingredient is present at about 3.4 to about 11 kg/ha (about 3 to about 10 pounds per acre).

Example 172

Dust Formulation

| Ingredient | Percent by Weight |
|---|---|
| N-[3-(1-ethyl-1-methylpropyl)-1H-pyrazol-5-yl]-2,6-dimethoxybenzamide (the compound of Example 115) | 5 |
| Diatomite, a diatomaceous earth, Witco Chemical Corporation, Inorganic Specialties Division | 95 |

The benzamide herbicide is dry mixed with the diatomaceous earth diluent. The mixture is ground to a fine powder of uniform particle size of about 10 to about 40 microns. If a more concentrated mixture is employed (e.g. about 30 to about 50% active), the formulation can be diluted by the addition of additional excipient such as silica or clay at the site of application. The dust thus formed is surface applied by conventional ground equipment, to the soil surface where vegetative control is desired, or if desired the dust can be applied by conventional aircraft units.

Example 173

Tank-Mix Composition

| Ingredient | Percent by Weight |
|---|---|
| N-[5-(1-ethyl-1-methylpropyl)-4H-1,2,4-triazol-3-yl]-2,6-dimethoxy benzamide (the compound of Example 118) 50WP | 60 |
| N,N-diethyl-2,6-dinitro-3-amino-4-trifluoro-methyl aniline (dinitramine) 2EC | 40 |

A wettable powder formulation containing 50% by weight of the benzamide of Example 118 is dispersed in water and the mixture is agitated while an aqueous suspension of emulsifiable concentrate (2 kg/l (2 lbs/gal)) formulation of the dinitroaniline herbicide is added. The resulting mixture is agitated and sprayed on the soil surface and incorporated therein at the rate of about 1.1 kg/ha (about 1 pound per acre) of benzamide and about 0.75 kg/ha (about 0.67 pound per acre) of the dinitroaniline. The soil can then be seeded to soybeans or the like, and the crop is grown substantially free of unwanted vegetation such as crabgrass, fall panicum, purslane and the like. The yield and quality of the desired crop is thereby greatly improved.

Example 174

Tank-mix Composition

| Ingredient | Percent by Weight |
|---|---|
| N-[5-(1-ethyl-1-methylpropyl)-1,3,4-thiadiazol-2-yl]-2,6-diethylbenzamide (the compound of Example 63) 50WP | 50 |
| N,N-di-*n*-propyl-2,6-dinitro-3-amino 4-trifluoromethylaniline (prodiamine) 50WP | 50 |

A 50% wettable powder formulation of the benzamide is mixed with an aqueous suspension of a 50% wettable powder of the prodiamine. The mixture is agitated and sprayed on soil where weed control is desired. The rate of application will be about 340 l/ha (about 30 gallons per acre) such that each active ingredient is applied at about 0.84 kg/ha (about 0.75 pounds per acre). The mixture is preferably incorporated into the soil, for instance by discing, and then the soil is planted to cotton, soybeans, sugarcane, or the like.

Example 175

Tank-mix Composition

| Ingredient | Percent by Weight |
|---|---|
| N-[5-(1-propylcyclohexyl)-1,3,4-thiadiazol-2-yl]-2,6-dimethoxy-benzamide (the compound of Example 105) 30WP | 30 |
| N,N-di-*n*-propyl-2,6-dinitro-4-trifluoro-methylaniline (trifluralin) 4EC | 70 |

A 30% wettable powder formulation of the benzamide of Example 105 is dispersed in water and agitated while an emulsifiable concentrate (0.4 kg/l (4 lbs/gal)) formulation of trifluralin is added portion-wise. The tank-mixed aqueous mixture is sprayed onto soil and incorporated for the control of chickweed, kochia, pigweed, Russian thistle, lambsquarters and the like. The treated soil can be seeded to cereal grains such as wheat, rye, oats, barley, and the like. Such crops grow substantially unaffected by undesired vegative growth.

Example 176
Combination use

A 4EC formulation of trifluralin is applied to soil prior to seeding. The trifluralin, at about 0.6 kg/ha (about 0.5 pound per acre), is incorporated into the soil by use of a double disc. The soil is seeded to soybeans, and then a 50% wettable powder formulation of N-[3-(1,1-dimethylethyl)-5-isoxazolyl]-2,6-dimethoxybenzamide (the compound of Example 6) is suspended in water and surface sprayed on the planted soil. The active benzamide is present at about 1.7 kg/ha (about 1.5 pounds per acre). The treatment permits the growth of weeds such as lambsquarter, pigweed, and the like.

A particularly preferred herbicidal method according to this invention employs the benzamide of Example 1, namely N-[3-(1-ethyl-1-methylpropyl)-5-isoxazolyl]-2,6-dimethoxybenzamide. Such compound is especially effective when employed in combination with other herbicides such as trifluralin. The use of such benzamide, as well as the combination, is contemplated to be of major importance in the control of unwanted vegetation in cereal grains such as barley and the like.

The benzamides provided by this invention are also useful in the control of unwanted vegetation such as broadleaf weeds in orchards and arbors. The compounds have demonstrated excellent selective herbicidal activity in citrus crops such as orange and lemon orchards. The compounds are also expected to be useful in the control of weeds in vines such as grapes and the like, as well as in crops such as sugarcane. The compounds additionally have exhibited plant growth regulator activity, for instance in the form of improved yields of grain as well as some activity as aquatic herbicides.

**Claims for the Contracting States: BE CH DE FR GB IT LI LU NL SE**

1. An N-arylbenzamide derivative of formula (I):

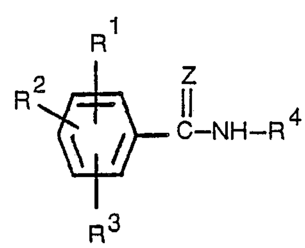

(I)

wherein:

Z is oxygen or sulfur;

$R^1$ is hydrogen, halogen, $C_1$—$C_4$ alkyl, or $C_1$—$C_4$ alkoxy;

$R^2$ is hydrogen, halogen, $C_1$—$C_4$ alkyl, $C_1$—$C_4$ alkoxy, $C_1$—$c_4$ alkylthio or trifluoromethyl;

$R^3$ is hydrogen, halogen, $C_1$—$C_4$ alkyl, $C_1$—$C_4$ alkoxy or $C_1$—$C_4$ alkylthio; provided that when one of $R^1$, $R^2$ or $R^3$ is alkyl, one or both of the other phenyl substituents is other than hydrogen; and when $R^2$ is trifluoromethyl, one or both of $R^1$ and $R^3$ is other than hydrogen;

$R^4$ is an aryl group selected from

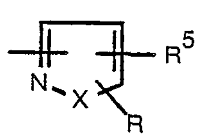 , 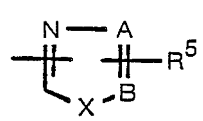 , 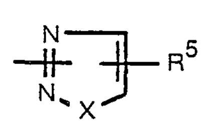 ,

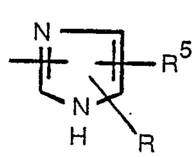 or 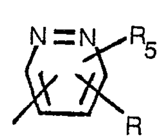

wherein:

A is CH or N and B is CH or N, provided that one of A and B is CH and the other is N;

X is NH, O or S;
R is hydrogen or $C_1$—$C_4$ alkyl;
$R^5$ is

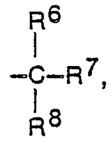 or 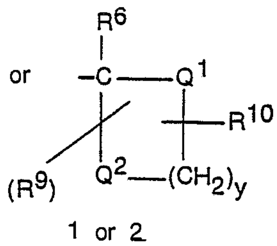

wherein:
y is an integer from 0 to 5;
$R^6$, $R^7$ and $R^8$ independently are hydrogen, $C_1$—$C_{13}$ alkyl, halo-C—$C_{13}$ alkyl, $C_2$—$C_{13}$ alkenyl, $C_2$—$C_{13}$ alkynyl, $C_1$—$C_4$ alkoxy-$C_1$—$C_6$ alkyl, $C_1$—$C_4$ alkylthio-$C_1$—$C_6$ alkyl, $C_1$—$C_6$ alkoxy, $C_2$—$C_4$ alkanoyloxy-$C_1$—$C_6$ alkyl, $C_1$—$C_6$ alkylthio, or

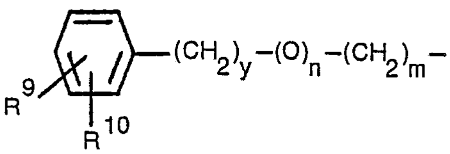 , or 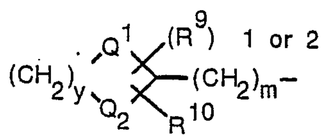

in which
m is an integer from 0 to 4;
n is zero or 1; and
$R^9$ and $R^{10}$ independently are hydrogen, halogen, $C_1$—$C_4$ alkyl or $C_2$—$C_4$ alkenyl;
$Q^1$ and $Q^2$ are independently $CH_2$, O, S, $CH_2O$ or $CH_2S$, provided that when $Q^1$ and $Q^2$ are both other than $CH_2$, y is not 0; and provided that $R^2$ and $R^3$ are other than hydrogen when $R^4$ is

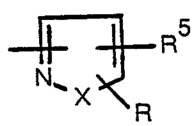 or 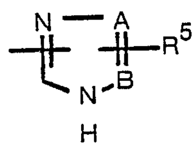

or an agronomically-acceptable salt thereof.

2. An N-arylbenzimide derivative of formula (I) as claimed in claim 1, wherein Z is oxygen, $R^4$ is

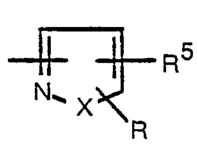 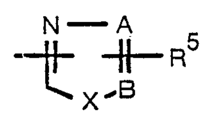

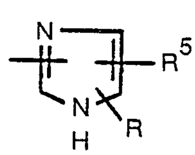 or 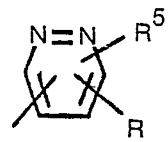

where R is hydrogen; $R^5$ is

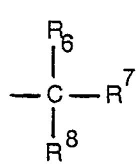 or 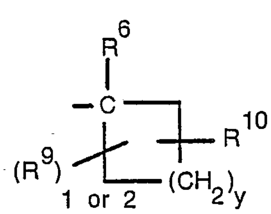

wherein:

$R^6$ and $R^7$ independently are hydrogen, $C_1$—$C_4$ alkyl, $C_2$—$C_4$ alkenyl or $C_2$—$C_4$ alkynyl;

y is an integer from 0 to 4;

$R^8$ is hydrogen, $C_1$—$C_{13}$ alkyl, halo-$C_1$—$C_{13}$ alkyl, $C_2$—$C_{13}$ alkenyl, $C_2$—$C_{13}$ alkynyl, $C_1$—$C_4$ alkoxy-$C_1$—$C_6$ alkyl, $C_1$—$C_4$ alkylthio-$C_1$—$C_6$ alkyl,

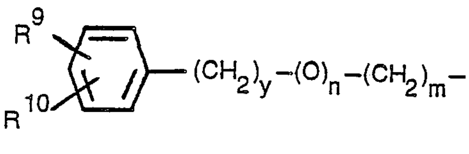

or

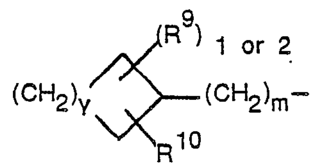

wherein:

m is an integer from 0 to 4;

n is zero or 1; and

$R^9$ and $R^{10}$ independently are hydrogen, halogen, $C_1$—$C_4$ alkyl or $C_2$—$C_4$ alkenyl.

3. An N-arylbenzamide derivative of formula (I) as claimed in claim 1 or 2, wherein Z is oxygen and $R^4$ is

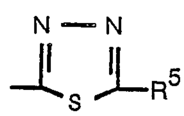

,

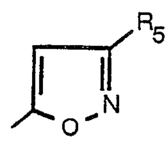

,

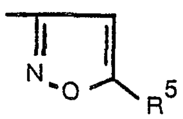

,

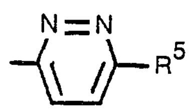

or

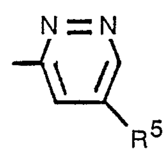

4. An N-arylbenzamide derivative of formula (I) as claimed in any one of claims 1 to 3, wherein $R^1$ is hydrogen, and $R^2$ and $R^3$ are $C_1$—$C_4$ alkoxy.

5. An N-arylbenzamide derivative of formula (I) as claimed in claim 4, wherein $R^2$ is a 2-methoxy and $R^3$ is a 6-methoxy substituent.

6. An N-arylbenzamide derivative of formula (I) as claimed in any one of claims 1 to 5, wherein $R^5$ is a sterically-hindered or bulky group.

7. An N-arylbenzamide derivative of formula (I) as claimed in claim 6, wherein $R^5$ is a group of formula:

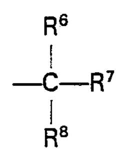

wherein $R^6$ is hydrogen or $C_1$—$C_4$ alkyl, $R^7$ is $C_1$—$C_4$ alkyl and $R^8$ is $C_1$—$C_{13}$ alkyl.

8. An N-arylbenzamide derivative of formula (I) as claimed in claim 7, wherein $R^5$ is 1-ethyl-1-methylpropyl.

9. An N-arylbenzamide derivative of formula (I) as claimed in claim 6, wherein $R^5$ is a group of formula:

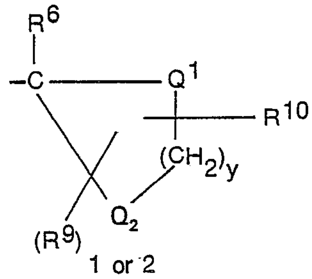

wherein $R^6$ is $C_1$—$C_4$ alkyl; $R^9$ and $R^{10}$ independently are hydrogen or $C_1$—$C_4$ alkyl; $Q^1$ and $Q^2$ are $CH_2$ and y is an integer from 0 to 4.

10. An N-arylbenzamide derivative of formula (I) as claimed in claim 9, wherein $R^5$ is 1-ethylcyclohexyl.

11. N-[3-(1-Ethyl-1-methylpropyl)-5-isoxazolyl]-2,6-dimethoxybenzamide.

12. N-[3-(1,1-Dimethylethyl)-5-isoxazolyl]-2,6-dimethoxybenzamide.

13. N-[3-(1-Ethylcyclohexyl)-5-isoxazolyl]-2,6-dimethoxybenzamide.

14. N-[6-(1,1-Dimethylethyl)pyridazin-3-yl]-2,6-dimethoxybenzamide.

15. N-[6-(1-Ethyl-1-methylpropyl)pyridazin-3-yl]-2,6-dimethoxybenzamide.

16. An N-arylbenzamide derivative of formula (I), or an agronomically-acceptable salt thereof, as claimed in any one of claims 1 to 15 for use as a herbicide.

17. A herbicidal formulation comprising as an active ingredient an N-arylbenzamide derivative of formula (I), or an agronomically-acceptable salt thereof, as claimed in any one of claims 1 to 15, associated with one or more agronomically-acceptable carriers or diluents therefor.

18. A herbicidal formulation as claimed in claim 17 which is a wettable powder.

19. A method for controlling the growth of undesired vegetation which comprises applying to the locus where vegetative control is desired a herbicidally effective amount of an N-arylbenzamide derivative of formula (I), or an agronomically-acceptable salt thereof, as claimed in any one of claims 1 to 15.

20. A process for preparing an N-arylbenzamide derivative of formula (I) as claimed in any one of claims 1 to 15 which comprises:

(a) acylating an amine of formula $H_2NR^4$ with a substituted benzoic acid derivative of formula:

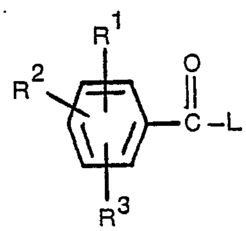

where L is a good leaving group, to form an N-arylbenzamide derivative of formula (I) in which Z is oxygen;

(b) optionally thiating the product of reaction (a) to produce an N-arylbenzamide of formula (I) in which Z is sulfur; and

(c) optionally salifying an N-arylbenzamide derivative of formula (I) produced by reaction (a) or (b) to provide an agronomically-acceptable salt.

**Claims for the Contracting State: AT**

1. Herbicidal formulation comprising as an active ingredient from 1 to 95% by weight of an N-arylbenzamide derivative of formula (I):

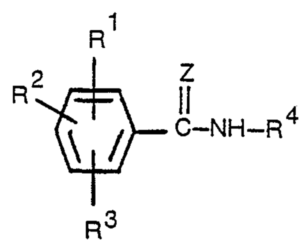

(I)

wherein

Z is oxygen or sulfur;

$R^1$ is hydrogen, halogen, $C_1$—$C_4$ alkyl, or $C_1$—$C_4$ alkoxy;

$R^2$ is hydrogen, halogen, $C_1$—$C_4$ alkyl, $C_1$—$C_4$ alkoxy, $C_1$—$c_4$ alkylthio or trifluoromethyl;

$R^3$ is hydrogen, halogen, $C_1$—$C_4$ alkyl, $C_1$—$C_4$ alkoxy or $C_1$—$C_4$ alkylthio; provided that when one of $R^1$, $R^2$ or $R^3$ is alkyl, one or both of the other phenyl substituents is other than hydrogen; and when $R^2$ is trifluoromethyl, one or both of $R^1$ and $R^3$ is other than hydrogen;

$R^4$ is an aryl group selected from

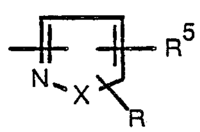, 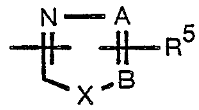, 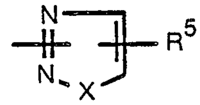,

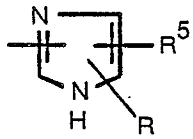 or 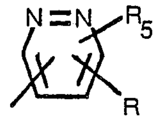

wherein:

A is CH or N and B is CH or N, provided that one of A and B is CH and the other is N;
X is NH, O or S;
R is hydrogen or $C_1$—$C_4$ alkyl;
$R^5$ is

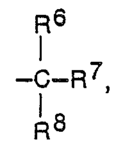 or 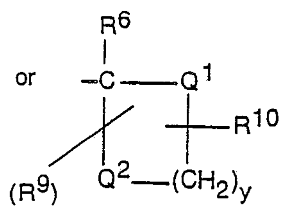

1 or 2

wherein:

y is an integer from 0 to 5;
$R^6$, $R^7$ and $R^8$ independently are hydrogen, $C_1$—$C_{13}$ alkyl, halo-C—$C_{13}$ alkyl, $C_2$—$C_{13}$ alkenyl, $C_2$—$C_{13}$ alkynyl, $C_1$—$C_4$ alkoxy-$C_1$—$C_6$ alkyl, $C_1$—$C_4$ alkylthio-$C_1$—$C_6$ alkyl, $C_1$—$C_6$ alkoxy, $C_2$—$C_4$ alkanoyloxy-$C_1$—$C_6$ alkyl, $C_1$—$C_6$ alkylthio, or

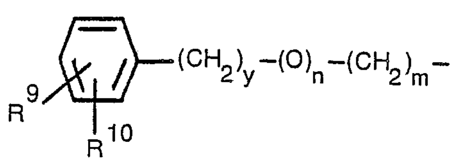, or 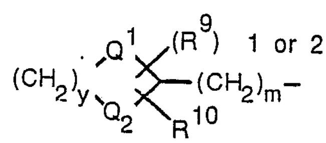

in which

m is an integer from 0 to 4;
n is zero or 1; and
$R^9$ and $R^{10}$ independently are hydrogen, halogen, $C_1$—$C_4$ alkyl or $C_2$—$C_4$ alkenyl;
$Q^1$ and $Q^2$ are independently $CH_2$, O, S, $CH_2O$ or $CH_2S$, provided that when $Q^1$ and $Q^2$ are both other than $CH_2$, y is not 0; and provided that $R^2$ and $R^3$ are other than hydrogen when $R^4$ is

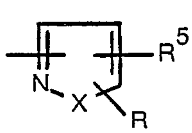 or 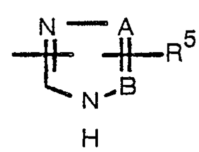

or an agronomically-acceptable salt thereof;
associated with one or more agronomically-acceptable carriers ore diluents therefor.

2. A herbidical formulation as claimed in claim 1, which contains as the active ingredient from 5 to 95% by weight the N-arylbenzamide of formula (I), wherein Z is oxygen, $R^4$ is

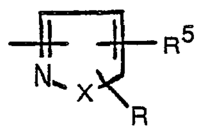

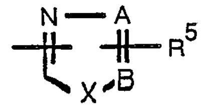

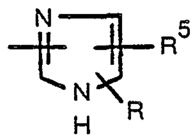

or

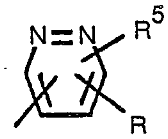

where R is hydrogen;

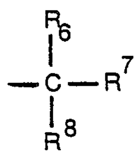

or

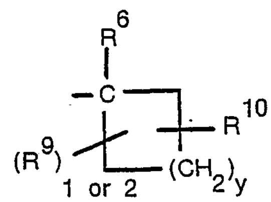

wherein:

$R^6$ and $R^7$ independently are hydrogen, $C_1$—$C_4$ alkyl, $C_2$—$C_4$ alkenyl or $C_2$—$C_4$ alkynyl;

y is an integer from 0 to 4;

$R^8$ is hydrogen, $C_1$—$C_{13}$ alkyl, halo-$C_1$—$C_{13}$ alkyl, $C_2$—$C_{13}$ alkenyl, $C_2$—$C_{13}$ alkynyl, $C_1$—$C_4$ alkoxy-$C_1$—$C_6$ alkyl, $C_1$—$C_4$ alkylthio-$C_1$—$C_6$ alkyl,

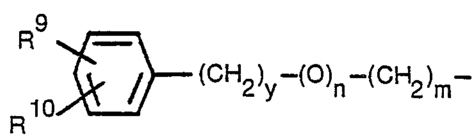

or

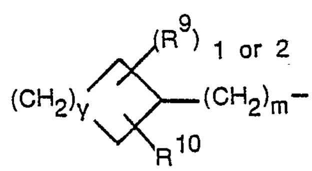

wherein:

m is an integer from 0 to 4;

n is zero or 1; and

$R^9$ and $R^{10}$ independently are hydrogen, halogen, $C_1$—$C_4$ alkyl or $C_2$—$C_4$ alkenyl.

3. A herbicidal formulation as claimed in claim 1 or 2, wherein the N-arylbenzamide derivative of formula (I) is one in which Z is oxygen and $R^4$ is

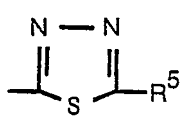

,

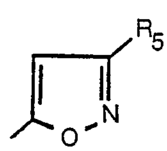

,

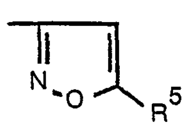

,

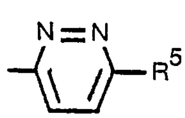

or

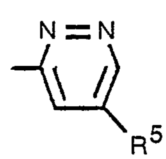

4. A herbicidal formulation as claimed in any one of claims 1 to 3, wherein the N-arylbenzamide derivative of formula (I) is one in which $R^1$ is hydrogen, and $R^2$ and $R^3$ are $C_1$—$C_4$ alkoxy.

5. A herbicidal formulation as claimed in claim 4, wherein the N-arylbenzamide derivative of formula (I) is one in which $R^2$ is a 2-methoxy and $R^3$ is a 6-methoxy substituent.

6. A herbicidal formulation as claimed in any one of claims 1 to 5, wherein the N-arylbenzamide derivative of formula (I) is one in which $R^5$ is a sterically-hindered or bulky group.

7. A herbicidal formulation as claimed in claim 6, wherein the N-arylbenzamide derivative of formula (I) is one in which $R^5$ is a group of formula:

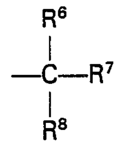

wherein $R^6$ is hydrogen or $C_1$—$C_4$ alkyl, $R^7$ is $C_1$—$C_4$ alkyl and $R^8$ is $C_1$—$C_{13}$ alkyl.

8. A herbicidal formulation as claimed in claim 7, wherein the N-arylbenzamide derivative of formula (I) is one in which $R^5$ is 1-ethyl-1-methylpropyl.

9. A herbicidal formulation as claimed in claim 6, wherein the N-arylbenzamide derivative of formula (I) is one in which $R^5$ is a group of formula:

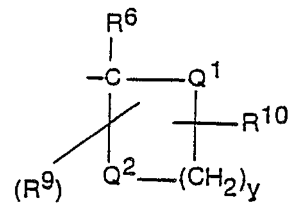

1 or 2

wherein $R^6$ is $C_1$—$C_4$ alkyl; R and $R^{10}$ independently are hydrogen or $C_1$—$C_4$ alkyl; $Q^1$ and $Q^2$ are $CH_2$ and y is an integer from 0 to 4.

10. A herbicidal formulation as claimed in claim 9, wherein the N-arylbenzamide derivative of formula (I) is one in which $R^5$ is 1-ethylcyclohexyl.

11. A herbicidal formulation as claimed in claim 1 or 2, wherein the N-arylbenzamide derivative of formula (I) is N - [3 - (1 - ethyl - 1 - methylpropyl) - 5 - isoxazolyl] - 2,6 - dimethoxybenzamide.

12. A herbicidal formulation as claimed in claim 1 or 2, wherein the N-arylbenzamide derivative of formula (I) is N - [3 - 1,1 - dimethylethyl) - 5 - isoxazolyl] - 2,6 - dimethoxybenzamide.

13. A herbicidal formulation as claimed in claim 1 or 2, wherein the N-arylbenzamide derivative of formula (I) is N - [3 - (1 - ethylcyclohexyl) - 5 - isoxazolyl] - 2,6-dimethoxybenzamide.

14. A herbicidal formulation as claimed in claim 1 or 2, wherein the N-arylbenzamide derivative of formula (I) is N - [6 - (1,1 - dimethylethyl)pyridazin - 3 - yl] - 2,6 - dimethoxybenzamide.

15. A herbicidal formulation as claimed in claim 1 or 2, wherein the N-arylbenzamide derivative of formula (I) is N - [6 - (1 - ethyl - 1 - methylpropyl)pyridazin - 3 - yl] - 2,6 - dimethoxybenzamide.

16. A herbicidal formulation as claimed in any one of claims 1 to 15 which is a wettable powder.

17. A method for controlling the growth of undesired vegetation which comprises applying to the locus where vegetative control is desired a herbicidally effective amount of an N-arylbenzamide derivative of formula (I), or an agronomically-acceptable salt thereof, as defined in any one of claims 1 to 15.

18. A process for preparing an N-arylbenzamide derivative of formula (I) as defined in any one of claims 1 to 15 which comprises:

(a) acylating an amine of formula $H_2NR_4$ with a substituted benzoic acid derivative of formula:

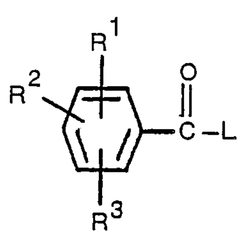

where L is a good leaving group, to form an N-arylbenzamide derivative of formula (I) in which Z is oxygen;

(b) optionally thiating the product of reaction (a) to produce an N-arylbenzamide of formula (I) in which Z is sulfur; and

(c) optionally salifying an N-arylbenzamide derivative of formula (I) produced by reaction (a) or (b) to provide an agronomically-acceptable salt.

19. An N-arylbenzamide derivative of formula (I), or an agronomically-acceptable salt thereof, whenever prepared by the process of claim 18.

**Patentansprüche für die Vertragsstaaten: BE CH DE FR GB IT LI LU NL SE**

1. N-Arylbenzamidderivat der Formel (I)

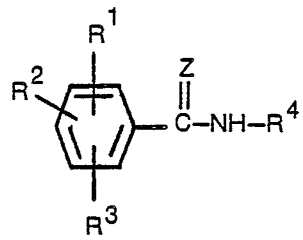

(I)

worin

Z Sauerstoff oder Schwefel ist,

$R^1$ Wasserstoff, Halogen, $C_1$—$C_4$-Alkyl oder $C_1$—$C_4$-Alkoxy ist,

$R^2$ Wasserstoff, Halogen, $C_1$—$C_4$-Alkyl, $C_1$—$C_4$-Alkoxy, $C_1$—$C_4$-Alkylthio oder Trifluormethyl ist,

$R^3$ Wasserstoff, Halogen, $C_1$—$C_4$-Alkyl, $C_1$—$C_4$-Alkoxy oder $C_1$—$C_4$-Alkylthio ist,

mit der Maßgabe, daß, falls einer der Substituenten $R^1$, $R^2$ oder $R^3$ Alkyl ist, einer oder beide der anderen Phenylsubstituenten etwas anderes als Wasserstoff sind, und falls $R^2$ Trifluormethyl ist, einer oder beide Substituenten $R^1$ und $R^3$ etwas anderes als Wasserstoff sind,

$R^4$ eine der folgenden Arylgruppen ist

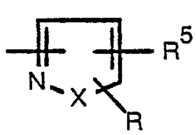,
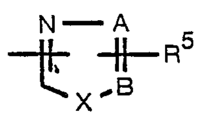,
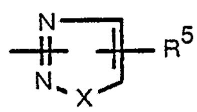

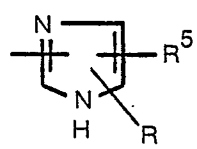 oder 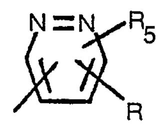

worin

A für CH oder N und

B für CH oder N steht, mit der Maßgabe, daß eine der Brücken A und B für CH steht und die andere für N steht,

X für NH, O oder S steht,

R Wasserstoff oder $C_1$—$C_4$-Alkyl ist,

$R^5$ für

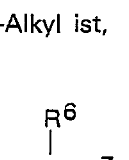, oder 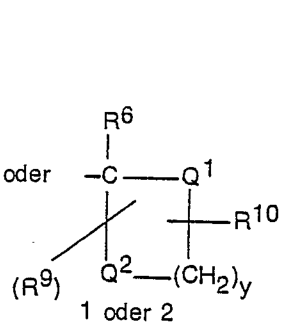

steht, worin

y eine Zahl von 0 bis 5 ist,

$R^6$, $R^7$ und $R^8$ unabhängig Wasserstoff, $C_1$—$C_{13}$-Alkyl, Halogen-$C_1$—$C_{13}$-alkyl, $C_2$—$C_{13}$-Alkenyl, $C_2$—$C_{13}$-Alkinyl, $C_1$—$C_4$-Alkoxy-$C_1$—$C_6$-alkyl, $C_1$—$C_4$-Alkylthio-$C_1$—$C_6$-alkyl, $C_1$—$C_6$-Alkoxy, $C_2$—$C_4$-Alkanoyloxy-$C_1$—$C_6$-alkyl, $C_1$—$C_6$-Alkylthio oder folgende Reste sind

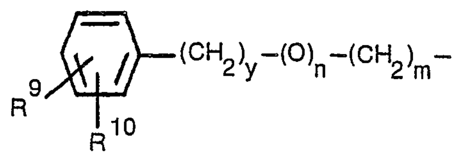 , oder 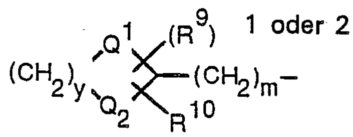

worin

m eine Zahl von 0 bis 4 ist,

n für 0 oder 1 steht und

$R^9$ und $R^{10}$ unabhängig Wasserstoff, Halogen, $C_1$—$C_4$ Alkyl- oder $C_2C_4$-Alkenyl sind,

$Q^1$ und $Q^2$ unabhängig $CH_2$, O, S, $CH_2O$ oder $CH_2S$ sind,

mit der Maßgabe, daß, falls $Q^1$ und $Q^2$ beide etwas anderes als $CH_2$ sind, y nicht 0 ist, und mit der Maßgabe, daß die Substituenten $R^2$ und $R^3$ etwas anderes als Wasserstoff sind, falls $R^4$ für folgende Reste steht

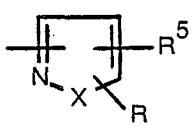 oder 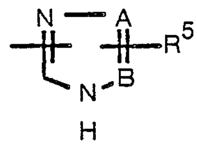

oder ein landwirtschaftlich annehmbares Salz hiervon.

2. N-Arylbenzamidderivat der Formel (I) gemäß Anspruch 1, dadurch gekennzeichnet, daß Z Sauerstoff ist, $R^4$ für folgende Reste steht

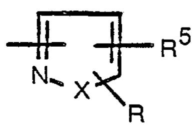 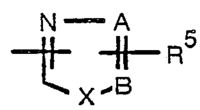

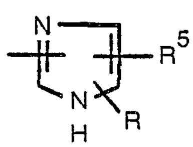 oder 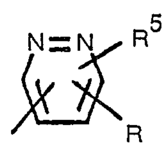

worin R Wasserstoff ist,

$R^5$ für

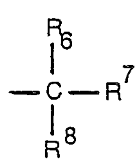 oder 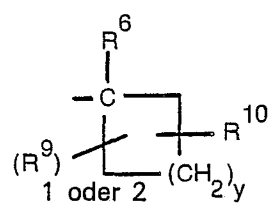

steht,

$R^6$ und $R^7$ unabhängig Wasserstoff, $C_1$—$C_4$-Alkyl, $C_2$—$C_4$-Alkenyl oder $C_2$—$C_4$-Alkinyl sind,

y eine Zahl von 0 bis 4 ist,

$R^8$ Wasserstoff, $C_1$—$C_{13}$-Alkyl, Halogen-$C_1$—$C_{13}$-alkyl, $C_2$—$C_{13}$-Alkenyl, $C_2$—$C_{13}$-Alkinyl, $C_1$—$C_4$-Alkoxy-$C_1$—$C_6$-alkyl, $C_1$—$C_4$-Alkylthio-$C_1$—$C_6$-alkyl oder folgende Reste bedeutet

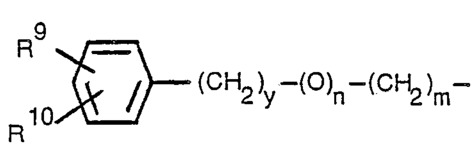 oder 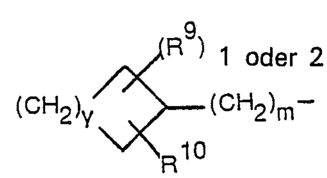

worin

m eine Zahl von 0 bis 4 ist,

n für 0 oder 1 steht und

$R^9$ und $R^{10}$ unabhängig Wasserstoff, Halogen, $C_1$—$C_4$-Alkyl oder $C_2$—$C_4$-Alkenyl sind.

3. N-Arylbenzamidderivat der Formel (I) gemäß Anspruch 1 oder 2, dadurch gekennzeichnet, daß Z Sauerstoff ist und R für folgende Reste steht

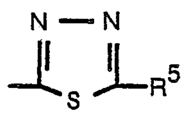 , 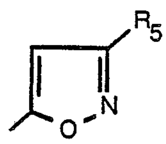 ,

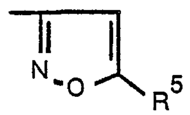 , 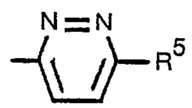 oder 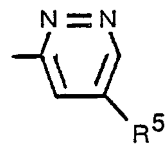

4. N-Arylbenzamidderivat der Formel (I) gemäß einem der Ansprüche 1 bis 3, dadurch gekennzeichnet, daß $R^1$ Wasserstoff ist und $R^2$ und $R^3$ für $C_1$—$C_4$-Alkoxy stehen.

5. N-Arylbenzamidderivat der Formel (I) gemäß Anspruch 4, dadurch gekennzeichnet, daß $R^2$ ein 2-Methoxy-substituent ist und $R^3$ ein 6-Methoxysubstituent ist.

6. N-Arylbenzamidderivat der Formel (I) gemäß einem der Ansprüche 1 bis 5, dadurch gekennzeichnet, daß $R^5$ eine sterisch gehinderte oder voluminöse Gruppe ist.

7. N-Arylbenzamidderivat der Formel (I) gemäß Anspruch 6, dadurch gekennzeichnet, daß $R^5$ eine Gruppe der Formel

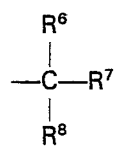

ist, worin $R^6$ Wasserstoff oder $C_1$—$C_4$-Alkyl ist, $R^7$ für $C_1$—$C_4$-Alkyl steht und $R^8$ für $C_1$—$C_{13}$-Alkyl steht.

8. N-Arylbenzamidderivat der Formel (I) gemäß Anspruch 7, dadurch gekennzeichnet, daß $R^5$ für 1-Ethyl-1-methylpropyl steht.

9. N-Arylbenzamidderivat der Formel (I) gemäß Anspruch 6, dadurch gekennzeichnet, daß $R^5$ eine Gruppe der Formel

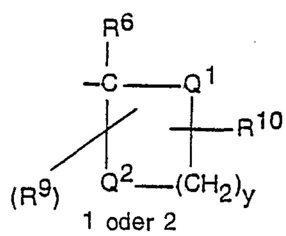

ist, worin $R^6$ für $C_1$—$C_4$-Alkyl steht, $R^9$ und $R^{10}$ unabhängig Wasserstoff oder $C_1$—$C_4$-Alkyl sind, $Q^1$ und $Q^2$ für $CH_2$ stehen und y eine Zahl von 0 bis 4 ist.

10. N-Arylbenzamidderivat der Formel (I) gemäß Anspruch 9, dadurch gekennzeichnet, daß $R^5$ für 1-Ethyl-cyclohexyl steht.

11. N-[3-(1-Ethyl-1-methylpropyl)-5-isoxazolyl]-2,6-dimethoxybenzamid.

12. N-[3-(1,1-Dimethylethyl)-5-isoxazolyl]-2,6-dimethoxybenzamid.

13. N-[3-(1-Ethylcyclohexyl)-5-isoxazolyl]-2,6-dimethoxybenzamid.

14. N-[6-(1,1-Dimethylethyl)pyridazin-3-yl]-2,6-dimethoxybenzamid.

15. N-[6-(1-Ethyl-1-methylpropyl)pyradazin-3-yl]-2,6-dimethoxybenzamid.

16. Verwendung eines N-Arylbenzamidderivats der Formel (I) oder eines landwirtschaftlich annehmbaren Salzes hiervon gemäß einem der Ansprüche 1 bis 15 als Herbizid.

17. Herbizide Formulierung, dadurch gekennzeichnet, daß sie als Wirkstoff ein N-Arylbenzamidderivat der Formel (I) oder ein landwirtschaftlich annehmbares Salz hiervon gemäß einem der Ansprüche 1 bis 15 in Verbindung mit einem oder mehreren landwirtschaftlich annehmbaren Trägern oder Verdünnungsmitteln hierfür enthält.

18. Herbizide Formulierung gemäß Anspruch 17, dadurch gekennzeichnet, daß sie ein benetzbares Pulver ist.

19. Verfahren zur Bekämpfung des Wachstums einer unerwünschten Vegetation, dadurch gekennzeichnet, daß man den Ort, an dem man eine Vegetation bekämpfen möchte, mit einer herbizid wirksamen Menge eines N-Arylbenzamid-derivats der Formel (I) oder eines landwirtschaftlich annehmbaren Salzes hiervon gemäß einem der Ansprüche 1 bis 15 behandelt.

20. Verfahren zur Herstellung eines N-Arylbenzamidderivats der Formel gemäß einem der Ansprüche 1 bis 15, dadurch gekennzeichnet, daß man

(a) ein Amin der Formel $H_2NR^4$ durch Acylierung mit einem substituierten Benzoesäurederivat der Formel

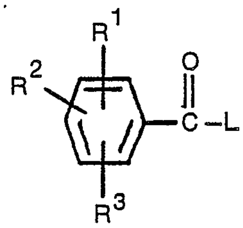

worin L eine gute Abgangsgruppe ist, in ein N-Arylbenzamidderivat der Formel (I) überführt, worin Z Sauerstoff ist,

(b) das Reaktionsprodukt (a) gegebenenfalls durch Thiierung in ein N-Arylbenzamid der Formel (I) überführt, worin Z Schwefel ist, und

(c) das durch die Umsetzung (a) oder (b) gebildete N-Arylbenzamidderivat der Formel (I) gegebenenfalls durch Ansäuerung in ein landwirtschaftlich annehmbares Salz überführt.

**Patentansprüche für den Vertragsstaat: AT**

1. Herbizide Formulierung, dadurch gekennzeichnet, daß sie als Wirkstoff 1 bis 95 Gewichtsprozent eines N-Arylbenzamidderivats der Formel (I)

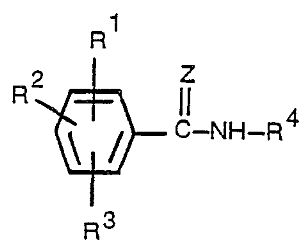

(I)

worin

Z Sauerstoff oder Schwefel ist,

$R^1$ Wasserstoff, Halogen, $C_1$—$C_4$-Alkyl oder $C_1$—$C_4$-Alkoxy ist,

$R^2$ Wasserstoff, Halogen, $C_1$—$C_4$-Alkyl, $C_1$—$C_4$-Alkoxy, $C_1$—$C_4$-Alkylthio oder Trifluormethyl ist,

$R^3$ Wasserstoff, Halogen, $C_1$—$C_4$-Alkyl, $C_1$—$C_4$-Alkoxy oder $C_1$—$C_4$-Alkylthio ist,

mit der Maßgabe, daß, falls einer der Substituenten $R^1$, $R^2$ oder $R^3$ Alkyl ist, einer oder beide der anderen Phenylsubstituenten etwas anderes als Wasserstoff sind, und falls $R^2$ Trifluormethyl ist, einer oder beide Substituenten $R^1$ und $R^3$ etwas anderes als Wasserstoff sind,

$R^4$ eine der folgenden Arylgruppen ist

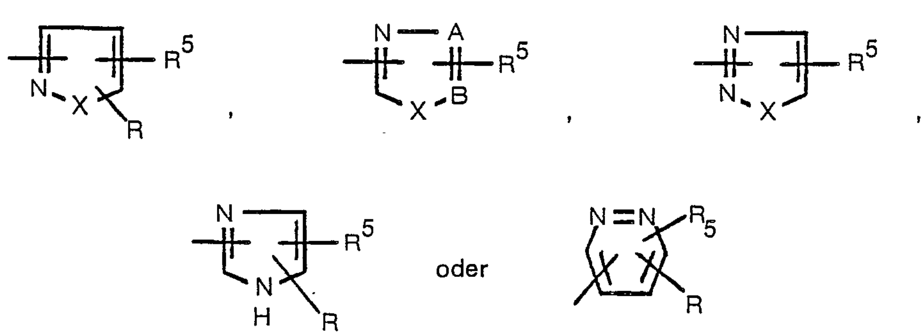

worin

A für CH oder N und

B für CH oder N steht, mit der Maßgabe, daß eine der Brücken A und B für CH steht und die andere für N steht,

X für NH, O oder S steht,

R Wasserstoff oder $C_1$—$C_4$-Alkyl ist,

$R^5$ für

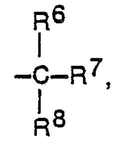

oder

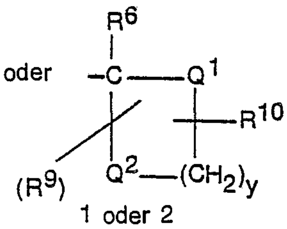

1 oder 2

steht, worin

y eine Zahl von 0 bis 5 ist,

$R^6$, $R^7$ und $R^8$ unabhängig Wasserstoff, $C_1$—$C_{13}$-Alkyl, Halogen-$C_1$—$C_{13}$-alkyl, $C_2$—$C_{13}$-Alkenyl, $C_2$—$C_{13}$-Alkinyl, $C_1$—$C_4$-Alkoxy-$C_1$—$C_6$-alkyl, $C_1$—$C_4$-Alkylthio-$C_1$—$C_6$-alkyl, $C_1$—$C_6$-Alkoxy, $C_2$—$C_4$-Alkanoyloxy-$C_1$—$C_6$-alkyl, $C_1$—$C_6$-Alkylthio oder folgende Reste sind

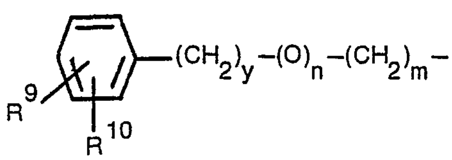

, oder

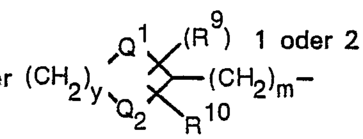

worin

m eine Zahl von 0 bis 4 ist,

n für 0 oder 1 steht und

$R^9$ und $R^{10}$ unabhängig Wasserstoff, Halogen, $C_1$—$C_4$ Alkyl- oder $C_2$—$C_4$-Alkenyl sind,

$Q^1$ und $Q^2$ unabhängig $CH_2$, O, S, $CH_2O$ oder $CH_2S$ sind,

mit der Maßgabe, daß, falls $Q^1$ und $Q^2$ beide etwas anderes als $CH_2$ sind, y nicht 0 ist, und mit der Maßgabe, daß die Substituenten $R^2$ und $R^3$ etwas anderes als Wasserstoff sind, falls $R^4$ für folgende Reste steht

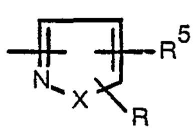

oder

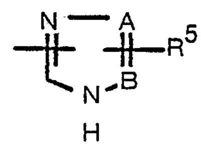

oder ein landwirtschaftlich annehmbares Salz hiervon in Verbindung mit einem oder mehreren landwirtschaftlich annehmbaren Trägern oder Verdünnungsmitteln hierfür enthält.

2. Herbizide Formulierung gemäß Anspruch 1, dadurch gekennzeichnet, daß sie als Wirkstoff 5 bis 95 Gewichtsprozent eines N-Arylbenzamidderivats der Formel (I) enthält, worin Z Sauerstoff ist, $R^4$ für folgende Reste steht

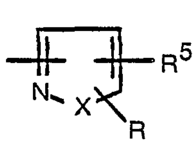

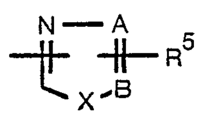

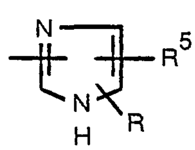

oder

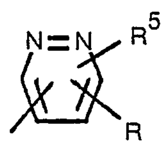

worin R Wasserstoff ist,

$R^5$ für

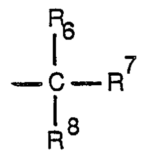

oder

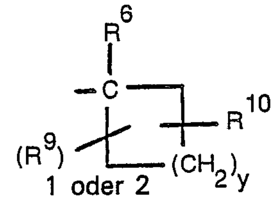

steht, worin

$R^6$ und $R^7$ unabhängig voneinander Wasserstoff, $C_1$—$C_4$-Alkyl, $C_2$—$C_4$-Alkenyl oder $C_2$—$C_4$-Alkinyl bedeuten,

y eine Zahl von 0 bis 4 ist,

$R^8$ Wasserstoff, $C_1$—$C_{13}$-Alkyl, Halogen-$C_1$—$C_{13}$-alkyl, $C_2$—$C_{13}$-Alkenyl, $C_2$—$C_{13}$-Alkinyl, $C_1$—$C_4$-Alkoxy-$C_1$—$C_6$-alkyl, $C_1$—$C_4$-Alkylthio-$C_1$—$C_6$-alkyl oder folgende Reste bedeutet

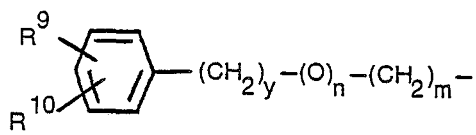

oder

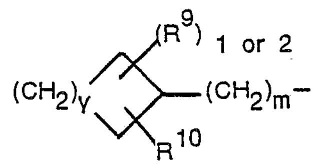

worin

m eine Zahl von 0 bis 4 ist,

n für 0 oder 1 steht und

$R^9$ und $R^{10}$ unabhängig Wasserstoff, Halogen, $C_1$—$C_4$-Alkyl oder $C_2$—$C_4$-Alkenyl sind.

3. Herbizide Formulierung gemäß Anspruch 1 oder 2, dadurch gekennzeichnet, daß sie ein N-Arylbenzamidderivat der Formel (I) enthält, worin Z Sauerstoff ist und $R^4$ für folgende Reste steht

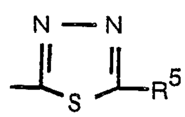

,

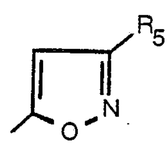

,

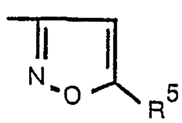

,

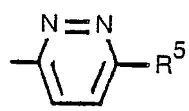

oder

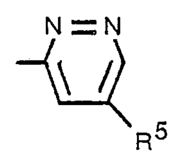

4. Herbizide Formulierung gemäß einem der Ansprüche 1 bis 3, dadurch gekennzeichnet, daß sie ein N-Arylbenzamidderivat der Formel (I) enthält, worin $R^1$ Wasserstoff ist und $R^2$ und $R^3$ für $C_1$—$C_4$-Alkoxy stehen.

5. Herbizide Formulierung gemäß Anspruch 4, dadurch gekennzeichnet, daß sie ein N-Arylbenzamidderivat der Formel (I) enthält, worin $R^2$ für 2-Methoxy steht und $R^3$ für 6-Methoxy steht.

6. Herbizide Formulierung gemäß einem der Ansprüche 1 bis 5, dadurch gekennzeichnet, daß sie ein N-Arylbenzamidderivat der Formel (I) enthält, worin $R^5$ eine sterisch gehinderte oder voluminöse Gruppe ist.

7. Herbizide Formulierung gemäß Anspruch 6, dadurch gekennzeichnet, daß sie ein N-Arylbenzamidderivat der Formel (I) enthält, worin $R^5$ eine Gruppe der Formel

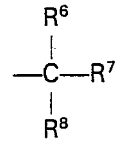

ist, worin $R^6$ Wasserstoff oder $C_1$—$C_4$-Alkyl ist, $R^7$ für $C_1$—$C_4$-Alkyl steht und $R^8$ für $C_1$—$C_{13}$-Alkyl steht.

8. Herbizide Formulierung gemäß Anspruch 7, dadurch gekennzeichnet, daß sie ein N-Arylbenzamidderivat der Formel (I) enthält, worin $R^5$ für 1-Ethyl-1-methylpropyl steht.

9. Herbizide Formulierung gemäß Anspruch 6, dadurch gekennzeichnet, daß sie ein N-Arylbenzamidderivat der Formel (I) enthält, worin $R^5$ eine Gruppe der Formel

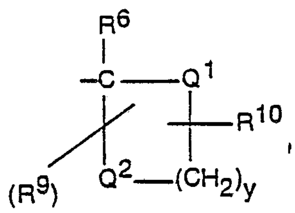

1 oder 2

ist, worin $R^6$ für $C_1$—$C_4$-Alkyl steht, $R^9$ und $R^{10}$ unabhängig Wasserstoff oder $C_1$—$C_4$-Alkyl sind, $Q^1$ und $Q^2$ für $CH_2$ stehen und Y eine Zahl von 0 bis 4 ist.

10. Herbizide Formulierung gemäß Anspruch 9, dadurch gekennzeichnet, daß sie ein N-Arylbenzamidderivat der Formel (I) enthält, worin $R^5$ für 1-Ethylcyclohexyl steht.

11. Herbizide Formulierung gemäß Anspruch 1 oder 2, dadurch gekennzeichnet, daß sie als N-Arylbenzamidderivat der Formel (I) N - [3 - (1 - Ethyl - 1 - methylpropyl) - 5 - isoxazolyl] - 2,6 - dimethoxybenzamid enthält.

12. Herbizide Formulierung gemäß Anspruch 1 oder 2, dadurch gekennzeichnet, daß sie als N-Arylbenzamidderivat der Formel (I) N - [3 - (1,1 - Dimethylethyl) - 5 - isoxazolyl] - 2,6 - dimethoxybenzamid enthält.

13. Herbizide Formulierung gemäß Anspruch 1 oder 2, dadurch gekennzeichnet, daß sie als N-Arylbenzamidderivat der Formel (I) N - [3 - (1 - Ethylcyclohexyl) - 5 - isoxazylol] - 2,6 - dimethoxybenzamid enthält.

14. Herbizide Formulierung gemäß Anspruch 1 oder 2, dadurch gekennzeichnet, daß sie als N-Arylbenzamidderivat der Formel (I) N - [6 - (1,1 - Dimethylethyl)pyridazin - 3 - yl] - 2,6 - dimethoxybenzamid enthält.

15. Herbizide Formulierung gemäß Anspruch 1 oder 2, dadurch gekennzeichnet, daß sie als N-Arylbenzamidderivat der Formel (I) N - [6 - (1 - Ethyl - 1 - methylpropyl) - pyridazin - 3 - yl] - 2,6 - dimethoxybenzamid enthält.

16. Herbizide Formulierung gemäß einer der Ansprüche 1 bis 15, dadurch gekennzeichnet, daß sie ein benetzbares Pulver ist.

17. Verfahren zur Bekämpfung des Wachstums einer unerwünschten Vegetation, dadurch gekennzeichnet, daß man den Ort, an dem man eine Vegetation bekämpfen möchte, mit einer herbizid wirksamen Menge eines N-Arylbenzamidderivats der Formel (I) oder eines landwirtschaftlich annehmbaren Salzes hiervon gemäß einem der Ansprüche 1 bis 15 behandelt.

18. Verfahren zur Herstellung eines N-Arylbenzamidderivats der Formel (I) gemaß einem der Ansprüche 1 bis 15, dadurch gekennzeichnet, daß man

(a) ein Amin der Formel $H_2NR^4$ durch Acylierung mit einem substituierten Benzoesäurederivat der Formel

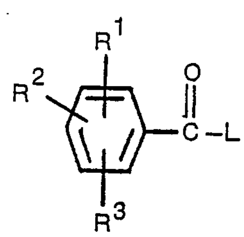

worin L eine gute Abgangsgruppe ist, in ein N-Arylbenzamidderivat der Formel (I) überführt, worin Z Sauerstoff ist,

(b) das Reaktionsprodukt (a) gegebenenfalls durch Thiierung in ein N-Arylbenzamid der Formel (I) überführt, worin Z Schwefel ist, und

(c) das durch die Umsetzung (a) oder (b) gebildete N-Arylbenzamidderivat der Formel (I) gegebenenfalls durch Ansäuerung in ein landwirtschaftlich annehmbares Salz überführt.

19. N-Arylbenzamidderivat der Formel I oder ein landwirtschaftlich annehmbares Salz hiervon, hergestellt nach dem Verfahren von Anspruch 19.

**Revendications pour les Etats contractants: BE CH DE FR GB IT LI LU NL SE**

1. Dérivé de N-arylbenzamide de formule (I):

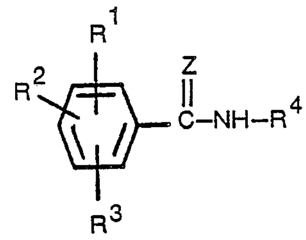

(I)

dans laquelle:

Z représente un atome d'oxygène ou un atome de soufre;

$R^1$ représente un atome d'hydrogène, un atome d'halogène, un groupe alkyle en $C_1$—$C_4$ ou un groupe alcoxy en $C_1$—$C_4$;

$R^2$ représente un atome d'hydrogène, un atome d'halogène, un groupe alkyle en $C_1$—$C_4$, un groupe alcoxy en $C_1$—$C_4$, un groupe alkyl (en $C_1$—$C_4$) thio ou un groupe trifluorométhyle;

$R^3$ représente un atome d'hydrogène, un atome d'halogène, un groupe alkyle en $C_1$—$C_4$, un groupe alcoxy en $C_1$—$C_4$ ou un groupe alkyl (en $C_1$—$C_4$) thio à condition que, si un des radicaux, $R^1$, $R^2$ et $R^3$ est un groupe alkyle, un ou les deux autres substituants du noyau phényle soit (soient) différent(s) de l'hydrogène et que, si $R^2$ est un groupe trifluorométhyle, un ou les deux radicaux $R^1$ et $R^3$ soit (soient) différent(s) de l'hydrogène;

$R^4$ est un groupe aryle choisi parmi:

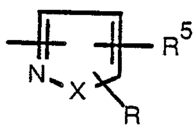 , 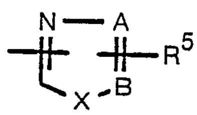 , 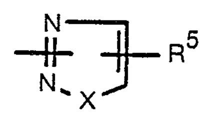 ,

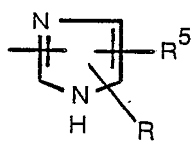 ou 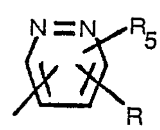

où:

A représente CH ou N et B représente CH ou N, à condition qu'un des radicaux A et B soit CH et que l'autre soit N;

X représente NH, O ou S;

R représente un atome d'hydrogène ou un groupe alkyle en $C_1$—$C_4$;

$R^5$ représente

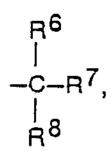 ou 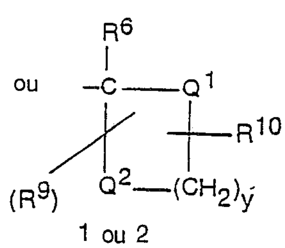

où:

y est un nombre entier de 0 à 5;

$R^6$, $R^7$ et $R^8$ représentent chacun indépendamment l'un de l'autre un atome d'hydrogène, un groupe alkyle en $C_1$—$C_{13}$, un groupe haloalkyle en $C_1$—$C_{13}$, un groupe alcényle en $C_2$—$C_{13}$, un groupe alcinyle en $C_2$—$C_{13}$, un groupe alcoxy (en $C_1$—$C_4$)alkyle en $C_1$—$C_6$, un groupe alkyl(en $C_1$—$C_4$)thioalkyle en $C_1$—$C_6$, un groupe alcoxy en $C_1$—$C_6$, un groupe alcanoyl(en $C_2$—$C_4$)oxyalkyle en $C_1$—$C_6$, un groupe alkyl(en $C_1$—$c_6$)thio ou un groupe

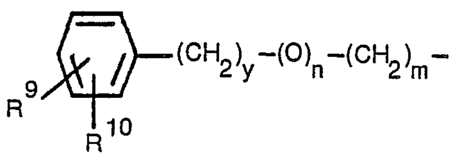 , ou 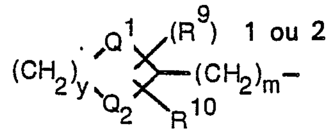

où m est un nombre entier de 0 à 4; n est égal à 0 ou 1; et $R^9$ et $R^{10}$ représentent indépendamment l'un de l'autre un atome d'hydrogène, un atome d'halogène, un groupe alkyle en $C_1$—$C_4$ ou un groupe alcényle en $C_2$—$C_4$;

$Q^1$ et $Q^2$ représentent chacun indépendamment l'un de l'autre $CH_2$, O, S, $CH_2O$ ou $CH_2S$ à condition que, si $Q^1$ et $Q^2$ représentent tous deux un groupe autre que $CH_2$, y ne soit pas égal à 0; et également que $R^2$ et $R^3$ soient différents de l'hydrogène lorsque $R^4$ est un groupe

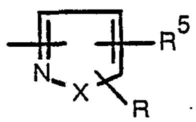 ou 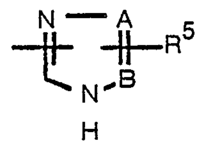

ou un sel de ce dérivé acceptable en agronomie.

2. Dérivé de N-arylbenzamide de formule (I) suivant la revendication 1, caractérisé en ce que Z représente un atome d'oxygène, $R^4$ représente un groupe

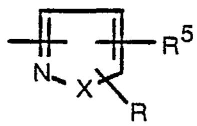 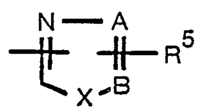

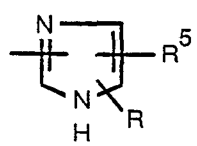 ou 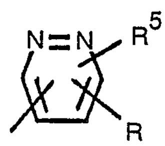

où R représente un atome d'hydrogène; $R^5$ représente un groupe

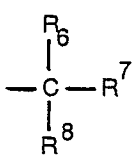 ou 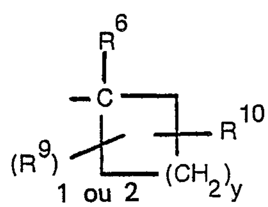

où:

$R^6$ et $R^7$ représentent chacun indépendamment l'un de l'autre un atome d'hydrogène, un groupe alkyle en $C_1$—$C_4$, un groupe alcényle en $C_2$—$C_4$ ou un groupe alcinyle en $C_2$—$C_4$;

y est un nombre entier de 0 à 4;

$R^8$ représente un atome d'hydrogène, un groupe alkyle en $C_1$—$C_{13}$, un groupe haloalkyle en $C_1$—$C_{13}$, un groupe alcényle en $C_2$—$C_{13}$, un groupe alcinyle en $C_2$—$C_{13}$, un groupe alcoxy(en $C_1$—$C_4$)alkyle en $C_1$—$C_6$, un groupe alkyl(en $C_1$—$C_4$)thioalkyle en $C_1$—$C_6$, un groupe

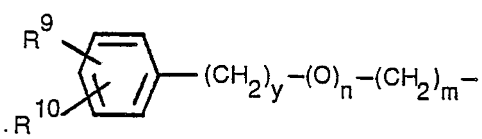 ou 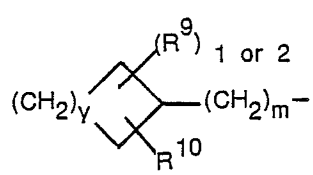

où:

m est un nombre entier de 0 à 4;

n est égal à 0 ou 1; et

$R^9$ et $R^{10}$ représentent chacun indépendamment l'un de l'autre un atome d'hydrogène, un atome d'halogène, un groupe alkyle en $C_1$—$C_4$ ou un groupe alcényle en $C_2$—$C_4$.

3. Dérivé de N-arylbenzamide de formule (I) suivant la revendication 1 ou 2, caractérisé en ce que Z est un atome d'oxygène et $R^4$ représente un groupe

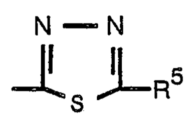 , 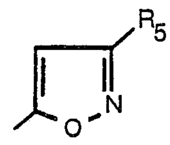 ,

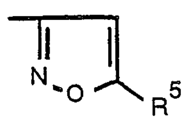 , 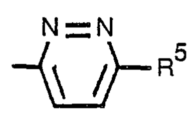 ou 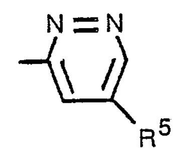

4. Dérivé de N-arylbenzamide de formule (I) suivant l'une quelconque des revendications 1 à 3, caractérisé en ce que $R^1$ est un atome d'hydrogène, tandis que $R^2$ et $R^3$ représentent chacun un groupe alcoxy en $C_1$—$C_4$.

5. Dérivé de N-arylbenzamide de formule (I) suivant la revendication 4, caractérisé en ce que $R^2$ est un substituant 2-méthoxy, tandis que $R^3$ est un substituant 6-méthoxy.

6. Dérivé de N-arylbenzamide de formule (I) suivant l'une quelconque des revendication 1 à 5, caractérisé en ce que $R^5$ est un groupe volumineux ou à empêchement stérique.

7. Dérivé de N-arylbenzamide de formule (I) suivant la revendication 6, caractérisé en ce que $R^5$ est un groupe de formule:

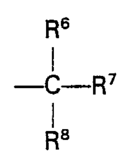

dans laquelle $R^6$ représente un atome d'hydrogène ou un groupe alkyle en $C_1$—$C_4$, $R^7$ représente un groupe alkyle en $C_1$—$C_4$ et $R^8$ représente un groupe alkyle en $C_1$—$C_{13}$.

8. Dérivé de N-arylbenzamide de formule (I) suivant la revendication 7, caractérisé en ce que $R^5$ est un groupe 1-éthyl-1-méthylpropyle.

9. Dérivé de N-arylbenzamide de formule (I) suivant la revendication 6, caractérisé en ce que $R^5$ est un groupe de formule:

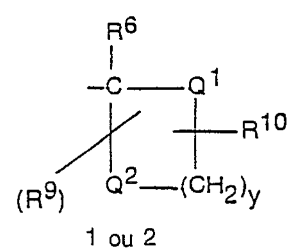

dans laquelle $R^6$ représente un groupe alkyle en $C_1$—$C_4$;

$R^9$ et $R^{10}$ représentent indépendamment l'un de l'autre un atome d'hydrogène ou un groupe alkyle en $C_1$—$C_4$;

$Q^1$ et $Q^2$ représentent chacun $CH_2$ et y est un nombre entier de 0 à 4.

10. Dérivé de N-arylbenzamide de formule (I) suivant la revendication 9, caractérisé en ce que $R^5$ est un groupe 1-éthylcyclohexyle.

11. Le N-[3-(1-éthyl-1-méthylpropyl)-5-isoxazolyl]-2,6-diméthoxybenzamide.

12. Le N-[3-(1,1-diméthyléthyl)-5-isoxazolyl]-2,6-diméthoxybenzamide.

13. Le N-[3-(1-éthylcyclohexyl)-5-isoxazolyl]-2,6-diméthoxybenzamide.

14. Le N-[6-(1,1-diméthyléthyl)pyridazin-3-yl]-2,6-diméthoxybenzamide..

15. Le N-[6-(1-éthyl-1-méthylpropyl)-pyridazin-3-yl]-2,6-diméthoxybenzamide.

16. Dérivé de N-arylbenzamide de formule (I) ou un de ses sels acceptables en agronomie suivant l'une quelconque des revendications 1 à 15 en vue de l'utiliser comme herbicide.

17. Formulation herbicide comprenant, comme ingrédient actif, un dérivé de N-arylbenzamide de formule (I) ou un de ses sels acceptables en agronomie suivant l'une quelconque des revendications 1 à 15, en association avec un ou plusieurs diluants ou supports acceptables en agronomie pour ce dérivé ou son sel.

18. Formulation herbicide suivant la revendication 17, caractérisée en ce qu'elle est une poudre mouillable.

19. Procédé en vue de combattre la croissance des végétations non désirées, caractérisée en ce qu'il consiste à appliquer, au locus où l'on désire combattre ces végétations, un dérivé de N-arylbenzamide de formule (I) ou un de ses sels acceptables en agronomie suivant l'une quelconque des revendications 1 à 15 en une quantité efficace du point de vue herbicide.

20. Procédé de préparation d'un dérivé de N-arylbenzamide de formule (I) suivant l'une quelconque des revendications 1 à 15, caractérisé en ce qu'il comprend les étapes qui consistent à:

(a) acyler une amine de formule $H_2NR^4$ avec un dérivé d'acide benzoïque substitué de formule:

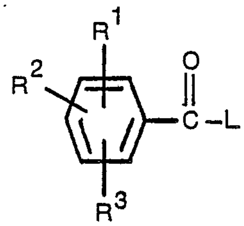

dans laquelle L est un bon groupe qui s'éloigne, pour former un dérivé de N-arylbenzamide de formule (I) dans laquelle Z est un atome d'oxygène;

(b) éventuellement sulfurer le produit de la réaction (a) pour obtenir un N-arylbenzamide de formule (I) dans laquelle Z est un atome de soufre; et

(c) éventuellement salifier un dérivé de N-arylbenzamide de formule (I) obtenu par la réaction (a) ou (b) pour obtenir un sel acceptable en agronomie.

**Revendications pour l'Etat contractant: AT**

1. Formulation herbicide comprenant, comme ingrédient actif, 1 à 95% en poids d'un dérivé de N-arylbenzamide de formule (I):

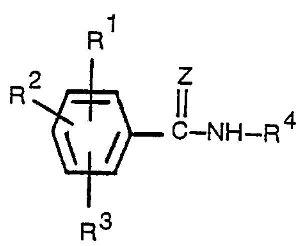

(I)

dans laquelle:

Z représente un atome d'oxygène ou un atome de soufre;

$R^1$ représente un atome d'hydrogène, un atome d'halogène, un groupe alkyle en $C_1$—$C_4$ ou un groupe alcoxy en $C_1$—$C_4$;

$R^2$ représente un atome d'hydrogène, un atome d'halogène, un groupe alkyle en $C_1$—$C_4$, un groupe alcoxy en $C_1$—$C_4$, un groupe alkyl (en $C_1$—$C_4$) thio ou un groupe trifluorométhyle;

$R^3$ représente un atome d'hydrogène, un atome d'halogène, un groupe alkyle en $C_1$—$C_4$, un groupe alcoxy en $C_1$—$C_4$ ou un groupe alkyl (en $C_1$—$C_4$) thio à condition que, si un des radicaux, $R^1$, $R^2$ et $R^3$ est un groupe alkyle, un ou les deux autres substituants du noyau phényle soit (soient) différent(s) de l'hydrogène et que, si $R^2$ est un groupe trifluorométhyle, un ou les deux radicaux $R^1$ et $R^3$ soit (soient) différent(s) de l'hydrogène;

$R^4$ représente un groupe aryle choisi parmi:

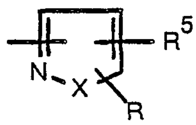 , 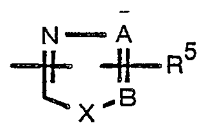 , 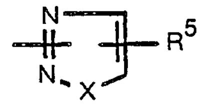 ,

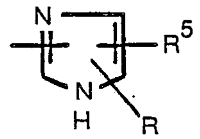 ou 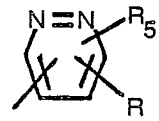

où:

A représente CH ou N et B représente CH ou N, à condition qu'un des radicaux A et B soit CH et que l'autre soit N;

X représente NH, O ou S;

R représente un atome d'hydrogène ou un groupe alkyle en $C_1$—$C_4$;

$R^5$ représente

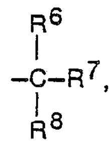 ou 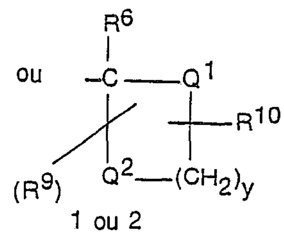

où:

y est un nombre entier de 0 à 5;

$R^6$, $R^7$ et $R^8$ représentent chacun indépendamment l'un de l'autre un atome d'hydrogène, un groupe alkyle en $C_1$—$C_{13}$, un groupe haloalkyle en $C_1$—$C_{13}$, un groupe alcényle en $C_2$—$C_{13}$, un groupe alcynyle en $C_2$—$C_{13}$, un groupe alcoxy (en $C_1$—$C_4$)alkyle en $C_1$—$C_6$, un groupe alkyl(en $C_1$—$C_4$)thioalkyle en $C_1$—$C_6$, un groupe alcoxy en $C_1$—$C_6$, un groupe alcanoyl(en $C_2$—$C_4$)oxyalkyle en $C_1$—$C_6$, un groupe alkyl(en $C_1$—$c_6$)thio ou un groupe

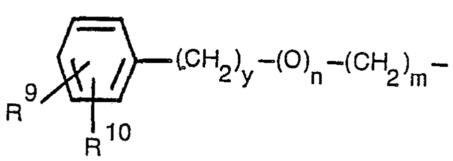 , ou 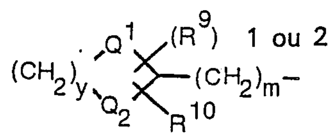

où m est un nombre entier de 0 à 4; n est égal à zéro ou 1; et $R^9$ et $R^{10}$ représentent indépendamment l'un de l'autre un atome d'hydrogène, un atome d'halogène, un groupe alkyle en $C_1$—$C_4$ ou un groupe alcényle en $C_2$—$C_4$;

$Q^1$ et $Q^2$ représentent chacun indépendamment l'un de l'autre $CH_2$, O, S, $CH_2O$ ou $CH_2S$ à condition que, si $Q^1$ et $Q^2$ représentent tous deux un groupe autre que $CH_2$, y ne soit pas égal à 0; et également que $R^2$ et $R^3$ soient différents de l'hydrogène lorsque $R^4$ est un groupe

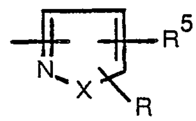 ou 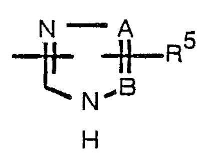

ou un sel de ce dérivé acceptable en agronomie; en association avec un ou plusieurs diluants ou supports acceptables en agronomie pour ce composé ou ce sel.

2. Formulation herbicide suivant la revendication 1, caractérisée en ce que, comme ingrédient actif, elle contient 5 à 95% en poids d'un N-arylbenzamide de formule (I) dans laquelle Z représente un atome d'oxygène, $R^4$ représente un groupe

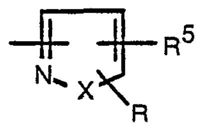

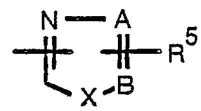

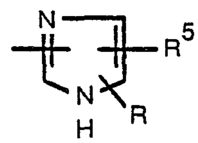

ou

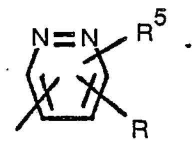

où R représente un atome d'hydrogène;
$R^5$ représente

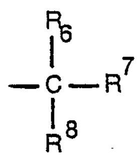

ou

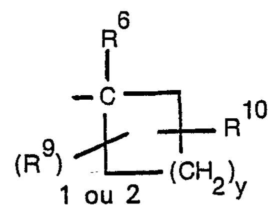

où;

$R^6$ et $R^7$ représentent chacun indépendamment l'un de l'autre un atome d'hydrogène, un groupe alkyle en $C_1$—$C_4$, un groupe alcényle en $C_2$—$C_4$ ou un groupe alcynyle en $C_2$—$C_4$;

y est un nombre entier de 0 à 4;

$R^8$ représente un atome d'hydrogène, un groupe alkyle en $C_1$—$C_{13}$, un groupe halo-alkyle en $C_1$—$C_{13}$, un groupe alcényle en $C_2$—$C_{13}$, un groupe alcynyle en $C_2$—$C_{13}$, un groupe alcoxy(en $C_1$—$C_4$)alkyle en $C_1$—$C_6$, un groupe alkyl(en $C_1$—$C_4$)thioalkyle en $C_1$—$C_6$, un groupe

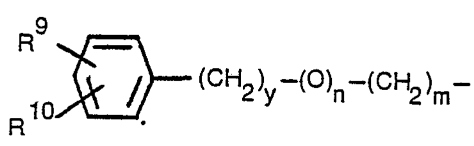

ou

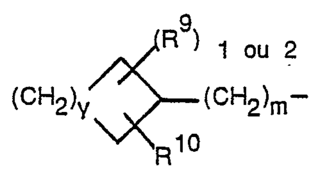

où:

m est un nombre entier de 0 à 4;

n est égal à 0 ou 1; et

$R^9$ et $R^{10}$ représentent chacun indépendamment l'un de l'autre un atome d'hydrogène, un atome d'halogène, un groupe alkyle en $C_1$—$C_4$ ou un groupe alcényle en $C_2$—$C_4$.

3. Formulation herbicide suivant la revendication 1 ou 2, caractérisée en ce que le dérivé de N-arylbenzamide de formule (I) est un dérivé dans lequel Z est un atome d'oxygène et $R^4$ représente un groupe

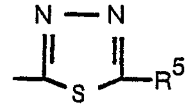

,

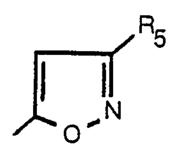

,

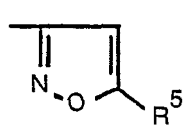

,

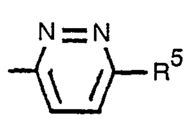

ou

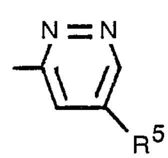

4. Formulation herbicide suivant l'une quelconque des revendications 1 à 3, caractérisée en ce que le dérivé de N-arylbenzamide de formule (I) est un dérivé dans lequel $R^1$ est un atome d'hydrogène, tandis que $R^2$ et $R^3$ représentent chacun un groupe alcoxy en $C_1$—$C_4$.

5. Formulation herbicide suivant la revendication 4, caractérisée en ce que le dérivé de N-arylbenzamide de formule (I) est un dérivé dans lequel $R^2$ est un substituant 2-méthoxy, tandis que $R^3$ est un substituant 6-méthoxy.

6. Formulation herbicide suivant l'une quelconque des revendications 1 à 5, caractérisée en ce que le dérivé de N-arylbenzamide de formule (I) est un dérivé dans lequel $R^5$ est un groupe volumineux ou à empêchement stérique.

7. Formulation herbicide suivant la revendication 6, caractérisée en ce que le dérivé de N-arylbenzamide de formule (I) est un dérivé dans lequel $R^5$ est un groupe de formule

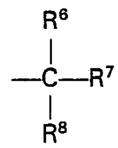

dans laquelle $R^6$ représente un atome d'hydrogène ou un groupe alkyle en $C_1$—$C_4$, $R^7$ représente un groupe alkyle en $C_1$—$C_4$ et $R^8$ représente un groupe alkyle en $C_1$—$C_{13}$.

8. Formulation herbicide suivant la revendication 7, caractérisée en ce que le dérivé de N-arylbenzamide de formule (I) est un dérivé dans lequel $R^5$ est un groupe 1-éthyl-1-méthyl-propyle.

9. Formulation herbicide suivant la revendication 6, caractérisée en ce que le dérivé de N-arylbenzamide de formule (I) est un dérivé dans lequel $R^5$ est un groupe de formule:

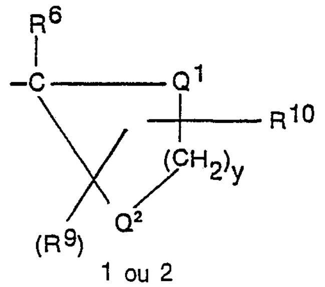

dans laquelle $R^6$ représente un groupe alkyle en $C_1$—$C_4$; $R^9$ et $R^{10}$ représentent chacun indépendamment l'un de l'autre un atome d'hydrogène ou un groupe alkyle en $C_1$—$C_4$; $Q^1$ et $Q^2$ représentent chacun $CH_2$ et y est un nombre entier de 0 à 4.

10. Formulation herbicide suivant la revendication 9, caractérisée en ce que le dérivé de N-arylbenzamide de formule (I) est un dérivé dans lequel $R^5$ est un groupe 1-éthyl-cyclohexyle.

11. Formulation herbicide suivant la revendication 1 ou 2, caractérisée en ce que le dérivé de N-arylbenzamide de formule (I) est le N-[3-(1-éthyl-1-méthylpropyl)-5-isoxazolyl]-2,6-diméthoxybenzamide.

12. Formulation herbicide suivant la revendication 1 ou 2, caractérisée en ce que le dérivé de N-arylbenzamide de formule (I) est le N-[3-(1,1-diméthyléthyl)-5-isoxazolyl]-2,6-diméthoxybenzamide.

13. Formulation herbicide suivant la revendication 1 ou 2, caractérisée en ce que le dérivé de N-arylbenzamide de formule (I) est le N-[3-(1-éthylcyclohexyl)-5-isoxazolyl]-2,6-diméthoxybenzamide.

14. Formulation herbicide suivant la revendication 1 ou 2, caractérisée en ce que le dérivé de N-arylbenzamide de formule (I) est le N-[6-(1,1-diméthyléthyl)pyridazin-3-yl]-2,6-diméthoxybenzamide.

15. Formulation herbicide suivant la revendication 1 ou 2, caractérisée en ce que le dérivé de N-arylbenzamide de formule (I) est le N-[6-(1-éthyl-1-méthylpropyl)-pyridazin-3-yl]-2,6-diméthoxybenzamide.

16. Formulation herbicide suivant l'une quelconque des revendications 1 à 15, caractérisée en ce qu'elle est une poudre mouillable.

17. Procédé en vue de combattre la croissance des végétations non désirées, caractérisé en ce qu'il consiste à appliquer, au locus où l'on désire combattre ces végétations, un dérivé de N-arylbenzamide de formule (I) ou un de ses sels acceptables en agronomie suivant l'une quelconque des revendications 1 à 15 en une quantité efficace du point de vue herbicide.

18. Procédé de préparation d'un dérivé de N-arylbenzamide de formule (I) suivant l'une quelconque des revendications 1 à 15, caractérisé en ce qu'il comprend les étapes qui consistent à:

(a) acyler une amine de formule $H_2NR^4$ avec un dérivé d'acide benzoïque substitué de formule:

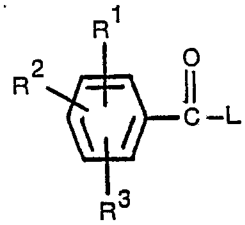

dans laquelle L est un bon groupe qui s'éloigne, pour former un dérivé de N-arylbenzamide de formule (I) dans laquelle Z est un atome d'oxygène;

(b) éventuellement sulfurer le produit de la réaction (a) pour former un N-arylbenzamide de formule (I) dans laquelle Z est un atome de soufre; et

(c) éventuellement salifier un dérivé de N-arylbenzamide de formule (I) obtenu par la réaction (a) ou (b) pour former un sel acceptable de agronomie.

19. Dérivé de N-arylbenzamide de formule (I) ou un de ses sels acceptables en agronomie, ce dérivé ou ce sel étant préparé par le procédé suivant la revendication 18.